(19) Europäisches Patentamt
European Patent Office
Office européen des brevets

(11) **EP 4 181 943 B1**

(12) **EUROPEAN PATENT SPECIFICATION**

(45) Date of publication and mention
of the grant of the patent:
**17.12.2025 Bulletin 2025/51**

(21) Application number: **21746543.4**

(22) Date of filing: **16.07.2021**

(51) International Patent Classification (IPC):
*A61K 38/16* (2006.01)    *A61K 38/48* (2006.01)
*A61P 29/02* (2006.01)    *A61P 25/22* (2006.01)

(52) Cooperative Patent Classification (CPC):
**A61K 38/164; A61K 38/4893; A61P 25/22;
A61P 29/02**

(86) International application number:
**PCT/GB2021/051838**

(87) International publication number:
**WO 2022/013575 (20.01.2022 Gazette 2022/03)**

(54) **TREATMENT OF POST-OPERATIVE SURGICAL PAIN**

BEHANDLUNG POSTOPERATIVER CHIRURGISCHER SCHMERZEN

TRAITEMENT DE LA DOULEUR CHIRURGICALE POST-OPÉRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.07.2020 GB 202011055**

(43) Date of publication of application:
**24.05.2023 Bulletin 2023/21**

(73) Proprietor: **Ipsen Biopharm Limited
Wrexham LL13 9UF (GB)**

(72) Inventors:
• **EVANS, Steve
Wrexham Industrial Estate Wrexham LL13 9UF
(GB)**
• **KALINICHEV, Mikhail
Wrexham Industrial Estate Wrexham LL13 9UF
(GB)**
• **PONS, Laurent
Wrexham Industrial Estate Wrexham LL13 9UF
(GB)**
• **CORNET, Sylvie
Wrexham Industrial Estate Wrexham LL13 9UF
(GB)**
• **LEZMI, Stéphane
Wrexham Industrial Estate Wrexham LL13 9UF
(GB)**

(74) Representative: **MacLean, Martin Robert et al
Mathys & Squire
The Shard
32 London Bridge Street
London SE1 9SG (GB)**

(56) References cited:
**WO-A1-2019/195454    WO-A1-2020/234573
WO-A2-01/78760        RU-C1- 2 628 238
US-A- 6 113 915        US-A1- 2008 113 051**

• **NOMA NOBORU ET AL: "Botulinum neurotoxin
type A alleviates mechanical hypersensitivity
associated with infraorbital nerve constriction
injury in rats", NEUROSCIENCE LETTERS, vol.
637, pages 96 - 101, XP029873548, ISSN:
0304-3940, DOI: 10.1016/J.NEULET.2016.11.043**
• **SHANE BARWOOD ET AL: "Analgesic effects of
botulinum toxin A: a randomized, placebo-
controlled clinical trial", DEVELOPMENTAL
MEDICINE AND CHILD NEUROLOGY, 1 February
2000 (2000-02-01), England, pages 116 - 121,
XP055766211, Retrieved from the Internet
<URL:https://onlinelibrary.wiley.com/doi/abs/
10.1111/j.1469-8749.2000.tb00056.x?sid=nlm%
3Apubmed> DOI: 10.1017/S0012162200000220**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE MEDLINE [online] US NATIONAL LIBRARY OF MEDICINE (NLM), BETHESDA, MD, US; November 2007 (2007-11-01), DOHIN B ET AL: "[Botulinum toxin for postoperative care after limb surgery in cerebral palsy children].", XP002801757, Database accession no. NLM18065878**
- **REVUE DE CHIRURGIE ORTHOPEDIQUE ET REPARATRICE DE L'APPAREIL MOTEUR NOV 2007, vol. 93, no. 7, November 2007 (2007-11-01), pages 674 - 681, ISSN: 0035-1040**
- **DOHIN ET AL: "Intérêt de la toxine botulique pour les suites opératoires en chirurgie des membres chez l'enfant infirme moteur cerebral - Botulinum toxin for postoperative care after limb surgery in cerebral palsy children", REVUE DE CHIRURGIE ORTHOPEDIQUE ET REPARATRICE DE L'APPAREILMOTEUR, MASSON, PARIS, FR, vol. 93, no. 7, 1 November 2007 (2007-11-01), pages 674 - 681, XP009525073, ISSN: 0035-1040, DOI: 10.1016/S0035-1040(07)73252-X**
- **SAEIDIBOROJENI SEPEHR ET AL: "Peri-operative Botulinum Neurotoxin injection to improve outcomes of surgeries on spastic limbs: A systematic review", TOXICON, ELMSFORD, NY, US, vol. 188, 10 October 2020 (2020-10-10), pages 48 - 54, XP086340691, ISSN: 0041-0101, [retrieved on 20201010], DOI: 10.1016/J.TOXICON.2020.10.005**

Remarks:
The complete document including Reference Table(s) and the Sequence Listing(s) can be downloaded from the EPO website

## Description

### FIELD OF THE INVENTION

The present invention relates to a botulinum neurotoxin A (BoNT/A) for use in the treatment of post-operative surgical pain.

### BACKGROUND

Post-operative surgical pain is an unpleasant sensation that results from a surgical procedure. Post-operative surgical pain may be caused by damage to tissue by surgical intervention, the surgical procedure itself, the closing of the wound, and any force that is applied during the surgical procedure. Surgical pain after surgery (e.g. post-operative surgical pain) can also stem from factors that accompany surgery. For example, a patient may suffer back pain due to the way the patient was positioned on the surgical table, or chest pain may be due to surgical intervention in the chest area. Throat pain may also occur after general anesthesia because the insertion of the breathing tube can cause irritation. However, most common is post-operative surgical pain caused by cutting into the skin and muscle from surgical intervention.

For example, the surgical intervention (or more particularly, surgical incision) may represent a 'noxious stimulus' causing pain. Noxious stimuli, stimuli which can elicit tissue damage, can activate the release of neurotransmitters from nociceptive afferent terminals and the release of neuropeptides such as Substance P and Calcitonin gene related peptide (CGRP) from sensory terminals. The noxious information is then transduced from the peripheral nervous system to the central nervous system, where pain is perceived by the individual.

Post-operative surgical pain can be caused by the combination of inflammation and neural tissue damage at a site of surgical intervention. Any inflammation and/or neural tissue damage is in addition to post-operative surgical pain. For example, degranulation of activated mast cells in response to tissue injury can result in the release of various substances including proteases, cytokines and serotonin. These substances can sensitize (activate at a lower threshold) primary afferent neurons to produce pain hypersensitivity. As tissue is extensively innervated, any region of the body is susceptible to nerve damage from surgery.

Post-operative anxiety may entail patients experiencing physical symptoms and behavioural changes including but not limited to fatigue, difficulties in concentrating and sleeping and muscle tension. Furthermore, the patient may experience emotional symptoms of anxiety, including restlessness, irritability, difficulties in controlling fear or worry, dread and panic. Post-operative anxiety may be caused by the effects of anesthesia, surgery itself, post-operative surgical pain and/or stress from the hospital environment. For example, surgery patients are often under considerable levels of mental, physical, and emotional stress both before (e.g. stress due to anticipation of surgery) and after surgery, with such stress manifesting in symptoms of anxiety.

Existing methods for the treatment of surgical (e.g. post-operative) pain typically target neurotransmitters and peptides, and include non-steroidal anti-inflammatory drugs (NSAIDS), opioids, local anesthetic blocks or their use in combination. However, these methods of treatment produce a variety of side effects, and, notably, often induce dependency (e.g. addiction). Additionally, these methods of treatment only provide acute post-operative surgical pain relief, for example providing surgical pain relief for only a short period of time after administration, thus necessitating the need for continuous/repeat administration (exacerbating the problem of patient dependency on/addiction to analgesics). Without effectively managing acute post-operative surgical pain, this can increase the chances of the patient developing chronic post-operative surgical pain. Such methods of pain management (requiring continuous administration of the drug) also often result in drug resistance. Further problems associated with previous methods for managing post-operative surgical pain include the need for high doses of analgesic drug administration to provide an analgesic effect, with adverse side effects often increasing congruent with the dose.

Barwood et al (Dev Med Child Neurol. 2000 Feb;42(2):116-21) describes analgesic effects of a BoNT/A. WO01/78760A2 describes a method for treating pain by peripheral administration of a neurotoxin. US6113915A1 describes a method for treating pain by intrathecal administration to a human patient of a therapeutically effective amount of a neurotoxin. WO2019/195454A1 describes compositions and methods for use in inhibiting CGRP production and release. Dohin et al (Rev Chir Orthop Reparatrice Appar Mot. 2007 Nov;93(7):674-81)) describes botulinum toxin for postoperative care after limb surgery in cerebral palsy children. RU2628238C1 describes a method of Botox injection to the pectoral muscle 8-10 days prior to mammoplasty. Saeidiborojeni et al (Toxicon. 2020; 188, 48-54) describes peri-operative botulinum neurotoxin injection to improve outcomes of surgeries on spastic limbs. WO2020/234573A1 describes a screening method to determine suitability for participation in a clinical trial. US2008/0113051A1 describes alleviation of tattoo pain.

Thus, current methods of treating surgical pain are not appropriate (e.g. sufficient) for managing/alleviating post-operative surgical pain, especially moderate to severe post-operative surgical pain and neither do they provide appropriate management of post-operative anxiety experienced by the patient (the latter generally requiring alternative/additional medication). Accordingly, there is an increasing need for alternative/improved methods for treating post-operative surgical pain and/or post-operative anxiety.

The present invention addresses one or more of these problems by providing a botulinum neurotoxin A (BoNT/A) for use in a method for the treatment of post-operative surgical pain according to claim 1 (and optionally post-operative anxiety caused by post-operative pain) (including reduced propensity for chronic post-operative surgical pain), for example even after a single (e.g. acute) administration. Preferred aspects of the invention are predicated on the surprising observation that initiating treatment (administration) prior to subjecting a patient to surgery allows for effective post-operative surgical pain (and optionally post-operative anxiety caused by post-operative pain) management as the patient emerges from surgery (advantageously mitigating post-operative surgical pain that would otherwise be perceived as the effect of general/local anaesthetic abates), and particular pre-surgery administration timepoints that are uniquely suited to BoNT/A based treatment are provided.

## SUMMARY OF THE INVENTION

The present invention is defined by the claims.

In more detail, the present invention is predicated on the surprising finding that administration of a BoNT/A pre-surgery by intradermal administration, 5-50 days prior to surgery treats post-operative surgical pain and reduces or suppresses post-operative anxiety caused by post-operative pain. This was completely unexpected as prior art methods reported optimal analgesic activity when BoNT/A was administered closer to the time of surgery and by alternative administration routes.

Advantageously, the inventors have demonstrated that, by administering the BoNT/A 5-50 days prior to surgery by intradermal administration, the BoNT/A is effective at treating post-operative surgical pain at a time point as early as one hour post-surgery, and continues to manage/treat post-operative surgical pain for several days (even weeks) post-surgery without the need for continuous administration and without the side effects associated with the traditional analgesic/anaesthetic drugs. As such, the BoNT/A can provide post-operative surgical pain relief as the effects of any 'general' or 'local' anaesthetic (used during surgery) wears off. In other words, pre-surgery (5-50 days pre-surgery) administration advantageously allows the analgesic effects of the BoNT/A to arise (e.g. reach maximal efficacy/effect) at a timepoint when the patient would otherwise begin to perceive post-operative surgical pain (and optionally post-operative anxiety caused by post-operative pain) as the effect of the principal anaesthetic/analgesic used during surgery tapers off. Therefore, post-operative surgical pain can be treated before it arises, preventing any associated (potentially significant) discomfort and distress in the patient. As described in more detail below, such early management of post-operative surgical pain (e.g. acute moderate to severe post-operative surgical pain) may advantageously mitigate the onset of chronic post-operative surgical pain.

This contrasts with treatment observed when the BoNT/A is administered closer to the time of surgery (or peri-operatively), wherein a distinct lag phase, or 'activation period', is observed post-surgery until any surgical pain-treatment effect of the BoNT/A is provided.

A yet further surprising observation by the inventors is that a BoNT/A is a particularly efficacious (e.g. rapid) post-operative surgical pain (and optionally post-operative anxiety caused by post-operative pain) treatment where the BoNT/A is administered intradermally. Indeed, the inventors observed that intradermal administration may provide for increased post-operative surgical pain (and optionally post-operative anxiety caused by post-operative pain) relief when compared with alternative administration routes such as subcutaneous administration and intramuscular administration. This was totally unexpected, as BoNT/A is regularly administered by such alternative administration routes (e.g. subcutaneous/intramuscular administration) in other indications without any apparent disadvantages in terms of efficacy.

Another advantageous finding by the inventors is that a BoNT/A is able to exert an effect at a site distal to the site of administration. For example, following administration of a BoNT/A at or proximal to a site of surgical intervention, the inventors observed SNARE protein cleavage (e.g. SNAP-25 protein cleavage) at the spinal cord (and minimal or no SNARE protein cleavage at or proximal to the site of surgical intervention), suggesting that the BoNT/A travels by retrograde transport from its site of administration to the spinal cord. This allows a BoNT/A to be administered at a site away from a(ny) site of injury (e.g. the site of surgical intervention) that may cause discomfort to a patient and thus minimise any further pain perceived by the patient.

## DETAILED DESCRIPTION

The present invention is defined by the claims.

In one aspect the invention provides a BoNT/A for use in treating post-operative surgical pain in a patient according to claim 1.

Optionally, the treatment also reduces or suppresses/prevents (e.g. completely prevents) the onset of anxiety in a patient post-surgery (post-operative anxiety caused by post-operative pain).

Various additional (optional) embodiments of the invention will now be described. It should be noted that each of the following embodiments may apply to any BoNT/A for use described herein.

In one embodiment, the administration of the BoNT/A does not include intramuscular administration. The BoNT/A is administered 5-50 days prior to surgery, for example 6-50 days prior to surgery, optionally 5-40 days prior to surgery. For example, the BoNT/A may be administered 5-30 days prior to surgery; preferably 5-20 days prior to surgery; more preferably 5-15 days prior to surgery.

In one embodiment, the BoNT/A may be administered in a single administration step. For example, the BoNT/A may be administered 10-20 days prior to surgery; 14-16 days prior to surgery , optionally in a single administration step.

The BoNT/A may be administered 15 or more days prior to surgery, preferably about 15 days prior to surgery.

In a preferred embodiment, the BoNT/A may be administered >5 days to ≤ 15 days prior to surgery, optionally in a single administration step.

In one embodiment, the BoNT/A is administered 12 or more days prior to surgery.

In one embodiment, the BoNT/A is administered intradermally 15 or more days prior to surgery. In a preferred embodiment, the BoNT/A is administered intradermally about 15 days prior to surgery.

The BoNT/A treats post-operative surgical pain through the provision of an analgesic effect. Thus, the term "treat" or "treating" as used herein is intended to encompass analgesic treatment. The term "treat" or "treating" encompasses treating post-operative surgical pain such that the patient no longer perceives surgical pain (or perceives less surgical pain compared to a control patient not treated with the BoNT/A).

Similarly, the BoNT/A suppresses post-operative anxiety caused by post-operative pain through the provision of an anxiolytic effect. Thus, the term "suppress" or "suppressing" encompasses suppression of post-operative anxiety (e.g. symptoms thereof) in a patient via an anxiolytic effect provided by administration of a BoNT/A. The suppression may be provided concomitantly with, and for example as a result of, post-operative surgical pain treatment. Thus, without being bound to any theories, the BoNT/A may provide an anxiolytic effect by means of the BoNT/A's analgesic effect. The BoNT/A may suppress symptoms of post-operative anxiety which are associated with (or arise from) effects of anaesthesia employed for surgery, surgery itself, post-operative surgical pain and/or stress (e.g. from the hospital environment).

Therefore, a BoNT/A may be administered to a subject in a therapeutically effective amount or a prophylactically effective amount (preferably prophylactically effective amount). A "therapeutically effective amount" is any amount of the BoNT/A that, when administered alone or in combination to a subject for treating post-operative surgical pain (and optionally post-operative anxiety caused by post-operative pain) is sufficient to effect such treatment of post-operative surgical pain (and optionally post-operative anxiety caused by post-operative pain). A "prophylactically effective amount" is any amount of the

BoNT/A that, when administered alone or in combination to a subject inhibits or delays the onset of post-operative surgical pain (and optionally post-operative anxiety caused by post-operative pain). In some embodiments, the prophylactically effective amount prevents the onset of post-operative anxiety entirely. "Inhibiting" the onset means either lessening the likelihood of post-operative surgical pain (and optionally post-operative anxiety caused by post-operative pain), or preventing the onset entirely.

Preferably, a therapeutically and/or prophylactically effective amount is an amount which does not lead to muscle paralysis. The term "muscle paralysis" preferably refers to long-term muscle paralysis, since transient muscle paralysis may occur for a short period following administration.

The terms "subject", "individual" and "patient" may be used interchangeably herein to refer to a mammalian subject. In one embodiment the "subject" is a human, a companion animal (e.g. a pet such as a dog, cat, and/or rabbit), livestock (e.g. a pig, sheep, cattle, and/or a goat), and/or a horse. In a preferable embodiment, the subject (patient) is a human.

The present invention relates specifically to post-operative surgical pain, which is distinct from other types of pain such as inflammatory pain and neuropathic pain. In this regard, inflammatory pain typically arises from an infection, irritants or an overactivated immune response and neuropathic pain typically arises from a nervous system disorder/syndrome. In contrast, the present invention does not relate to pain arising from these stimuli.

In one embodiment administration of a BoNT/A treats post-operative surgical pain preferentially to inflammatory pain. In one embodiment administration of a BoNT/A treats minimal to no inflammatory pain. In one embodiment administration of a BoNT/A treats post-operative surgical pain and minimal to no inflammatory pain.

Reference to "surgical intervention" means a medical procedure involving the treatment of an injury or disease in a subject comprising subjecting a part of the body to an incision (optionally removing or repairing a damaged part of the body). Although the level of invasiveness (e.g. level of surgical incision required) may vary amongst surgery types, surgery having a level of invasiveness that causes post-operative surgical pain and/or post-operative anxiety in the subject once surgery is complete is intended to be encompassed. Post-operative surgical pain is typically caused by a surgical incision that cuts through the skin ; and fascia, muscle, bone, and/or an organ in a patient. Thus, surgical pain is typically experienced at/or proximal to the site of surgical intervention.

The surgical intervention comprises an incision to skin ; and fascia, muscle, bone, and/or an organ.

The surgical intervention is not limited to that which may be carried out by a physician, but also includes for example dental surgical intervention. Non-limiting examples of surgical intervention include appendectomy, breast biopsy, breast augmentation or reduction, facelift, cholecystectomy, coronary artery bypass, debridement (e.g. of a wound, a burn, or infection), skin graft, organ transplant and tonsillectomy.

Preferably, "post-operative" may refer to a time period beginning at most one day subsequent to surgery (e.g. post-surgery). In other words, the term "post-operative" may refer to a time period beginning not greater than one day post-surgery. For example, the term "post-operative" may refer to a time point beginning 1-20 hours post-surgery; optionally 2-15 hours post-surgery; optionally 5-10 hours post-surgery. Such time may represent a time period beginning at the chronological interface at which the analgesic effects from a surgical anaesthetic administered to a patient diminish (e.g. taper) and thus the patient begins to perceive surgical pain.

Furthermore, the term "post-operative" may be used interchangeably with the term "post-surgical", as 'operative' is used in the sense of 'surgery' herein.

Similarly, the term "post-operative surgical pain" may refer to surgical pain that is perceived (or more particularly, begins to be perceived) for a time period beginning at most one day subsequent to surgery (e.g. post-surgery). In other words, the term "post-operative surgical pain" may refer to surgical pain that is perceived by a patient for a time period beginning not greater than one day post-surgery. For example, the term "post-operative surgical pain" may refer to pain that is perceived for a time period beginning 1-20 hours post-surgery; optionally 2-15 hours post-surgery; optionally 5-10 hours post-surgery.

Said time period may be 1-50 weeks; for example 5-45 weeks, 10-40 weeks or 10-35 weeks post-surgery.

This contrasts with the term "peri-operative", which may refer, for example, to a time period at or around the time that a patient is undergoing surgery (e.g. the time when the patient is in the operating theatre), suitably a period beginning at least 1 hour pre-surgery and/or ending less than 1 hour post-surgery.

The post-surgery treatment of the present invention may be combined with a peri-operative and/or post-operative treatment strategy to improve efficacy and preferably increase duration or post-operative surgical pain suppression (e.g. in patients at high risk of developing surgical pain).

The post-operative surgical pain may be pain caused by the release of neurotransmitters from nociceptive afferent terminals and/or the release of neuropeptides such as Substance P and Calcitonin gene related peptide (CGRP) from sensory terminals, for example induced by a noxious stimulus of surgery (preferably a surgical incision). For example, noxious information (resulting from noxious stimuli) may then be transduced from the peripheral nervous system to the central nervous system, where surgical pain is perceived by the patient.

Post-operative surgical pain may be caused by inflammation or neural tissue damage at a site of surgical intervention, or a combination thereof. Any inflammation and/or neural tissue damage is in addition to post-operative surgical pain. For example, degranulation of activated mast cells in response to tissue injury may result in the release of various substances including proteases, cytokines, and serotonin. These substances can sensitize (activate at a lower threshold) primary afferent neurons to produce pain hypersensitivity. As tissue is extensively innervated, any region of the body may be susceptible to nerve damage from surgery.

In other words, the pain may be nociceptive pain, for example where post-operative surgical pain arises from tissue damage and is perceived by the activation of nociceptors (pain receptors) in response to noxious stimuli.

Post-operative surgical pain may manifest as other types of pain such as allodynia. Allodynia means "other pain." It is a pain that results from a stimulus that is not normally painful. A sufferer of 'tactile' allodynia (aka static tactile allodynia or mechanical allodynia) may experience pain to touch, such as with resting the (body) site of surgical incision on a bed, or with wearing clothing which contacts said site. Thus, allodynia is considered "pain due to a stimulus that does not usually provoke pain", as opposed to hyperalgesia (increased pain from a stimulus that does usually provoke pain).

The post-operative surgical pain may preferably be acute post-operative surgical pain; for example, a type of surgical pain that may last less than 3 months (post-surgery).

In one embodiment, the post-operative surgical pain is chronic post-operative surgical pain; for example, a type of surgical pain that may last longer than three months (post-surgery) and may continue to be perceived after tissue damage (e.g. due to surgical incision) has healed.

In more detail, the term "chronic post-operative surgical pain" as used herein preferably refers to pain persisting for longer than 3 months beyond resolution of the underlying insult (e.g. damage to muscles from surgical incision), for example pain persisting beyond 3 months post-surgery. "Chronic post-operative surgical pain" may, for example, develop from insufficient (or lack of) treatment of acute post-operative surgical pain (e.g. a type of surgical pain that typically lasts for less than 3 months). Poor management of post-operative "acute surgical pain" may increase the chance of such acute surgical pain becoming chronic post-operative surgical pain. Thus, advantageously, by managing acute post-operative surgical pain at an early stage (advantageously due to the pre-surgery administration providing for analgesic efficacy shortly after surgery), the prevent invention mitigates the occurrence of chronic post-operative surgical pain.

Chronic post-operative surgical pain may be perceived at or around a scar (a scar formed at the site of surgical incision).

In more detail, reference to "reduced" (in terms of post-operative surgical pain) preferably means a lower level of surgical pain is perceived by the subject (e.g. patient) administered with BoNT/A when compared with a level of surgical pain perceived by a subject (that has likewise been subjected to surgery) administered no BoNT/A (or administered a placebo). For example, the level of surgical pain perception may be reduced by at least 15%, 25%, 35%, 45%, 55%, 65%, 75%, 85% or 95% post-administration of the BoNT/A, when compared with a subject (that has likewise been subjected to surgery) administered no BoNT/A (or administered a placebo). For example, the level of surgical pain perception may be reduced by at least 75%; preferably at least 85%; more preferably at least 95% post-administration of the BoNT/A, when compared with a subject (that has likewise been subjected to surgery) administered no BoNT/A (or administered a placebo).

A variety of means for assessing pain perception are known to those skilled in the art. For example, evaluation of mechanical allodynia (either static or dynamic) is routinely used in human pain studies as described in Pogatzki-Zahn et. al. (Pain Rep. 2017 Mar; 2(2): e588).

A suitable (albeit non-limiting example) method for assessing pain perception in a subject includes the following: Numerical Rating Scale (NRS) score; although the skilled person is aware of other methods which may be used additionally or alternatively such as sensory threshold, pain perception threshold, static mechanical allodynia, dynamic mechanical allodynia, temporal summation, pressure pain threshold, conditioned pain modulation, and temperature threshold.

Other non-limiting examples of pain perception measures include: change from baseline in SF-36 scores at each scheduled time point; amount of rescue medication taken during the study and time to first intake of rescue medication. These may be considered "exploratory" endpoints or pain perception assessment measures.

The skilled person is aware of such methods for assessing pain perception. For convenience, further description of the Numerical Rating Score and Quality of Life questionnaire Short Form-36 are provided below.

Numerical Rating Scale (NRS): Typically surgical pain perception according to the present invention uses the Numerical Rating Scale (NRS). The NRS is an 11-point scale to assess subject surgical pain perception. Subjects are asked to give a number between 0 and 10 that fits best to their surgical pain intensity. Zero represents 'no surgical pain at all' whereas the upper limit, 10, represents 'the worst surgical pain possible'.

The NRS can be used to assess numerous facets of surgical pain, including spontaneous average surgical pain, spontaneous worst surgical pain and spontaneous current surgical pain. Spontaneous average surgical pain is assessed by asking a subject to select a number that best describes the subject's average surgical pain (e.g. perceived surgical pain) over a period of time, for example at least 6 hours, 12 hours, 24 hours, or at least 48 hours. Spontaneous worst surgical pain is assessed by asking a subject to select a number that best describes the subject's surgical pain at its worst during a specified period, e.g. at least the previous 6 hours, 12 hours, 24 hours or previous 48 hours. Spontaneous current surgical pain is assessed by asking a subject to select a number that best describes how much surgical pain the subject is in at the time of assessment.

The NRS can also be used to assess a subject's surgical pain perception in response to a variety of different stimuli. To assess surgical pain perception in response to a stimulus, the subject will be submitted to stimuli of various nature applied to the painful area. Subjects will be asked what are their current NRS scores pre-dose and post-stimulus.

Examples of stimuli used include: (i) light touch (which can be assessed by measuring pain on the surface of the painful area on radial spokes following application of a von Frey filament as described herein); (ii) pressure (pressure pain threshold), which can be assessed by asking the subject to give a NRS score as increasing pressure is applied using a pressure algometer as described herein; and (iii) temperature (which can be assessed by asking the subject for an NRS score for warm, cold and hot stimulation using a thermode applied to the painful area, as described herein).

Preferably, administration of a BoNT/A described herein reduces the patient's NRS score post-surgery (e.g. from a rating of ≥7 to a rating of ≤6) when compared with an NRS score of a control patient that is not administered a BoNT/A

Quality of Life questionnaire Short Form-36 (SF-36): The SF-36 quality of life questionnaire may be used to assess a subject's surgical pain perception. The SF-36 is a 36-item, subject-reported survey of subject health. The SF-36 consists of eight scaled scores (vitality, physical functioning, bodily pain, general health perceptions, physical role functioning, emotional role functioning, social role functioning and mental health). Each scale is directly transformed into a 0-100 scale on the assumption that each question carries equal weight. The higher the score recorded in the SF-36, the less disability.

Relevant parameters commonly tested in clinical trials for the treatment of surgical pain are known in the art and could be readily selected by one of ordinary skill in the art. Examples of such parameters include, but are not limited to NRS; stimulus-evoked NRS; temperature of the painful area; size of the painful area; time to onset of analgesic effect; peak analgesic effect; time to peak analgesic effect; duration of analgesic effect; and/or SF-36 quality of life as described herein. Methods for assessing these parameters are also known in the art and can be carried out by one of ordinary skill using routine methods and procedures.

Preferably, administration of a BoNT/A described herein increases the patient's SF-36 score post-surgery (e.g.

from a score of ≤50 to a score of ≥50) when compared with an SF-36 score of a control patient that is not administered a BoNT/A.

Turning now to "post-operative anxiety", reference to the same (i.e, post-operative anxiety) may refer to a condition in which a patient experiences physical symptoms and behavioural changes including but not limited to fatigue, difficulties in concentrating and sleeping and muscle tension. Furthermore, the patient may experience emotional symptoms of anxiety, including restlessness, irritability, difficulties in controlling fear or worry, dread and panic. Post-operative anxiety may be caused by the effects of anesthesia, surgery itself, post-operative surgical pain and stress from the hospital environment.

Thus, in one embodiment, post-operative anxiety is caused by post-operative surgical pain.

The post-operative anxiety may be defined as a panic disorder, a phobia, a post-traumatic stress disorder, a social anxiety disorder (social phobia) or a generalised anxiety disorder (GAD) (e.g. a long-term condition that causes you to feel anxious about a wide range of situations and issues, rather than one specific event).

Reference to "reduced" (in terms of post-operative anxiety) preferably means a lower level of anxiety is perceived by the subject (e.g. patient) administered with BoNT/A when compared with a level of anxiety perceived by a subject (that has likewise been subjected to surgery) administered no BoNT/A (or administered a placebo). For example, the level of anxiety perception may be reduced by at least 15%, 25%, 35%, 45%, 55%, 65%, 75%, 85% or 95% post-administration of the BoNT/A, when compared with a subject (that has likewise been subjected to surgery) administered no BoNT/A (or administered a placebo). For example, the level of anxiety perception may be reduced by at least 75%; preferably at least 85%; more preferably at least 95% post-administration of the BoNT/A, when compared with a subject (that has likewise been subjected to surgery) administered no BoNT/A (or administered a placebo).

The inventors have demonstrated that a BoNT/A may be administered to both treat post-surgical pain as well as suppress post-operative anxiety. Thus, in one embodiment the BoNT/A treats post-operative pain and reduces or suppresses post-operative anxiety.

Further details of clostridial neurotoxins are provided below, together with technological background information.

Bacteria in the genus Clostridia produce highly potent and specific protein toxins, which can poison neurons and other cells to which they are delivered. Examples of such clostridial toxins include the neurotoxins produced by *C. tetani* (TeNT) and by *C. botulinum* (BoNT) serotypes A-G, as well as those produced by *C. baratii* and *C. butyricum.*

Clostridial neurotoxins (for example, in nature) cause muscle paralysis by inhibiting cholinergic transmission in the peripheral nervous system, in particular at the neuromuscular junction, and can thus be lethal. In nature, clostridial neurotoxins are synthesised as a single-chain polypeptide that is modified post-translationally by a proteolytic cleavage event to form two polypeptide chains joined together by a disulphide bond. Cleavage occurs at a specific cleavage site, often referred to as the activation site, which is located between the cysteine residues that provide the inter-chain disulphide bond. It is this di-chain form that is the active form of the toxin. The two chains are termed the heavy chain (H-chain), which has a molecular mass of approximately 100 kDa, and the light chain (L-chain), which has a molecular mass of approximately 50 kDa. The H-chain comprises an N-terminal translocation component (HN domain) and a C-terminal targeting component (HC domain). The cleavage site is located between the L-chain and the HN domain.

The mode of action of clostridial neurotoxins relies on five distinct steps: (1) binding of the HC domain to the cell membrane of its target neuron, followed by (2) internalisation of the bound toxin into the cell via an endosome, (3) translocation of the L-chain by the HN domain across the endosomal membrane and into the cytosol, (4) proteolytic cleavage of intracellular transport proteins known as SNARE proteins by the L-chain which provides a non-cytotoxic protease function, and (5) inhibition of cellular secretion from the target cell.

Non-cytotoxic proteases act by proteolytically cleaving intracellular transport proteins known as SNARE proteins (e.g. SNAP-25, VAMP, or Syntaxin) - see Gerald K (2002) "Cell and Molecular Biology" (4th edition) John Wiley & Sons, *Inc.* The acronym SNARE derives from the term Soluble NSF Attachment Receptor, where NSF means N-ethylmaleimide-Sensitive Factor. SNARE proteins are integral to intracellular vesicle fusion, and thus to secretion of molecules via vesicle transport from a cell. The protease function is a zinc-dependent endopeptidase activity and exhibits a high substrate specificity for SNARE proteins. Accordingly, once delivered to a desired target cell, the non-cytotoxic protease is capable of inhibiting cellular secretion from the target cell. The L-chain proteases of clostridial neurotoxins are non-cytotoxic proteases that cleave SNARE proteins.

Thanks to their unique properties, Clostridial neurotoxins, such as botulinum toxin, have been successfully employed in a wide range of therapeutic applications, in particular for motor and autonomic disorders, to restore for example the activity of hyperactive nerve endings to normal levels. At least seven antigenically distinct BoNTs serotypes have been described so far, namely BoNT/A, BoNT/B, BoNT/C, BoNT/D, BoNT/E, BoNT/F, BoNT/G (Rossetto, O. et al., "Botulinum neurotoxins: genetic structural and mechanistic insights." Nature Reviews Microbiology 12.8 (2014): 535-549).

Despite this diversity, BoNT/A remains the serotype of choice in therapy, with three commonly available commercial preparations (Botox®, Dysport® and Xeomin®), while only one BoNT/B product is available on the market (Neurobloc®/Myobloc®). To this day, these BoNT/A and BoNT/B products, which are toxins purified from clostridial strains, are the only two BoNT serotypes that are currently approved by regulatory agencies for use in humans for applications ranging, among others, from spasticity, bladder dysfunction, or hyperhidrosis (for BoNT/A) (see for example: https://www.medicines.org.uk/emc/medicine/112,https://www.medicines.org.uk/emc/medicine /870, https://www.medicines.org.uk/emc/medicine/2162) to cervical dystonia (for BoNT/B) (see for example, https://www.medicines.org.uk/emc/medicine/20568 ).

In contrast to a cytotoxic protease (e.g. ricin, diphtheria toxin, pseudomonas exotoxin), which acts by killing its natural target cell, clostridial neurotoxins are non-cytotoxic proteases acting by transiently incapacitating the cellular function of its natural target cell. Importantly, a non-cytotoxic protease does not kill the natural target cell upon which it acts. In addition to clostridial neurotoxins (e.g. botulinum neurotoxin, marketed under names such as Dysport™, Neurobloc™, and Botox™), some of the best known examples of non-cytotoxic proteases include IgA proteases (see, for example, WO99/032272), and antarease proteases (see, for example, WO2011/022357).

The term "clostridial neurotoxin" as used herein means any polypeptide that enters a neuron and inhibits neurotransmitter release. This process encompasses the binding of the neurotoxin to a low or high affinity receptor, the internalisation of the neurotoxin, the translocation of the endopeptidase portion of the neurotoxin into the cytoplasm and the enzymatic modification of the neurotoxin substrate. More specifically, the term "neurotoxin" encompasses any polypeptide produced by *Clostridium* bacteria (clostridial neurotoxins) that enters a neuron and inhibits neurotransmitter release, and such polypeptides produced by recombinant technologies or chemical techniques. Preferably, the clostridial neurotoxin is a botulinum neurotoxin (BoNT).

BoNT serotypes A to G can be distinguished based on inactivation by specific neutralising antisera, with such classification by serotype correlating with percentage sequence identity at the amino acid level. BoNT proteins of a given serotype are further divided into different subtypes on the basis of amino acid percentage sequence identity.

An example of a BoNT/A neurotoxin amino acid sequence is provided as SEQ ID NO: 1 (UniProt accession number A5HZZ9) and SEQ ID NO: 13, which are encoded by the nucleotide sequence provided as SEQ ID NO: 12. An example of a BoNT/B neurotoxin amino acid sequence is provided as SEQ ID NO: 2 (UniProt accession number B1INP5). An example of a BoNT/C neurotoxin amino acid sequence is provided as SEQ ID NO: 3 (UniProt accession number P18640). An example of a BoNT/D neurotoxin amino acid sequence is provided as SEQ ID NO: 4 (UniProt accession number P19321). An example of a BoNT/E neurotoxin amino acid sequence is provided as SEQ ID NO: 5 (accession number WP_003372387). An example of a BoNT/F neurotoxin amino acid sequence is provided as SEQ ID NO: 6 (UniProt accession number Q57236) or as SEQ ID NO: 9 (UniProt/UniParc accession number UPI0001DE3DAC). An example of a BoNT/G neurotoxin amino acid sequence is provided as SEQ ID NO: 7 (accession number WP_039635782). An example of a BoNT/D-C neurotoxin amino acid sequence is provided as SEQ ID NO: 8 (accession number BAM65681). An example of a BoNT/X neurotoxin amino acid sequence is provided as SEQ ID NO: 11 (accession number BAQ12790.1). Preferably BoNT is BoNT/A, more preferably wild-type BoNT/A.

The term "$H_c$ domain" as used herein refers to a functionally distinct region of the neurotoxin heavy chain with a molecular weight of approximately 50 kDa that enables the binding of the neurotoxin to a receptor located on the surface of the target cell. The $H_c$ domain consists of two structurally distinct subdomains, the "$H_{CN}$ subdomain" (N-terminal part of the $H_c$ domain) and the "$H_{CC}$ subdomain" (C-terminal part of the $H_c$ domain, also named Hcc domain), each of which having a molecular weight of approximately 25 kDa. A $H_{CC}$ domain is capable of binding to a clostridial neurotoxin protein receptor.

The term "$LH_N$ domain" as used herein refers to a neurotoxin region that is distinct from the $H_c$ domain, and which consists of an endopeptidase domain ("L" or "light chain") and of a domain responsible for translocation of the endopeptidase into the cytoplasm ($H_N$ domain of the heavy chain). An endopeptidase domain ("L" or "light chain") is capable of cleaving a SNARE protein.

Exemplary L, $H_N$, $H_{CN}$ and $H_{CC}$ domains are shown in Table 1.

Table 1 - Exemplary L, $H_N$, $H_{CN}$ and $H_{CC}$ domains

| BoNT | Accession Number | SEQ ID NO: | L | $H_N$ | $H_{CN}$ | $H_{CC}$ |
|---|---|---|---|---|---|---|
| BoNT/A1 | A5HZZ9 | 1 | 1-448 | 449-872 | 873-1094 | 1095-1296 |
| BoNT/B1 | B1INP5 | 2 | 1-441 | 442-859 | 860-1081 | 1082-1291 |
| BoNT/C1 | P18640 | 3 | 1-449 | 450-867 | 868-1095 | 1096-1291 |
| BoNT/D | P19321 | 4 | 1-442 | 443-863 | 864-1082 | 1083-1276 |
| BoNT/E1 | WP_003372387 | 5 | 1-423 | 424-846 | 847-1069 | 1070-1252 |
| BoNT/F1 | Q57236 | 6 | 1-439 | 440-865 | 866-1087 | 1088-1278 |
| BoNT/F7 | UPI0001DE3DAC | 9 | 1-508 | 509-862 | 863-1076 | 1077-1268 |
| BoNT/G | WP_039635782 | 7 | 1-446 | 447-864 | 865-1089 | 1090-1297 |
| BoNT/DC | BAM65681 | 8 | 1-442 | 443-863 | 864-1091 | 1092-1285 |

(continued)

| BoNT | Accession Number | SEQ ID NO: | L | H$_N$ | H$_{CN}$ | H$_{CC}$ |
|------|------------------|------------|---|-------|----------|----------|
| BoNT/X | BAQ12790.1 | 11 | 1-439 | 440-892 | 893-1306 | |

The above-identified reference sequences should be considered a guide, as slight variations may occur according to sub-serotypes. By way of example, US 2007/0166332 cites slightly different clostridial sequences.

The term "activation loop" refers to a polypeptide domain comprising a proteolytic cleavage site. Activation loops of neurotoxins have been described in the art, such as in WO2016156113.

In one embodiment, the BoNT/A consists of or comprises an amino acid sequence of SEQ ID NO: 1

The BoNT/A of the present invention can be produced using recombinant technologies.

Employing such recombinant neurotoxins may advantageously widen the choice of BoNT/A to employ in the methods described herein, for example chosen based on properties such as potency and duration of action as deemed appropriate for any given surgery. Suitable (known) recombinant BoNT/A include modified botulinum neurotoxin A (BoNT/A) which preferably has a longer duration of action when compared to unmodified BoNT/A (e.g. Dysport®). Said duration of action may be at least 1.25x, 1.5x, 1.75x, 2.0x, or 2.25x greater. The duration of action of modified BoNT/A may be between 6 and 9 months. For example, a duration of action may be at least: 4.5 months (from onset), 5.0 months, 5.5 months, 6 months, 6.5 months, 7.0 months, 7.5 months, 8.0 months, 8.5 months or 9.0 months.

Suitable modified BoNT/A polypeptides (and nucleotide sequences encoding the same, where present) are described in WO 2015/004461 A1 and WO 2017/191315.

In one embodiment, the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, preferably wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue.

The modification may be a modification when compared to unmodified BoNT/A shown as SEQ ID NO: 1, wherein the amino acid residue numbering is determined by alignment with SEQ ID NO: 1. As the presence of a methionine residue at position 1 of SEQ ID NO: 1 (as well as the SEQ ID NOs corresponding to modified BoNT/A polypeptides described herein) is optional, the skilled person will take the presence/absence of the methionine residue into account when determining amino acid residue numbering. For example, where SEQ ID NO: 1 includes a methionine, the position numbering will be as defined above (e.g. ASN 886 will be ASN 886 of SEQ ID NO: 1). Alternatively, where the methionine is absent from SEQ ID NO: 1 the amino acid residue numbering should be modified by -1 (e.g. ASN 886 will be ASN 885 of SEQ ID NO: 1). Similar considerations apply when the methionine at position 1 of the other polypeptide sequences described herein is present/absent, and the skilled person will readily determine the correct amino acid residue numbering using techniques routine in the art. The same applies to any other BoNT described herein (e.g. the chimeric BoNTs described above).

The amino acid residue(s) indicated for modification are surface exposed amino acid residue(s).

The modified BoNT/A may be encoded by a nucleic acid sequence having at least 70% sequence identity to a nucleic acid sequence selected from SEQ ID NOs: 14, 16, 18, and 20. For example, a nucleic acid sequence having at least 80%, 90%, 95% or 99.9% sequence identity to a nucleic acid sequence selected from SEQ ID NOs: 14, 16, 18, and 20. Preferably, a modified BoNT/A for use in the invention may be encoded by a nucleic acid sequence comprising (or consisting of) SEQ ID NOs: 14, 16, 18 or 20. The modified BoNT/A may comprise a polypeptide sequence having at least 70% sequence identity to a polypeptide sequence selected from SEQ ID NOs: 15, 17, 19, and 21. For example, a polypeptide sequence having at least 80%, 90%, 95% or 99.9% sequence identity to a polypeptide sequence selected from SEQ ID NOs: 15, 17, 19, and 21. Preferably, a modified BoNT/A for use in the invention may comprise (more preferably consist of) a polypeptide sequence selected from SEQ ID NOs: 15, 17, 19, and 21.

The term "one or more amino acid residue(s)" when used in the context of modified BoNT/A preferably means at least 2, 3, 4, 5, 6 or 7 of the indicated amino acid residue(s). Thus, a modified BoNT/A may comprise at least 2, 3, 4, 5, 6 or 7 (preferably 7) modifications at the indicated amino acid residue(s). A modified BoNT/A may comprise 1-30, 3-20, or 5-10 amino acid modifications. More preferably, the term "one or more amino acid residue(s)" when used in the context of modified BoNT/A means all of the indicated amino acid residue(s).

Preferably, beyond the one or more amino acid modification(s) at the indicated amino acid residue(s), the modified BoNT/A does not contain any further amino acid modifications when compared to SEQ ID NO: 1.

Most preferably, a modified BoNT/A comprises (more preferably consists of) a modification at one or more amino acid residue(s) selected from: ASN 886, ASN 930, SER 955, GLN 991, ASN 1026, ASN 1052, and GLN 1229. The modified BoNT/A may be encoded by a nucleic acid sequence having at least 70% sequence identity to SEQ ID NO: 14. For example, a nucleic acid sequence having at least 80%, 90%, 95% or 99.9% sequence identity to SEQ ID NO: 14. Preferably, a modified BoNT/A for use in the invention may be encoded by a nucleic acid comprising (or consisting of) SEQ ID NO: 14. The modified BoNT/A may comprise a polypeptide sequence having at least 70% sequence identity to SEQ ID NO: 15. For example, a polypeptide sequence having at least 80%, 90%, 95% or 99.9% sequence identity to SEQ ID NO: 15. Preferably, a modified BoNT/A for use in the invention may comprise (more preferably consist of) SEQ ID NO: 15.

The modification may be selected from:(i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue.

A modification as indicated above results in a modified BoNT/A that has an increased positive surface charge and increased isoelectric point when compared to the corresponding unmodified BoNT/A.

The isoelectric point (pI) is a specific property of a given protein. In more detail, the isoelectric point (pI) is defined as the pH value at which a protein displays a net charge of zero. An increase in pI means that a higher pH value is required for the protein to display a net charge of zero. Thus, an increase in pI represents an increase in the net positive charge of a protein at a given pH. Conversely, a decrease in pI means that a lower pH value is required for the protein to display a net charge of zero. Thus, a decrease in pI represents a decrease in the net positive charge of a protein at a given pH.

Methods of determining the pI of a protein are known in the art and would be familiar to a skilled person. By way of example, the pI of a protein can be calculated from the average pKa values of each amino acid present in the protein ("calculated pI"). Such calculations can be performed using computer programs known in the art, such as the Compute pI/MW Tool from ExPASy (https://web.expasy.org/compute_pi), which is the preferred method for calculating pI in accordance with the present invention. Comparisons of pI values between different molecules should be made using the same calculation technique/program. Where appropriate, the calculated pI of a protein can be confirmed experimentally using the technique of isoelectric focusing ("observed pI"). This technique uses electrophoresis to separate proteins according to their pI. Isoelectric focusing is typically performed using a gel that has an immobilised pH gradient. When an electric field is applied, the protein migrates through the pH gradient until it reaches the pH at which it has zero net charge, this point being the pI of the protein. Results provided by isoelectric focusing are typically relatively low-resolution in nature, and thus the present inventors believe that results provided by calculated pI (as described above) are more appropriate to use.

Throughout the present specification, "pI" means "calculated pI" unless otherwise stated. The pI of a protein may be increased or decreased by altering the number of basic and/or acidic groups displayed on its surface. This can be achieved by modifying one or more amino acids of the protein. For example, an increase in pI may be provided by reducing the number of acidic residues, or by increasing the number of basic residues.

A modified BoNT/A may have a pI value that is at least 0.2, 0.4, 0.5 or 1 pI units higher than that of an unmodified BoNT/A (e.g. SEQ ID NO: 1). Preferably, a modified BoNT/A may have a pI of at least 6.6, e.g. at least 6.8.

The properties of the 20 standard amino acids are indicated in the table below:

| Amino Acid | | | Side Chain | Amino Acid | | | Side Chain |
|---|---|---|---|---|---|---|---|
| Aspartic acid | Asp | D | Charged (acidic) | Methionine | Met | M | Uncharged (polar) |
| Glutamic acid | Glu | E | Charged (acidic) | Tryptophan | Trp | W | Uncharged (polar) |
| Arginine | Arg | R | Charged (basic) | Cysteine | Cys | C | Uncharged (polar) |
| Lysine | Lys | K | Charged (basic) | Alanine | Ala | A | Uncharged (hydrophobic) |
| Histidine | His | H | Uncharged (polar) | Glycine | Gly | G | Uncharged (hydrophobic) |
| Asparagine | Asn | N | Uncharged (polar) | Valine | Val | V | Uncharged (hydrophobic) |
| Glutamine | Gln | Q | Uncharged (polar) | Leucine | Leu | L | Uncharged (hydrophobic) |
| Serine | Ser | S | Uncharged (polar) | Isoleucine | Ile | I | Uncharged (hydrophobic) |
| Threonine | Thr | T | Uncharged (polar) | Proline | Pro | P | Uncharged (hydrophobic) |
| Tyrosine | Tyr | Y | Uncharged (polar) | Phenylalanine | Phe | F | Uncharged (hydrophobic) |

The following amino acids are considered charged amino acids: aspartic acid (negative), glutamic acid (negative), arginine (positive), and lysine (positive).

At a pH of 7.4, the side chains of aspartic acid (pKa 3.1) and glutamic acid (pKa 4.1) have a negative charge, while the side chains of arginine (pKa 12.5) and lysine (pKa 10.8) have a positive charge. Aspartic acid and glutamic acid are referred to as acidic amino acid residues. Arginine and lysine are referred to as basic amino acid residues.

The following amino acids are considered uncharged, polar (meaning they can participate in hydrogen bonding) amino acids: asparagine, glutamine, histidine, serine, threonine, tyrosine, cysteine, methionine, and tryptophan. The following amino acids are considered uncharged, hydrophobic amino acids: alanine, valine, leucine, isoleucine, phenylalanine, proline, and glycine.

In an amino acid insertion, an additional amino acid residue (one that is not normally present) is incorporated into the BoNT/A polypeptide sequence, thus increasing the total number of amino acid residues in said sequence. In an amino acid deletion, an amino acid residue is removed from the BoNT/A amino acid sequence, thus reducing the total number of amino acid residues in said sequence.

Preferably, the modification is a substitution, which advantageously maintains the same number of amino acid residues in the modified BoNT/A. In an amino acid substitution, an amino acid residue that forms part of the BoNT/A polypeptide sequence is replaced with a different amino acid residue. The replacement amino acid residue may be one of the 20 standard amino acids, as described above. Alternatively, the replacement amino acid in an amino acid substitution may be a non-standard amino acid (an amino acid that is not part of the standard set of 20 described above). By way of example, the replacement amino acid may be a basic non-standard amino acid, e.g. L-Ornithine, L-2-amino-3-guanidinopropionic acid, or D-isomers of Lysine, Arginine and Ornithine). Methods for introducing non-standard amino acids into proteins are known in the art and include recombinant protein synthesis using *E. coli* auxotrophic expression hosts.

In one embodiment, the substitution is selected from: substitution of an acidic amino acid residue with a basic amino acid residue, substitution of an acidic amino acid residue with an uncharged amino acid residue, and substitution of an uncharged amino acid residue with a basic amino acid residue. In one embodiment, wherein the substitution is a substitution of an acidic amino acid residue with an uncharged amino acid residue, the acidic amino acid residue is replaced with its corresponding uncharged amide amino acid residue (i.e. aspartic acid is replaced with asparagine, and glutamic acid is replaced with glutamine).

Preferably, the basic amino acid residue is a lysine residue or an arginine residue. In other words, the substitution is substitution with lysine or arginine. Most preferably, the modification is substitution with lysine.

Following modification in accordance with the invention, the modified BoNT/A is capable of binding to the target cell receptors that unmodified BoNT/A (e.g. SEQ ID NO: 1) binds.

Preferably, the BoNT/A (e.g. for use as described herein) is part of a pharmaceutical composition together with at least one pharmaceutically acceptable carrier. By "pharmaceutically acceptable carrier", it is meant herein any component that is compatible with the other ingredients of the pharmaceutical composition, in particular with the BoNT/A , and which is not deleterious to the human patient. The pharmaceutically acceptable carrier can be selected on the basis of the desired route of administration, in accordance with standard pharmaceutical practices, and include, without limitation, excipients, diluents, adjuvants, propellants and salts.

Accordingly, the present disclosure further relates to a pharmaceutical composition for use in the treatment of post-operative surgical pain (and optionally post-operative anxiety caused by post-operative pain) in a human patient as defined in the claims, wherein said composition comprises the BoNT/A of the invention and at least one pharmaceutically acceptable carrier.

The BoNT/A for use according to the present invention may preferably be formulated for intradermal administration.

A route of administration is via intradermal administration. Preferably, intradermal administration means intradermal injection.

Preferably, said BoNT
is a purified BoNT. As used herein, the term "purified BoNT" means a botulinum neurotoxin purified from a clostridial strain which naturally produces it (naturally-occurring clostridial strain) or purified using recombinant technology. The purified BoNT/A may be associated with complexing proteins or free of complexing proteins, but is preferably free of complexing proteins. Thus, in one embodiment, the BoNT/A is associated with BoNT complexing proteins, also known as non-toxic neurotoxin-associated proteins (NAP).

Hence, in one embodiment the BoNT/A is complexed with one or more BoNT complexing proteins. Examples of commercially available purified and complexing protein-associated BoNT/A include Botox®, Dysport® (associated with BoNT complexing proteins) and Xeomin® (purified).

In another embodiment, the BoNT/A is free of (or not associated with, or combined with) BoNT complexing proteins.

The doses of BoNT/A may be measured in nanograms.

Doses of BoNT/A according to the invention are to be understood as doses of active di-chain BoNT/A , i.e. without including the quantity of complexing proteins to which the neurotoxin may be associated with.

As well-known to the skilled practitioner, an active di-chain BoNT/A is capable of binding to a membrane (e.g. cell membrane) receptor, translocating the light chain into the cytoplasm and of cleaving a SNARE protein, while complexing proteins do not display such biological activity (i.e. are not "active").

Additionally or alternatively, the doses of BoNT/A may be measured in "Units" (U) of clostridial neurotoxin. For example, the measurement of doses in Units may be particularly suitable when administering BoNT/A (or more particularly, for example, Dysport®).

Indeed, as well known to the skilled practitioner, the potency of a BoNT/A is related to the quantity (e.g. nanograms) of neurotoxin required to achieve an LD50 (lethal dose 50) unit; one LD50 unit being defined as the median lethal intraperitoneal dose (as measured in mice). However, BoNT pharmaceutical preparations currently on the market contain different amount of 150 kD neurotoxin, but also of LD50 Units. Besides, in these preparations, the neurotoxin may, or may not, be associated with (i.e. combined with) non-toxic neurotoxin-associated proteins (NAP), also known as complexing proteins. For ease of conversion (as reported in Field et. al, "AbobotulinumtoxinA (Dysport®), OnabotulinumtoxinA (Botox®), and IncobotulinumtoxinA (Xeomin®) Neurotoxin Content and Potential Implications for Duration of Response in Patients". Toxins 2018, 10(12), 535):

- 100 Units of Botox® (also known as OnabotulinumtoxinA) contains about 0.9 ng of 150 kD BoNT/A, as well as complexing proteins;
- 500 Units of Dysport® (also known as AbobotulinumtoxinA) contains about 2.69 ng of 150 kD BoNT/A, as well as complexing proteins; 1 Unit of Dysport® contains about 5.38 pg BoNT/A;
- 100 Units of Xeomin® (also known as IncobotulinumtoxinA) contains about 0.40 ng of 150 kD BoNT/A, with no complexing proteins.

It should be noted that conversion values may vary slightly. For example, conversion values reported in Frevert, 2012 ("Content of botulinum neurotoxin in Botox®/Vistabel®, Dysport®/Azzalure®, and Xeomin®/Bocouture®"; Drugs R D. 2010;10(2):67-73) are as follows:

- 100 Units of Botox® (also known as OnabotulinumtoxinA) contains about 0.73 ng of 150 kD BoNT/A, as well as complexing proteins;
- 100 Units of Dysport® (also known as AbobotulinumtoxinA) contains about 0.65 ng of 150 kD BoNT/A, as well as complexing proteins;
- 100 Units of Xeomin® (also known as IncobotulinumtoxinA) contains about 0.44 ng of 150 kD BoNT/A, with no complexing proteins;
- 100 Units of Neurobloc/Myobloc® (also known as RimabotulinumtoxinB) contains about 0.2 ng to about 1 ng of 150 kD BoNT/B, as well as complexing proteins.

The quantity of BoNT/A can be measured by the skilled practitioner according to methods conventionally used in the art to quantify proteins preferably at nanograms levels, including, among others, mass spectroscopy such as isotopic dilution mass spectroscopy (Muñoz et al., Quantification of protein calibrants by amino acid analysis using isotope dilution mass spectrometry, Anal. Biochem. 2011, 408, 124-131), or fluorimetric assay (Poras et al., Detection and Quantification of Botulinum Neurotoxin Type A by a Novel Rapid In Vitro Fluorimetric Assay, Appl Environ Microbiol. 2009 Jul; 75(13): 4382-4390).

Intradermal administration may comprise intradermal injection with a needle, such as a 30 gauge needle, preferably wherein the needle (such as a 30 gauge needle) is inserted into dermis of the skin at an angle of about 5°-15° relative to the surface of the skin at the site of surgical intervention (which may be on the flank). The depth of the injection (depth relative to the surface of the skin) may be around 0.2-0.3 (preferably around 0.25) inches.

In one embodiment following administration, the BoNT/A travels by retrograde transport to the spinal cord and effects SNARE protein cleavage (SNAP-25 protein cleavage) in said spinal cord.

In one embodiment when a BoNT/A is administered at an intradermal site, minimal or no SNARE protein cleavage (SNAP-25 protein cleavage) by said BoNT/A is observed at or proximal to said intradermal site following administration of the BoNT/A.

In one embodiment observations were made 5-7 days following administration of the BoNT/A and minimal or no SNARE protein cleavage (SNAP-25 protein cleavage) by said BoNT/A is observed at or proximal to said intradermal site following administration of the BoNT/A.

Thus, a BoNT/A may be administered distal to a site of surgical intervention to treat post-operative surgical pain and optionally post-operative anxiety.

The BoNT/A may be administered at a site distal to the site of surgical intervention (e.g. at one or more administration sites distal to the site of incision on the patient).

The BoNT/A may be administered at one or more sites distal to the site of surgical intervention, for example at a site at least 15 cm from the site of surgical intervention; at least 50 cm from the site of surgical intervention or at least 100 cm from the site of surgical intervention.

The skilled person would understand that the invention is directed to pre-surgery administration, such that the reference to administration "at or proximal to the site of surgical intervention" refers to administration at or proximal to a site that will be (e.g. subsequently) subjected to surgical intervention once surgery commences. The reference to administration "at a site distal to the site of surgical intervention" refers to administration distal to a site that will be (e.g. subsequently) subjected to surgical intervention once surgery commences.

The BoNT/A may be administered at up to 15 (preferably up to 10) sites (e.g. sites proximal to the site of surgical intervention). Such sites may traverse the periphery of the site of surgical intervention.

In a preferred embodiment, the dose of BoNT/A to be administered for treating surgical pain in a human patient (i.e. therapeutic dose) is ranging from about 0.00025 ng to about 3 ng.

In a preferred embodiment, the therapeutic dose of the BoNT/A is ranging from about 0.0003 ng to about 2 ng, preferably from about 0.0004 ng to about 1.5 ng, from about 0.0005 ng to about 1 ng, still preferably from about 0.0006 ng to about 0.5 ng of said BoNT/A.

For example, the dose (e.g. total dose) of the BoNT/A is preferably ranging from about 1 ng to about 2 ng.

The patient may be administered 80-250 picograms (pg) of the BoNT/A per kilogram (kg) of the patient's bodyweight (e.g. 850-250 pg/kg). For example, the patient may be administered 100-200 pg/kg, 115-175 pg/kg, or 130-150 pg/kg.

The patient may be administered the BoNT/A at a total dose of 10-170 pg per administration site. In a preferable embodiment, the patient may be administered the BoNT/A at a dose of 1-14 pg/kg (bodyweight) per administration site.

In another embodiment the therapeutic dose of the BoNT/A is preferably ranging from about 0.001 ng to about 2 ng. Yet, for example, the therapeutic dose of the BoNT/A is preferably ranging from about 0.0003 ng to about 0.05 ng.

It will nevertheless be appreciated that the dose range required depends on the precise nature of the BoNT/A, the maximum tolerated dose in the particular subject (e.g. human subject), the skin condition, the route of administration, the nature of the formulation, the age of the patient, the weight of the patient, the nature, extent or severity of the patient's condition, contraindications, if any, and the judgement of the attending physician. Variations in these dosage levels can be adjusted using standard empirical routines for optimisation.

In one embodiment, a patient is administered a monotherapy based on a single botulinum neurotoxin serotype (e.g. BoNT/A). Thus in one embodiment, the present invention employs the use of a single botulinum neurotoxin serotype (e.g. BoNT/A).

## SEQUENCE HOMOLOGY

Any of a variety of sequence alignment methods can be used to determine percent identity, including, without limitation, global methods, local methods and hybrid methods, such as, e.g., segment approach methods. Protocols to determine percent identity are routine procedures within the scope of one skilled in the art. Global methods align sequences from the beginning to the end of the molecule and determine the best alignment by adding up scores of individual residue pairs and by imposing gap penalties. Non-limiting methods include, e.g., CLUSTAL W, see, e.g., Julie D. Thompson et al., CLUSTAL W: Improving the Sensitivity of Progressive Multiple Sequence Alignment Through Sequence Weighting, Position- Specific Gap Penalties and Weight Matrix Choice, 22(22) Nucleic Acids Research 4673-4680 (1994); and iterative refinement, see, e.g., Osamu Gotoh, Significant Improvement in Accuracy of Multiple Protein. Sequence Alignments by Iterative Refinement as Assessed by Reference to Structural Alignments, 264(4) J. Mol. Biol. 823-838 (1996). Local methods align sequences by identifying one or more conserved motifs shared by all of the input sequences. Non-limiting methods include, e.g., Match-box, see, e.g., Eric Depiereux and Ernest Feytmans, Match-Box: A Fundamentally New Algorithm for the Simultaneous Alignment of Several Protein Sequences, 8(5) CABIOS 501 -509 (1992); Gibbs sampling, see, e.g., C. E. Lawrence et al., Detecting Subtle Sequence Signals: A Gibbs Sampling Strategy for Multiple Alignment, 262(5131 ) Science 208-214 (1993); Align-M, see, e.g., Ivo Van Walle et al., Align-M - A New Algorithm for Multiple Alignment of Highly Divergent Sequences, 20(9) Bioinformatics:1428-1435 (2004).

Thus, percent sequence identity is determined by conventional methods. See, for example, Altschul et al., Bull. Math. Bio. 48: 603-16, 1986 and Henikoff and Henikoff, Proc. Natl. Acad. Sci. USA 89:10915-19, 1992. Briefly, two amino acid sequences are aligned to optimize the alignment scores using a gap opening penalty of 10, a gap extension penalty of 1, and the "blosum 62" scoring matrix of Henikoff and Henikoff (ibid.) as shown below (amino acids are indicated by the standard one-letter codes).

The "percent sequence identity" between two or more nucleic acid or amino acid sequences is a function of the number of identical positions shared by the sequences. Thus, % identity may be calculated as the number of identical nucleotides / amino acids divided by the total number of nucleotides / amino acids, multiplied by 100. Calculations of % sequence identity may also take into account the number of gaps, and the length of each gap that needs to be introduced to optimize alignment of two or more sequences. Sequence comparisons and the determination of percent identity between two or more sequences can be carried out using specific mathematical algorithms, such as BLAST, which will be familiar to a skilled person.

ALIGNMENT SCORES FOR DETERMINING SEQUENCE IDENTITY

```
        A  R  N  D  C  Q  E  G  H  I  L  K  M  F  P  S  T  W  Y  V
A  4
R -1  5
N -2  0  6
D -2 -2  1  6
C  0 -3 -3 -3  9
Q -1  1  0  0 -3  5
E -1  0  0  2 -4  2  5
G  0 -2  0 -1 -3 -2 -2  6
H -2  0  1 -1 -3  0  0 -2  8
I -1 -3 -3 -3 -1 -3 -3 -4 -3  4
L -1 -2 -3 -4 -1 -2 -3 -4 -3  2  4
K -1  2  0 -1 -3  1  1 -2 -1 -3 -2  5
M -1 -1 -2 -3 -1  0 -2 -3 -2  1  2 -1  5
F -2 -3 -3 -3 -2 -3 -3 -3 -1  0  0 -3  0  6
P -1 -2 -2 -1 -3 -1 -1 -2 -2 -3 -3 -1 -2 -4  7
S  1 -1  1  0 -1  0  0  0 -1 -2 -2  0 -1 -2 -1  4
T  0 -1  0 -1 -1 -1 -1 -2 -2 -1 -1 -1 -1 -2 -1  1  5
W -3 -3 -4 -4 -2 -2 -3 -2 -2 -3 -2 -3 -1  1 -4 -3 -2 11
Y -2 -2 -2 -3 -2 -1 -2 -3  2 -1 -1 -2 -1  3 -3 -2 -2  2  7

V  0 -3 -3 -3 -1 -2 -2 -3 -3  3  1 -2  1 -1 -2 -2  0 -3 -1  4
```

The percent identity is then calculated as:

$$\frac{\text{Total number of identical matches}}{[\text{length of the longer sequence plus the number of gaps introduced into the longer sequence in order to align the two sequences}]} \times 100$$

Substantially homologous polypeptides are characterized as having one or more amino acid substitutions, deletions or additions. These changes are preferably of a minor nature, that is conservative amino acid substitutions (see below) and other substitutions that do not significantly affect the folding or activity of the polypeptide; small deletions, typically of one to about 30 amino acids; and small amino- or carboxyl-terminal extensions, such as an amino-terminal methionine residue, a small linker peptide of up to about 20-25 residues, or an affinity tag.

CONSERVATIVE AMINO ACID SUBSTITUTIONS

| | |
|---|---|
| Basic: | arginine; lysine; histidine |
| Acidic: | glutamic acid; aspartic acid |
| Polar: | glutamine; asparagine |
| Hydrophobic: | leucine; isoleucine; valine |

(continued)

| | |
|---|---|
| Aromatic: | phenylalanine; tryptophan; tyrosine |
| Small: | glycine; alanine; serine; threonine; methionine |

In addition to the 20 standard amino acids, non-standard amino acids (such as 4-hydroxyproline, 6-N-methyl lysine, 2-aminoisobutyric acid, isovaline and $\alpha$ -methyl serine) may be substituted for amino acid residues of the polypeptides of the present invention. A limited number of non-conservative amino acids, amino acids that are not encoded by the genetic code, and unnatural amino acids may be substituted for polypeptide amino acid residues. The polypeptides of the present invention can also comprise non-naturally occurring amino acid residues.

Non-naturally occurring amino acids include, without limitation, trans-3-methylproline, 2,4-methano-proline, cis-4-hydroxyproline, trans-4-hydroxy-proline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins. For example, an in vitro system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is carried out in a cell free system comprising an E. coli S30 extract and commercially available enzymes and other reagents. Proteins are purified by chromatography. See, for example, Robertson et al., J. Am. Chem. Soc. 113:2722, 1991; Ellman et al., Methods Enzymol. 202:301, 1991; Chung et al., Science 259:806-9, 1993; and Chung et al., Proc. Natl. Acad. Sci. USA 90:10145-9, 1993). In a second method, translation is carried out in Xenopus oocytes by microinjection of mutated mRNA and chemically aminoacylated suppressor tRNAs (Turcatti et al., J. Biol. Chem. 271:19991-8, 1996). Within a third method, E. coli cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the polypeptide in place of its natural counterpart. See, Koide et al., Biochem. 33:7470-6, 1994. Naturally occurring amino acid residues can be converted to non-naturally occurring species by in vitro chemical modification. Chemical modification can be combined with site-directed mutagenesis to further expand the range of substitutions (Wynn and Richards, Protein Sci. 2:395-403, 1993).

A limited number of non-conservative amino acids, amino acids that are not encoded by the genetic code, non-naturally occurring amino acids, and unnatural amino acids may be substituted for amino acid residues of polypeptides of the present invention.

Essential amino acids in the polypeptides of the present invention can be identified according to procedures known in the art, such as site-directed mutagenesis or alanine-scanning mutagenesis (Cunningham and Wells, Science 244: 1081-5, 1989). Sites of biological interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-12, 1992; Smith et al., J. Mol. Biol. 224:899-904, 1992; Wlodaver et al., FEBS Lett. 309:59-64, 1992. The identities of essential amino acids can also be inferred from analysis of homologies with related components (e.g. the translocation or protease components) of the polypeptides of the present invention.

Multiple amino acid substitutions can be made and tested using known methods of mutagenesis and screening, such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide the skilled person with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure.

Amino acids are referred to herein using the name of the amino acid, the three letter abbreviation or the single letter abbreviation. The term "protein", as used herein, includes proteins, polypeptides, and peptides. As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme". The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

Other definitions of terms may appear throughout the specification. Before the exemplary embodiments are described in more detail, it is to be understood that this disclosure is not limited to particular embodiments described, and as such may vary. It is also to be understood that the terminology used herein is for the purpose of describing particular embodiments only, and is not intended to be limiting, since the scope of the present disclosure will be defined only by the appended claims.

Where a range of values is herein provided, it shall be understood that, unless the context clearly dictates otherwise, each intervening value to the tenth of the unit between the upper and lower limits of that range is also specifically disclosed. Each smaller range between any stated value or intervening value in a stated range and any other stated or intervening value in that stated range is encompassed within this disclosure. It shall be further understood that any range of numerical values denoted herein by the expression "from a to b" means the range of numerical values extending from a to b (i.e. including the strict end points a and b).

Besides, the term "about" shall be understood herein as plus or minus ($\pm$) 5%, preferably $\pm$ 4%, $\pm$ 3%, $\pm$ 2%, $\pm$ 1%, $\pm$ 0.5%, $\pm$ 0.1%, of the numerical value of the number with which it is being used.

It must be noted that as used herein and in the appended claims, the singular forms "a", "an", and "the" include plural referents unless the context clearly dictates otherwise. Thus, for example, reference to "a botulinum neurotoxin" includes a plurality of such candidate agents and reference to "the botulinum neurotoxin" includes reference to one or more clostridial neurotoxins and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

## BRIEF DESCRIPTION OF THE DRAWINGS

Embodiments of the invention will now be described, by way of example only, with reference to the following Figures and Examples.

**Figure 1** shows a schematic of injection sites along the surgical incision, in which Dysport, saline or the reference compound Exparel were injected.

**Figure 2** shows a schematic of the arena used for the open field test for locomotion activity.

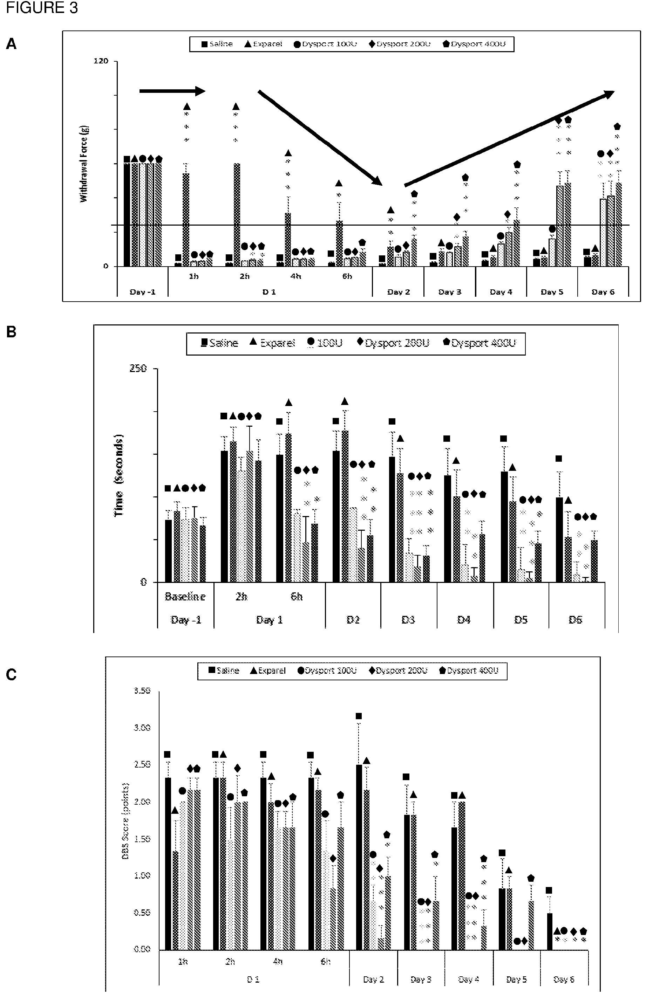

**Figure 3** shows the effects from peri-operative administration of intradermal injections of either saline, Exparel (control agent) or different concentrations of Dysport (100, 200 and 400U) on mitigating post-operative surgical pain and anxiety. A Von Frey assay as a measure of surgical pain perception was conducted and results are shown in (A). The horizontal line cutting across the graph, set at 26g, indicates the baseline for not sensing surgical pain (e.g. a threshold, above which the subject's post-operative surgical pain may be considered treated). Pain can be defined as moderate/severe when the mechanical sensitivity is 0-15g, mild/moderate when between 15-26g and little/no pain is sensed when above 26g. The time taken (in seconds) for pigs to approach their handlers following peri-operative, intradermal administration of Exparel or Dysport is shown in (B). The distress behaviour was scored following peri-operative administration of Exparel or Dysport and is shown in (C).

**Figure 4** shows the mean group total walking distance (in metres) that the animals walked in a period of 5 minutes (A) and the mean percentage of time spent in the central zone of the open field apparatus (B) following the peri-operative intradermal injection of either saline, Exparel (control agent) or different concentrations of Dysport (100, 200 and 400U).

**Figure 5** shows a Von Frey assay of saline (control) versus BoNT/A (Dysport) intradermal administration at 15 days (A), 5 days (B) or 1 day (C) prior to surgery.

**Figure 6** shows the latency (in seconds) of pigs to approach their handler following the intradermal administration of either saline or Dysport (200U/pig) at 15 days (A), 5 days (B) or 1 day (C) prior to surgery. For each timepoint (day) in the bar charts (A-C), the bar on the left (lighter) shows results for Dysport treatment, and the bar on the right (darker) shows results for saline treatment.

**Figure 7** shows the distress behaviour score of pigs following the intradermal administration of either saline or Dysport at 15 days (A), 5 days (B) or 1 day (C) prior to surgery. For each timepoint (day) in the bar charts (A-C), the bar on the left (lighter) shows results for Dysport treatment, and the bar on the right (darker) shows results for saline treatment.

**Figure 8** shows the total walking distance (in meters) of pigs following the intradermal administration of either saline or Dysport at 15 days (A), 5 days (B) or 1 day (C) prior to surgery.

**Figure 9** shows the time course of time spent in the central zone of an open-field apparatus in a period of 5 minutes (Individual values and Median) following the intradermal administration of either saline or Dysport at 15 days (A), 5 days (B) or 1 day (C) prior to surgery.

**Figure 10** shows a Von Frey assay of saline versus BoNT/A (Dysport) when administered via intradermal (A), subcutaneous (B) or intramuscular (C) injections. For each injection route tested, a total of 200U of Dysport per pig was administered.

**Figure 11** shows the latency (in seconds) of pigs to approach their handler when administered saline or BoNT/A (Dysport) via intradermal (A), subcutaneous (B) or intramuscular (C) injections. For each injection route tested, a total of 200U of Dysport per pig was administered.

**Figure 12** shows the distress behaviour score of pigs when administered saline or BoNT/A (Dysport) via intradermal (a), subcutaneous (b) or intramuscular (c) injections. For each injection route tested, a total of 200U of Dysport per pig was administered.

**Figure 13** shows the time course of total distance (in metres) that the animals walked in a period of 5 minutes in open-field following intradermal, subcutaneous or intramuscular administration of saline or Dysport (Individual values and Mean±SEM).

**Figure 14** shows the time course of time (percentage) spent in the central zone of an open-field apparatus following the intradermal, subcutaneous or intramuscular administration of saline or Dysport (Individual values and Mean ±SEM).

**Figure 15** shows immunohistochemistry staining of SNAP-25 in skin samples with small nerves around arterioles (A), nerve endings in the hair erector muscles (B) and small-middle sized nerves in the dermis (C).

**Figure 16** shows immunohistochemistry staining of cleaved SNAP-25 in the spinal cord of a pig when untreated (A) and cleaved SNAP-25 staining in the ipsilateral horn (B) or contralateral horn (C) of a pig treated with Dysport.

**Figure 17** shows the expression levels of calcitonin gene related peptide (CGRP) and Substance P in the spinal cord of a pig when either untreated (A, C) or administered an intradermal injection of Dysport (B, D).

**Figure 18** shows the expression levels of Iba1 (A, B) and glial fibrillary acidic protein (GFAP) (C, D) in the spinal cord of a pig when untreated or when administered with intradermal injections of Dysport.

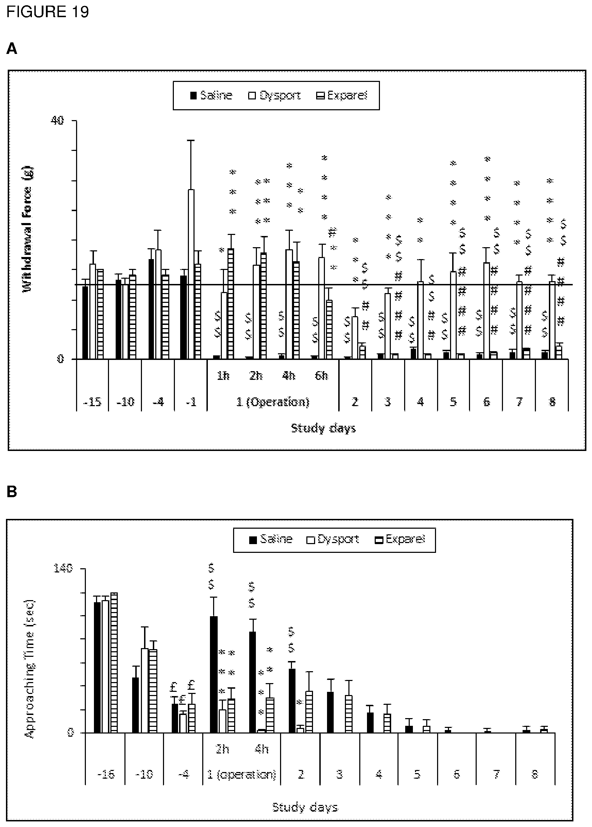

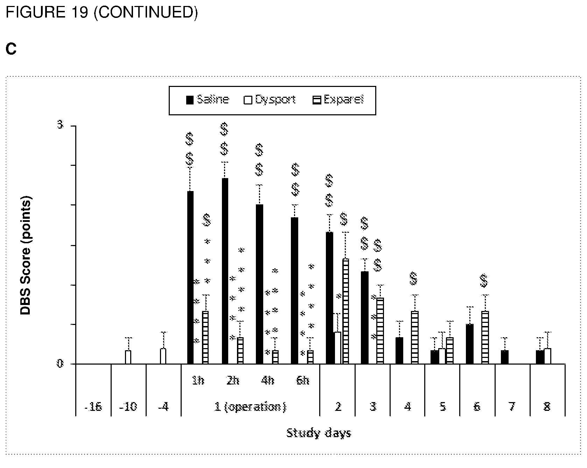

**Figure 19** shows a Von Frey assay of either saline (control) or BoNT/A (Dysport) intradermal administration at 15 days prior to surgery or Exparel intradermal administration on the day of surgery (D1), and when pigs are subjected to a surgical incision in the left leg (A) * p<0.05; ** p<0.01; *** p<0.001; ****p<0.0001 vs. saline group using one way ANOVA followed by Tukey test. #p<0.05; ## p<0.01 ####p<0.0001 Dysport vs. Exparel group using one way ANOVA followed by Tukey test. $$ p<0.01: post-surgery timepoint vs. Day -4 using paired T-test). (B) shows the latency (in seconds) of pigs to approach their handler following the intradermal administration of either saline (control) or BoNT/A (Dysport) intradermal administration at 15 days prior to surgery or Exparel intradermal administration on the day of surgery (D1), and when pigs are subjected to a surgical incision in the left leg ($$ p<0.01: post-surgery timepoint vs. Day -4 using paired T-test. £££ p<0.001: Day -16 vs. Day -4 using paired T-test. *p<0.05; ** p<0.01; ***p<0.001 treatment vs. saline group using one-way ANOVA followed by Tukey test). (C) shows the distress behaviour score of pigs following the intradermal administration of either saline (control) or BoNT/A (Dysport) intradermal administration at 15 days prior to surgery or Exparel intradermal administration on the day of surgery (D1), and when pigs are subjected to a surgical incision in the left leg (*p<0.05, ***p<0.001 and ****p<0.0001 vs. saline group using one way ANOVA followed by Tukey test. $$ p<0.01, $p<0.05: post-surgery timepoint vs. Day -4 using paired T-test).

**Figure 20** shows a summary of the tissue samples (formalin-fixed-paraffin-embedded tissues) that were collected for immunohistochemistry staining and the specific regions where tissue samples were collected from the spinal cord.

**Figure 21** shows the immunohistochemistry staining for cleaved SNAP-25 in the skin (A), muscle (B), and dorsal root ganglia (C) in pigs with a surgical incision in the left leg.

**Figure 22** shows cleaved SNAP-25 staining in the ipsilateral dorsal horn of lumbar L5-L6 in the spinal cord of pigs with a surgical incision to the left leg (A) and a magnified view (B).

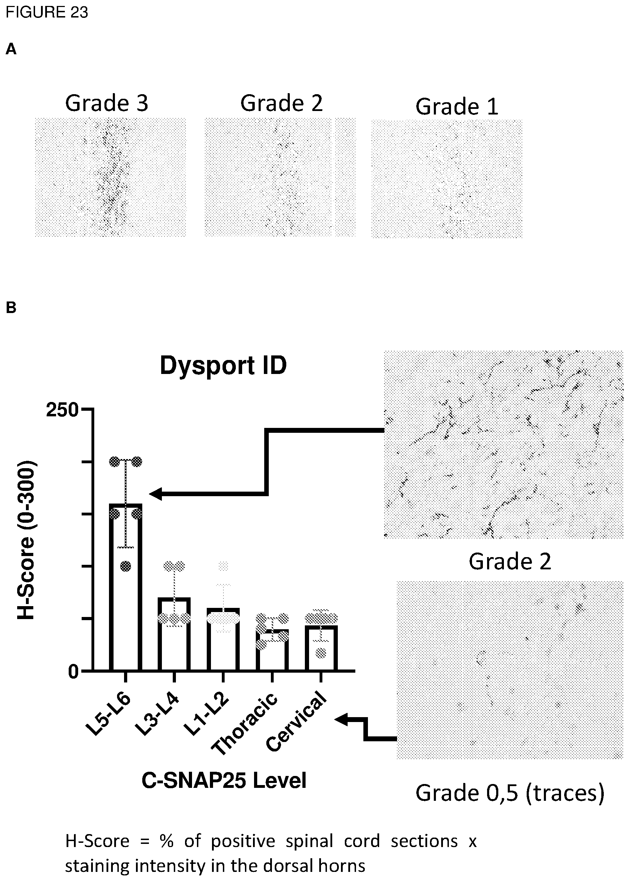

**Figure 23** shows a grading scale used to determine the intensity of cleaved SNAP-25 staining. Cleaved SNAP-25 staining was graded on a scale of 1-3, with grade 0 = no cleaved SNAP-25 staining, grade 1 = low intensity cleaved SNAP-25 staining, grade 2 = average cleaved SNAP-25 intensity staining and grade 3 = high intensity cleaved SNAP-25 staining (A). Figure 23 also shows the quantification of staining intensity for different regions of the spinal cord: lumbar L5-L6, L3-L4, L1-L2 and the thoracic and cervical regions. Staining intensity was measured by way of a "H-Score" and calculated by % of positive spinal cord sections x staining intensity in the dorsal horns (B).

**Figure 24** provides a summary of the presence, "positive" or absence, "negative" of cleaved SNAP-25 staining in collected tissue samples.

## SEQUENCE LISTING

Where an initial Met amino acid residue or a corresponding initial codon is indicated in any of the following SEQ ID NOs, said residue/codon is optional.

SEQ ID NO: 1 - BoNT/A1, accession number A5HZZ9, amino acid sequence

```
MPFVNKQFNYKDPVNGVDIAYIKIPNAGQMQPVKAFKIHNKIWVIPERDTFTNPEEGDLNPPPEAKQVPVSYYDSTYLST
DNEKDNYLKGVTKLFERIYSTDLGRMLLTSIVRGIPFWGGSTIDTELKVIDTNCINVIQPDGSYRSEELNLVIIGPSADI
IQFECKSFGHEVLNLTRNGYGSTQYIRFSPDFTFGFEESLEVDTNPLLGAGKFATDPAVTLAHELIHAGHRLYGIAINPN
RVFKVNTNAYYEMSGLEVSFEELRTFGGHDAKFIDSLQENEFRLYYYNKFKDIASTLNKAKSIVGTTASLQYMKNVFKEK
YLLSEDTSGKFSVDKLKFDKLYKMLTEIYTEDNFVKFFKVLNRKTYLNFDKAVFKINIVPKVNYTIYDGFNLRNTNLAAN
FNGQNTEINNMNFTKLKNFTGLFEFYKLLCVRGIITSKTKSLDKGYNKALNDLCIKVNNWDLFFSPSEDNFTNDLNKGEE
ITSDTNIEAAEENISLDLIQQYYLTFNFDNEPENISIENLSSDIIGQLELMPNIERFPNGKKYELDKYTMFHYLRAQEFE
HGKSRIALTNSVNEALLNPSRVYTFFSSDYVKKVNKATEAAMFLGWVEQLVYDFTDETSEVSTTDKIADITIIIPYIGPA
LNIGNMLYKDDFVGALIFSGAVILLEFIPEIAIPVLGTFALVSYIANKVLTVQTIDNALSKRNEKWDEVYKYIVTNWLAK
VNTQIDLIRKKMKEALENQAEATKAIINYQYNQYTEEEKNNINFNIDDLSSKLNESINKAMININKFLNQCSVSYLMNSM
IPYGVKRLEDFDASLKDALLKYIYDNRGTLIGQVDRLKDKVNNTLSTDIPFQLSKYVDNQRLLSTFTEYIKNIINTSILN
LRYESNHLIDLSRYASKINIGSKVNFDPIDKNQIQLFNLESSKIEVILKNAIVYNSMYENFSTSFWIRIPKYFNSISLNN
EYTIINCMENNSGWKVSLNYGEIIWTLQDTQEIKQRVVFKYSQMINISDYINRWIFVTITNNRLNNSKIYINGRLIDQKP
ISNLGNIHASNNIMFKLDGCRDTHRYIWIKYFNLFDKELNEKEIKDLYDNQSNSGILKDFWGDYLQYDKPYYMLNLYDPN
KYVDVNNVGIRGYMYLKGPRGSVMTTNIYLNSSLYRGTKFIIKKYASGNKDNIVRNNDRVYINVVVKNKEYRLATNASQA
GVEKILSALEIPDVGNLSQVVVMKSKNDQGITNKCKMNLQDNNGNDIGFIGFHQFNNIAKLVASNWYNRQIERSSRTLGC
SWEFIPVDDGWGERPL
```

SEQ ID NO: 2 - BoNT/B1, accession number B1INP5, amino acid sequence

```
MPVTINNFNYNDPIDNNNIIMMEPPFARGTGRYYKAFKITDRIWIIPERYTFGYKPEDFNKSSGIFNRDVCEYYDPDYLN
TNDKKNIFLQTMIKLFNRIKSKPLGEKLLEMIINGIPYLGDRRVPLEEFNTNIASVTVNKLISNPGEVERKKGIFANLII
FGPGPVLNENETIDIGIQNHFASREGFGGIMQMKFCPEYVSVFNNVQENKGASIFNRRGYFSDPALILMHELIHVLHGLY
GIKVDDLPIVPNEKKFFMQSTDAIQAEELYTFGGQDPSIITPSTDKSIYDKVLQNFRGIVDRLNKVLVCISDPNININIY
KNKFKDKYKFVEDSEGKYSIDVESFDKLYKSLMFGFTETNIAENYKIKTRASYFSDSLPPVKIKNLLDNEIYTIEEGFNI
SDKDMEKEYRGQNKAINKQAYEEISKEHLAVYKIQMCKSVKAPGICIDVDNEDLFFIADKNSFSDDLSKNERIEYNTQSN
YIENDFPINELILDTDLISKIELPSENTESLTDFNVDVPVYEKQPAIKKIFTDENTIFQYLYSQTFPLDIRDISLTSSFD
DALLFSNKVYSFFSMDYIKTANKVVEAGLFAGWVKQIVNDFVIEANKSNTMDKIADISLIVPYIGLALNVGNETAKGNFE
NAFEIAGASILLEFIPELLIPVVGAFLLESYIDNKNKIIKTIDNALTKRNEKWSDMYGLIVAQWLSTVNTQFYTIKEGMY
KALNYQAQALEEIIKYRYNIYSEKEKSNINIDFNDINSKLNEGINQAIDNINNFINGCSVSYLMKKMIPLAVEKLLDFDN
TLKKNLLNYIDENKLYLIGSAEYEKSKVNKYLKTIMPFDLSIYTNDTILIEMFNKYNSEILNNIILNLRYKDNNLIDLSG
YGAKVEVYDGVELNDKNQFKLTSSANSKIRVTQNQNIIFNSVFLDFSVSFWIRIPKYKNDGIQNYIHNEYTIINCMKNNS
GWKISIRGNRIIWTLIDINGKTKSVFFEYNIREDISEYINRWFFVTITNNLNNAKIYINGKLESNTDIKDIREVIANGEI
IFKLDGDIDRTQFIWMKYFSIFNTELSQSNIEEERYKIQSYSEYLKDFWGNPLMYNKEYYMFNAGNKNSYIKLKKDSPVGE
ILTRSKYNQNSKYINYRDLYIGEKFIIRRKSNSQSINDDIVRKEDYIYLDFFNLNQEWRVYTYKYFKKEEEKLFLAPISD
SDEFYNTIQIKEYDEQPTYSCQLLFKKDEESTDEIGLIGIHRFYESGIVFEEYKDYFCISKWYLKEVKRKPYNLKLGCNW
QFIPKDEGWTE
```

SEQ ID NO: 3 - BoNT/C1, accession number P18640, amino acid sequence

```
MPITINNFNYSDPVDNKNILYLDTHLNTLANEPEKAFRITGNIWVIPDRFSRNSNPNLNKPPRVTSPKSGYYDPNYLSTD
SDKDPFLKEIIKLFKRINSREIGEELIYRLSTDIPFPGNNNTPINTFDFDVDFNSVDVKTRQGNNWVKTGSINPSVIITG
PRENIIDPETSTFKLTNNTFAAQEGFGALSIISISPRFMLTYSNATNDVGEGRFSKSEFCMDPILILMHELNHAMHNLYG
IAIPNDQTISSVTSNIFYSQYNVKLEYAEIYAFGGPTIDLIPKSARKYFEEKALDYYRSIAKRLNSITTANPSSFNKYIG
EYKQKLIRKYRFVVESSGEVTVNRNKFVELYNELTQIFTEFNYAKIYNVQNRKIYLSNVYTPVTANILDDNVYDIQNGFN
IPKSNLNVLFMGQNLSRNPALRKVNPENMLYLFTKFCHKAIDGRSLYNKTLDCRELLVKNTDLPFIGDISDVKTDIFLRK
DINEETEVIYYPDNVSVDQVILSKNTSEHGQLDLLYPSIDSESEILPGENQVFYDNRTQNVDYLNSYYYLESQKLSDNVE
DFTFTRSIEEALDNSAKVTYFPTLANKVNAGVQGGLFLMWANDVVEDFTTNILRKDTLDKISDVSAIIPYIGPALNISN
SVRRGNFTEAFAVTGVTILLEAFPEFTIPALGAFVIYSKVQERNEIIKTIDNCLEQRIKRWKDSYEWMMGTWLSRIITQF
NNISYQMYDSLNYQAGAIKAKIDLEYKKYSGSDKENIKSQVENLKNSLDVKISEAMNNINKFIRECSVTYLFKNMLPKVI
DELNEFDRNTKAKLINLIDSHNIILVGEVDKLKAKVNNSFQNTIPFNIFSYTNNSLLKDIINEYFNNINDSKILSLQNRK
NTLVDTSGYNAEVSEEGDVQLNPIFPFDFKLGSSGEDRGKVIVTQNENIVYNSMYESFSISFWIRINKWVSNLPGYTIID
SVKNNSGWSIGIISNFLVFTLKQNEDSEQSINFSYDISNNAPGYNKWFFVTVTNNMMGNMKIYINGKLIDTIKVKELTGI
NFSKTITFEINKIPDTGLITSDSDNINMWIRDFYIFAKELDGKDINILFNSLQYTNVVKDYWGNDLRYNKEYYMVNIDYL
NRYMYANSRQIVFNTRRNNNDFNEGYKIIIKRIRGNTNDTRVRGGDILYFDMTINNKAYNLFMKNETMYADNHSTEDIYA
IGLREQTKDINDNIIFQIQPMNNTYYYASQIFKSNFNGENISGICSIGTYRFRLGGDWYRHNYLVPTVKQGNYASLLEST
STHWGFVPVSE
```

### SEQ ID NO: 4 - BoNT/D, accession number P19321, amino acid sequence

```
MTWPVKDFNYSDPVNDNDILYLRIPQNKLITTPVKAFMITQNIWVIPERFSSDTNPSLSKPPRPTSKYQSYYDPSYLSTD
EQKDTFLKGIIKLFKRINERDIGKKLINYLVVGSPFMGDSSTPEDTFDFTRHTTNIAVEKFENGSWKVTNIITPSVLIFG
PLPNILDYTASLTLQGQQSNPSFEGFGTLSILKVAPEFLLTFSDVTSNQSSAVLGKSIFCMDPVIALMHELTHSLHQLYG
INIPSDKRIRPQVSEGFFSQDGPNVQFEELYTFGGLDVEIIPQIERSQLREKALGHYKDIAKRLNNINKTIPSSWISNID
KYKKIFSEKYNFDKDNTGNFVVNIDKFNSLYSDLTNVMSEVVYSSQYNVKNRTHYFSRHYLPVFANILDDNIYTIRDGFN

LTNKGFNIENSGQNIERNPALQKLSSESVVDLFTKVCLRLTKNSRDDSTCIKVKNNRLPYVADKDSISQEIFENKIITDE
TNVQNYSDKFSLDESILDGQVPINPEIVDPLLPNVNMEPLNLPGEEIVFYDDITKYVDYLNSYYYLESQKLSNNVENITL
TTSVEEALGYSNKIYTFLPSLAEKVNKGVQAGLFLNWANEVVEDFTTNIMKKDTLDKISDVSVIIPYIGPALNIGNSALR
GNFNQAFATAGVAFLLEGFPEFTIPALGVFTFYSSIQEREKIIKTIENCLEQRVKRWKDSYQWMVSNWLSRITTQFNHIN
YQMYDSLSYQADAIKAKIDLEYKKYSGSDKENIKSQVENLKNSLDVKISEAMNNINKFIRECSVTYLFKNMLPKVIDELN
KFDLRTKTELINLIDSHNIILVGEVDRLKAKVNESFENTMPFNIFSYTNNSLLKDIINEYFNSINDSKILSLQNKKNALV
DTSGYNAEVRVGDNVQLNTIYTNDFKLSSSGDKIIVNLNNNILYSAIYENSSVSFWIKISKDLTNSHNEYTIINSIEQNS
GWKLCIRNGNIEWILQDVNRKYKSLIFDYSESLSHTGYTNKWFFVTITNNIMGYMKLYINGELKQSQKIEDLDEVKLDKT
IVFGIDENIDENQMLWIRDFNIFSKELSNEDINIVYEGQILRNVIKDYWGNPLKFDTEYYIINDNYIDRYIAPESNVLVL
VQYPDRSKLYTGNPITIKSVSDKNPYSRILNGDNIILHMLYNSRKYMIIRDTDTIYATQGGECSQNCVYALKLQSNLGNY
GIGIFSIKNIVSKNKYCSQIFSSFRENTMLLADIYKPWRFSFKNAYTPVAVTNYETKLLSTSSFWKFISRDPGWVE
```

### SEQ ID NO: 5 - BoNT/E1, accession number WP 003372387, amino acid sequence

```
MPKINSFNYNDPVNDRTILYIKPGGCQEFYKSFNIMKNIWIIPERNVIGTTPQDFHPPTSLKNGDSSYYDPNYLQSDEEK
DRFLKIVTKIFNRINNNLSGGILLEELSKANPYLGNDNTPDNQFHIGDASAVEIKFSNGSQDILLPNVIIMGAEPDLFET
NSSNISLRNNYMPSNHGFGSIAIVTFSPEYSFRFNDNSMNEFIQDPALTLMHELIHSLHGLYGAKGITTKYTITQKQNPL
ITNIRGTNIEEFLTFGGTDLNIITSAQSNDIYTNLLADYKKIASKLSKVQVSNPLLNPYKDVFEAKYGLDKDASGIYSVN
INKFNDIFKKLYSFTEFDLATKFQVKCRQTYIGQYKYFKLSNLLNDSIYNISEGYNINNLKVNFRGQNANLNPRIITPIT
GRGLVKKIIRFCKNIVSVKGIRKSICIEINNGELFFVASENSYNDDNINTPKEIDDTVTSNNNYENDLDQVILNFNSESA
PGLSDEKLNLTIQNDAYIPKYDSNGTSDIEQHDVNELNVFFYLDAQKVPEGENNVNLTSSIDTALLEQPKIYTFFSSEFI
NNVNKPVQAALFVSWIQQVLVDFTTEANQKSTVDKIADISIVVPYIGLALNIGNEAQKGNFKDALELLGAGILLEFEPEL
LIPTILVFTIKSFLGSSDNKNKVIKAINNALKERDEKWKEVYSFIVSNWMTKINTQFNKRKEQMYQALQNQVNAIKTIIE
SKYNSYTLEEKNELTNKYDIKQIENELNQKVSIAMNNIDRFLTESSISYLMKLINEVKINKLREYDENVKTYLLNYIIQH
GSILGESQQELNSMVTDTLNNSIPFKLSSYTDDKILISYFNKFFKRIKSSSVLNMRYKNDKYVDTSGYDSNININGDVYK
YPTNKNQFGIYNDKLSEVNISQNDYIIYDNKYKNFSISFWVRIPNYDNKIVNVNNEYTIINCMRDNNSGWKVSLNHNEII
WTLQDNAGINQKLAFNYGNANGISDYINKWIFVTITNDRLGDSKLYINGNLIDQKSILNLGNIHVSDNILFKIVNCSYTR
YIGIRYFNIFDKELDETEIQTLYSNEPNTNILKDFWGNYLLYDKEYYLLNVLKPNNFIDRRKDSTLSINNIRSTILLANR
LYSGIKVKIQRVNNSSTNDNLVRKNDQVYINFVASKTHLFPLYADTATTNKEKTIKISSSGNRFNQVVVMNSVGNNCTMN
FKNNNGNNIGLLGFKADTVVASTWYYTHMRDHTNSNGCFWNFISEEHGWQEK
```

### SEQ ID NO: 6 - BoNT/F1, accession number Q57236, amino acid sequence

```
MPVVINSFNYNDPVNDDTILYMQIPYEEKSKKYYKAFEIMRNVWIIPERNTIGTDPSDFDPPASLENGSSAYYDPNYLTT
DAEKDRYLKTTIKLFKRINSNPAGEVLLQEISYAKPYLGNEHTPINEFHPVTRTTSVNIKSSTNVKSSIILNLLVLGAGP
DIFENSSYPVRKLMDSGGVYDPSNDGFGSINIVTFSPEYEYTFNDISGGYNSSTESFIADPAISLAHELIHALHGLYGAR
GVTYKETIKVKQAPLMIAEKPIRLEEFLTFGGQDLNIITSAMKEKIYNNLLANYEKIATRLSRVNSAPPEYDINEYKDYF
QWKYGLDKNADGSYTVNENKFNEIYKKLYSFTEIDLANKFKVKCRNTYFIKYGFLKVPNLLDDDIYTVSEGFNIGNLAVN
NRGQNIKLNPKIIDSIPDKGLVEKIVKFCKSVIPRKGTKAPPRLCIRVNNRELFFVASESSYNENDINTPKEIDDTTNLN
NNYRNNLDEVILDYNSETIPQISNQTLNTLVQDDSYVPRYDSNGTSEIEEHNVVDLNVFFYLHAQKVPEGETNISLTSSI
DTALSEESQVYTFFSSEFINTINKPVHAALFISWINQVIRDFTTEATQKSTFDKIADISLVVPYVGLALNIGNEVQKENF
KEAFELLGAGILLEFVPELLIPTILVFTIKSFIGSSENKNKIIKAINNSLMERETKWKEIYSWIVSNWLTRINTQFNKRK
EQMYQALQNQVDAIKTVIEYKYNNYTSDERNRLESEYNINNIREELNKKVSLAMENIERFITESSIFYLMKLINEAKVSK
LREYDEGVKEYLLDYISEHRSILGNSVQELNDLVTSTLNNSIPFELSSYTNDKILILYFNKLYKKIKDNSILDMRYENNK
FIDISGYGSNISINGDVYIYSTNRNQFGIYSSKPSEVNIAQNNDIIYNGRYQNFSISFWVRIPKYFNKVNLNNEYTIIDC
IRNNNSGWKISLNYNKIIWTLQDTAGNNQKLVFNYTQMISISDYINKWIFVTITNNRLGNSRIYINGNLIDEKSISNLGD
IHVSDNILFKIVGCNDTRYVGIRYFKVFDTELGKTEIETLYSDEPDPSILKDFWGNYLLYNKRYYLLNLLRTDKSITQNS
NFLNINQQRGVYQKPNIFSNTRLYTGVEVIIRKNGSTDISNTDNFVRKNDLAYINVVDRDVEYRLYADISIAKPEKIIKL
IRTSNSNNSLGQIIVMDSIGNNCTMNFQNNNGGNIGLLGFHSNNLVASSWYYNNIRKNTSSNGCFWSFISKEHGWQEN
```

### SEQ ID NO: 7 - BoNT/G, accession number WP 039635782, amino acid sequence

MPVNIKNFNYNDPINNDDIIMMEPFNDPGPGTYYKAFRIIDRIWIVPERFTYGFQPDQFNASTGVFSKDVYEYYDPTYLK
TDAEKDKFLKTMIKLFNRINSKPSGQRLLDMIVDAIPYLGNASTPPDKFAANVANVSINKKIIQPGAEDQIKGLMTNLII
FGPGPVLSDNFTDSMIMNGHSPISEGFGARMMIRFCPSCLNVFNNVQENKDTSIFSRRAYFADPALTLMHELIHVLHGLY
GIKISNLPITPNTKEFFMQHSDPVQAEELYTFGGHDPSVISPSTDMNIYNKALQNFQDIANRLNIVSSAQGSGIDISLYK
QIYKNKYDFVEDPNGKYSVDKDFDKLYKALMFGFTETNLAGEYGIKTRYSYFSEYLPPIKTEKLLDNTIYTQNEGFNIA
SKNLKTEFNGQNKAVNKEAYEEISLEHLVIYRIAMCKPVMYKNTGKSEQCIIVNNEDLFFIANKDSFSKDLAKAETIAYN
TQNNTIENNFSIDQLILDNDLSSGIDLPNENTEPFTNFDDIDIPVYIKQSALKKIFVDGDSLFEYLHAQTFPSNIENLQL
TNSLNDALRNNNKVYTFFSTNLVEKANTVVGASLFVNWVKGVIDDFTSESTQKSTIDKVSDVSIIIPYIGPALNVGNETA
KENFKNAFEIGGAAILMEFIPELIVPIVGFFTLESYVGNKGHIIMTISNALKKRDQKWTDMYGLIVSQWLSTVNTQFYTI
KERMYNALNNQSQAIEKIIEDQYNRYSEEDKMNINIDFNDIDFKLNQSINLAINNIDDFINQCSISYLMNRMIPLAVKKL
KDFDDNLKRDLLEYIDTNELYLLDEVNILKSKVNRHLKDSIPFDLSLYTKDTILIQVFNNYISNISSNAILSLSYRGGRL
IDSSGYGATMNVGSDVIFNDIGNGQFKLNNSENSNITAHQSKFVVYDSMFDNFSINFWVRTPKYNNNDIQTYLQNEYTII
SCIKNDSGWKVSIKGNRIIWTLIDVNAKSKSIFFEYSIKDNISDYINKWFSITITNDRLGNANIYINGSLKKSEKILNLD
RINSSNDIDFKLINCTDTTKFVWIKDFNIFGRELNATEVSSLYWIQSSTNTLKDFWGNPLRYDTQYYLFNQGMQNIYIKY

FSKASMGETAPRTNFNNAAINYQNLYLGLRFIIKKASNSRNINNDNIVREGDYIYLNIDNISDESYRVYVLVNSKEIQTQ
LFLAPINDDPTFYDVLQIKKYYEKTTYNCQILCEKDTKTFGLFGIGKFVKDYGYVWDTYDNYFCISQWYLRRISENINKL
RLGCNWQFIPVDEGWTE

<u>SEQ ID NO: 8 - BoNT/DC, accession number BAM65681, amino acid sequence</u>

MTWPVKDFNYSDPVNDNDILYLRIPQNKLITTPVKAFMITQNIWVIPERFSSDTNPSLSKPPRPTSKYQSYYDPSYLSTD
EQKDTFLKGIIKLFKRINERDIGKKLINYLVVGSPFMGDSSTPEDTFDFTRHTTNIAVEKFENGSWKVTNIITPSVLIFG
PLPNILDYTASLTLQGQQSNPSFEGFGTLSILKVAPEFLLTFSDVTSNQSSAVLGKSIFCMDPVIALMHELTHSLHQLYG
INIPSDKRIRPQVSEGFFSQDGPNVQFEELYTFGGSDVEIIPQIERLQLREKALGHYKDIAKRLNNINKTIPSSWSSNID
KYKKIFSEKYNFDKDNTGNFVVNIDKFNSLYSDLTNVMSEVVYSSQYNVKNRTHYFSKHYLPVFANILDDNIYTIINGFN
LTTKGFNIENSGQNIERNPALQKLSSESVVDLFTKVCLRLTRNSRDDSTCIQVKNNTLPYVADKDSISQEIFESQIITDE
TNVENYSDNFSLDESILDAKVPTNPEAVDPLLPNVNMEPLNVPGEEEVFYDDITKDVDYLNSYYYLEAQKLSNNVENITL
TTSVEEALGYSNKIYTFLPSLAEKVNKGVQAGLFLNWANEVVEDFTTNIMKKDTLDKISDVSAIIPYIGPALNIGNSALR
GNFKQAFATAGVAFLLEGFPEFTIPALGVFTFYSSIQEREKIIKTIENCLEQRVKRWKDSYQWMVSNWLSRITTQFNHIS
YQMYDSLSYQADAIKAKIDLEYKKYSGSDKENIKSQVENLKNSLDVKISEAMNNINKFIRECSVTYLFKNMLPKVIDELN
KFDLKTKTELINLIDSHNIILVGEVDRLKAKVNESFENTIPFNIFSYTNNSLLKDMINEYFNSINDSKILSLQNKKNTLM
DTSGYNAEVRVEGNVQLNPIFPFDFKLGSSGDDRGKVIVTQNENIVYNAMYESFSISFWIRINKWVSNLPGYTIIDSVKN
NSGWSIGIISNFLVFTLKQNENSEQDINFSYDISKNAAGYNKWFFVTITTNMMGNMMIYINGKLIDTIKVKELTGINFSK
TITFQMNKIPNTGLITSDSDNIMWIRDFYIFAKELDDKDINILFNSLQYTNVVKDYWGNDLRYDKEYYMINVNYMNRYM
SKKGNGIVFNTRKNNNDFNEGYKIIIKRIIGNTNDTRVRGENVLYFNTTIDNKQYSLGMYKPSRNLGTDLVPLGALDQPM
DEIRKYGSFIIQPCNTFDYYASQLFLSSNATTNRIGILSIGSYSFKLGDDYWFNHEYLIPVIKIEHYASLLESTSTHWVF
VPASE

<u>SEQ ID NO: 9 - BoNT/F7, amino acid sequence</u>

MPVNINNFNYNDPINNTTILYMKMPYYEDSNKYYKAFEIMDNVWIIPERNIIGKKPSDFYPPISLDSGSSAYYDPNYLTT
DAEKDRFLKTVIKLFNRINSNPAGQVLLEEIKNGKPYLGNDHTAVNEFCANNRSTSVEIKESKGTTDSMLLNLVILGPGP
NILECSTFPVRIFPNNIAYDPSEKGFGSIQLMSFSTEYEYAFNDNTDLFIADPAISLAHELIHVLHGLYGAKGVTNKKVI
EVDQGALMAAEKDIKIEEFITFGGQDLNIITNSTNQKIYDNLLSNYTAIASRLSQVNINNSALNTTYYKNFFQWKYGLDQ
DSNGNYTVNISKFNAIYKKLFSFTECDLAQKFQVKNRSNYLFHFKPFRLLDLLDDNIYSISEGFNIGSLRVNNNGQNINL
NSRIVGPIPDNGLVERFVGLCKSIVSKKGTKNSLCIKVNNRDLFFVASESSYNENGINSPKEIDDTTITNNNYKKNLDEV
ILDYNSDAIPNLSSRLLNTTAQNDSYVPKYDSNGTSEIKEYTVDKLNVFFYLYAQKAPEGESAISLTSSVNTALLDASKV
YTFFSSDFINTVNKPVQAALFISWIQQVINDFTTEATQKSTIDKIADISLVVPYVGLALNIGNEVQKGNFKEAIELLGAG
ILLEFVPELLIPTILVFTIKSFINSDDSKNKIIKAINNALRERELKWKEVYSWIVSNWLTRINTQFNKRKEQMYQALQNQ
VDGIKKIIEYKYNNYTLDEKNRLKAEYNIYSIKEELNKKVSLAMQNIDRFLTESSISYLMKLINEAKINKLSEYDKRVNQ
YLLNYILENSSTLGTSSVQELNNLVSNTLNNSIPFELSEYTNDKILISYFNRFYKRIIDSSILNMKYENNRFIDSSGYGS
NISINGDIYIYSTNRNQFGIYSSRLSEVNITQNNTIIYNSRYQNFSVSFWVRIPKYNNLKNLNNEYTIINCMRNNNSGWK
ISLNYNNIIWTLQDTTGNNQKLVFNYTQMIDISDYINKWTFVTITNNRLGHSKLYINGNLTDQKSILNLGNIHVDDNILF
KIVGCNDTRYVGIRYFKIFNMELDKTEIETLYHSEPDSTILKDFWGNYLLYNKKYYLLNLLKPNMSVTKNSDILNINRQR
GIYSKTNIFSNARLYTGVEVIIRKVGSTDTSNTDNFVRKNDTVYINVVDGNSEYQLYADVSTSAVEKTIKLRRISNSNYN
SNQMIIMDSIGDNCTMNFKTNNGNDIGLLGFHLNNLVASSWYYKNIRNNTRNNGCFWSFISKEHGWQE

<u>SEQ ID NO: 10 - BoNT/AB<sub>MY</sub>, amino acid sequence</u>

```
MPFVNKQFNYKDPVNGVDIAYIKIPNAGQMQPVKAFKIHNKIWVIPERD          49
TFTNPEEGDLNPPPEAKQVPVSYYDSTYLSTDNEKDNYLKGVTKLFERIY          99
STDLGRMLLTSIVRGIPFWGGSTIDTELKVIDTNCINVIQPDGSYRSEEL         149
NLVIIGPSADIIQFECKSFGHEVLNLTRNGYGSTQYIRFSPDFTFGFEES         199
LEVDTNPLLGAGKFATDPAVTLAHELIHAGHRLYGIAINPNRVFKVNTNA         249
YYEMSGLEVSFEELRTFGGHDAKFIDSLQENEFRLYYYNKFKDIASTLNK         299
AKSIVGTTASLQYMKNVFKEKYLLSEDTSGKFSVDKLKFDKLYKMLTEIY         349
TEDNFVKFFKVLNRKTYLNFDKAVFKINIVPKVNYTIYDGFNLRNTNLAA         399
NFNGQNTEINNMNFTKLKNFTGLFEFYKLLCVRGIITSKTKSLDKGYNKA         449
LNDLCIKVNNWDLFFSPSEDNFTNDLNKGEEITSDTNIEAAEENISLDLI         499
QQYYLTFNFDNEPENISIENLSSDIIGQLELMPNIERFPNGKKYELDKYT         549
MFHYLRAQEFEHGKSRIALTNSVNEALLNPSRVYTFFSSDYVKKVNKATE         599
AAMFLGWVEQLVYDFTDETSEVSTTDKIADITIIIPYIGPALNIGNMLYK         649
DDFVGALIFSGAVILLEFIPEIAIPVLGTFALVSYIANKVLTVQTIDNAL         699
SKRNEKWDEVYKYIVTNWLAKVNTQIDLIRKKMKEALENQAEATKAIINY         749
QYNQYTEEEKNNINFNIDDLSSKLNESINKAMININKFLNQCSVSYLMNS         799
MIPYGVKRLEDFDASLKDALLKYIYDNRGTLIGQVDRLKDKVNNTLSTDI         849
PFQLSKYVDNQRLLSTFTEYIKNILNNIILNLRYKDNNLIDLSGYGAKVE         899
VYDGVELNDKNQFKLTSSANSKIRVTQNQNIIFNSVFLDFSVSFWIRIPK         949
YKNDGIQNYIHNEYTIINCMKNNSGWKISIRGNRIIWTLIDINGKTKSVF         999
FEYNIREDISEYINRWFFVTITNNLNNAKIYINGKLESNTDIKDIREVIA        1049


NGEIIFKLDGDIDRTQFIWMKYFSIFNTELSQSNIEERYKIQSYSEYLKD        1099
FWGNPLMYNKEYYMFNAGNKNSYIKLKKDSPVGEILTRSKYNQNSKYINY        1149
RDLYIGEKFIIRRKSNSQSINDDIVRKEDYIYLDFFNLNQEWRVYTYKYF        1199
KKEEMKLFLAPIYDSDEFYNTIQIKEYDEQPTYSCQLLFKKDEESTDEIG        1249
LIGIHRFYESGIVFEEYKDYFCISKWYLKEVKRKPYNLKLGCNWQFIPKD        1299
EGWTE                                                      1304
```

<u>SEQ ID NO: 11 - BoNT/X, amino acid sequence (GenBank: BAQ12790.1)</u>

```
MKLEINKFNYNDPIDGINVITMRPPRHSDKINKGKGPFKAFQVIKNIWIVPERYNFTNNTNDLNIPSEPIMEADAIYNPNYLN
TPSEKDEFLQGVIKVLERIKSKPEGEKLLELISSSIPLPLVSNGALTLSDNETIAYQENNNIVSNLQANLVIYGPGPDIANNA
TYGLYSTPISNGEGTLSEVSFSPFYLKPFDESYGNYRSLVNIVNKFVKREFAPDPASTLMHELVHVTHNLYGISNRNFYYNFD
TGKIETSRQQNSLIFEELLTFGGIDSKAISSLIIKKIIETAKNNYTTLISERLNTVTVENDLLKYIKNKIPVQGRLGNFKLDT
AEFEKKLNTILFVLNESNLAQRFSILVRKHYLKERPIDPIYVNILDDNSYSTLEGFNISSQGSNDFQGQLLESSYFEKIESNA
LRAFIKICPRNGLLYNAIYRNSKNYLNNIDLEDKKTTSKTNVSYPCSLLNGCIEVENKDLFLISNKDSLNDINLSEEKIKPET
TVFFKDKLPPQDITLSNYDFTEANSIPSISQQNILERNEELYEPIRNSLFEIKTIYVDKLTTFHFLEAQNIDESIDSSKIRVE
LTDSVDEALSNPNKVYSPFKNMSNTINSIETGITSTYIFYQWLRSIVKDFSDETGKIDVIDKSSDTLAIVPYIGPLLNIGNDI
RHGDFVGAIELAGITALLEYVPEFTIPILVGLEVIGGELAREQVEAIVNNALDKRDQKWAEVYNITKAQWWGTIHLQINTRLA
HTYKALSRQANAIKMNMEFQLANYKGNIDDKAKIKNAISETEILLNKSVEQAMKNTEKFMIKLSNSYLTKEMIPKVQDNLKNF
DLETKKTLDKFIKEKEDILGTNLSSSLRRKVSIRLNKNIAFDINDIPFSEFDDLINQYKNEIEDYEVLNLGAEDGKIKDLSGT
TSDINIGSDIELADGRENKAIKIKGSENSTIKIAMNKYLRFSATDNFSISFWIKHPKPTNLLNNGIEYTLVENFNQRGWKISI
QDSKLIWYLRDHNNSIKIVTPDYIAFNGWNLITITNNRSKGSIVYVNGSKIEEKDISSIWNTEVDDPIIFRLKNNRDTQAFTL
LDQFSIYRKELNQEVVKLYNYYFNSNYIRDIWGNPLQYNKKYYLQTQDKPGKGLIREYWSSFGYDYVILSDSKTITFPNNIR
YGALYNGSKVLIKNSKKLDGLVRNKDFIQLEIDGYNMGISADRFNEDTNYIGTTYGTTHDLTTDFEIIQRQEKYRNYCQLKTP
YNIFHKSGLMSTETSKPTFHDYRDWVYSSAWYFQNYENLNLRKHTKTNWYFIPKDEGWDED
```

<u>SEQ ID NO: 12 (Nucleotide Sequence of Unmodified BoNT/A)</u>

```
ATGCCATTCGTCAACAAGCAATTCAACTACAAAGACCCAGTCAACGGCGTCGACATCGCATACATCAAGATTCCGAACGCCGG
TCAAATGCAGCCGGTTAAGGCTTTTAAGATCCACAACAAGATTTGGGTTATCCCGGAGCGTGACACCTTCACGAACCCGGAAG
AAGGCGATCTGAACCCGCCACCGGAAGCGAAGCAAGTCCCTGTCAGCTACTACGATTCGACGTACCTGAGCACGGATAACGAA
AAAGATAACTACCTGAAAGGTGTGACCAAGCTGTTCGAACGTATCTACAGCACGGATCTGGGTCGCATGCTGCTGACTAGCAT
TGTTCGCGGTATCCCGTTCTGGGGTGGTAGCACGATTGACACCGAACTGAAGGTTATCGACACTAACTGCATTAACGTTATTC
AACCGGATGGTAGCTATCGTAGCGAAGAGCTGAATCTGGTCATCATTGGCCCGAGCGCAGACATTATCCAATTCGAGTGCAAG
AGCTTTGGTCACGAGGTTCTGAATCTGACCCGCAATGGCTATGGTAGCACCCAGTACATTCGTTTTTCGCCGGATTTTACCTT
CGGCTTTGAAGAGAGCCTGGAGGTTGATACCAATCCGTTGCTGGGTGCGGGCAAATTCGCTACCGATCCGGCTGTCACGCTGG
CCCATGAACTGATCCACGCAGGCCACCGCCTGTACGGCATTGCCATCAACCCAAACCGTGTGTTCAAGGTTAATACGAATGCA
TACTACGAGATGAGCGGCCTGGAAGTCAGCTTCGAAGAACTGCGCACCTTCGGTGGCCATGACGCTAAATTCATTGACAGCTT
GCAAGAGAATGAGTTCCGTCTGTACTACTATAACAAATTCAAAGACATTGCAAGCACGTTGAACAAGGCCAAAAGCATCGTTG
GTACTACCGCGTCGTTGCAGTATATGAAGAATGTGTTTAAAGAGAAGTACCTGCTGTCCGAGGATACCTCCGGCAAGTTTAGC
GTTGATAAGCTGAAGTTTGACAAACTGTACAAGATGCTGACCGAGATTTACACCGAGGACAACTTTGTGAAATTCTTCAAAGT
GTTGAATCGTAAAACCTATCTGAATTTTGACAAAGCGGTTTTCAAGATTAACATCGTGCCGAAGGTGAACTACACCATCTATG
ACGGTTTTAACCTGCGTAACACCAACCTGGCGGCGAACTTTAACGGTCAGAATACGGAAATCAACAACATGAATTTCACGAAG
TTGAAGAACTTCACGGGTCTGTTCGAGTTCTATAAGCTGCTGTGCGTGCGCGGTATCATCACCAGCAAAACCAAAAGCCTGGA
CAAAGGCTACAACAAGGCGCTGAATGACCTGTGCATTAAGGTAAACAATTGGGATCTGTTCTTTTCGCCATCCGAAGATAATT
TTACCAACGACCTGAACAAGGGTGAAGAAATCACCAGCGATACGAATATTGAAGCAGCGGAAGAGAATATCAGCCTGGATCTG
ATCCAGCAGTACTATCTGACCTTTAACTTCGACAATGAACCGGAGAACATTAGCATTGAGAATCTGAGCAGCGACATTATCGG
TCAGCTGGAACTGATGCCGAATATCGAACGTTTCCCGAACGGCAAAAAGTACGAGCTGGACAAGTACACTATGTTCCATTACC
TGCGTGCACAGGAGTTTGAACACGGTAAAAGCCGTATCGCGCTGACCAACAGCGTTAACGAGGCCCTGCTGAACCCGAGCCGT
GTCTATACCTTCTTCAGCAGCGACTATGTTAAGAAAGTGAACAAAGCCACTGAGGCCGCGATGTTCCTGGGCTGGGTGGAACA
GCTGGTATATGACTTCACGGACGAGACGAGCGAAGTGAGCACTACCGACAAAATTGCTGATATTACCATCATTATCCCGTATA
TTGGTCCGGCACTGAACATTGGCAACATGCTGTACAAAGACGATTTTGTGGGTGCCCTGATCTTCTCCGGTGCCGTGATTCTG
CTGGAGTTCATTCCGGAGATTGCGATCCCGGTGTTGGGTACCTTCGCGCTGGTGTCCTACATCGCGAATAAGGTTCTGACGGT
TCAGACCATCGATAACGCGCTGTCGAAACGTAATGAAAATGGGACGAGGTTTACAAATACATTGTTACGAATTGGCTGGCGA
AAGTCAATACCCAGATCGACCTGATCCGTAAGAAATGAAAGAGGCGCTGGAGAATCAGGCGGAGGCCACCAAAGCAATTATC
AACTACCAATACAACCAGTACACGGAAGAAGAGAAGAATAACATTAACTTCAATATCGATGATTTGAGCAGCAAGCTGAATGA
ATCTATCAACAAAGCGATGATCAATATCAACAAGTTTTTGAATCAGTGTAGCGTTTCGTACCTGATGAATAGCATGATTCCGT
ATGGCGTCAAACGTCTGGAGGACTTCGACGCCAGCCTGAAAGATGCGTTGCTGAAATACATTTACGACAATCGTGGTACGCTG
ATTGGCCAAGTTGACCGCTTGAAAGACAAAGTTAACAATACCCTGAGCACCGACATCCCATTTCAACTGAGCAAGTATGTTGA
TAATCAACGTCTGTTGAGCACTTTCACCGAGTATATCAAAAACATCATCAATACTAGCATTCTGAACCTGCGTTACGAGAGCA
ATCATCTGATTGATCTGAGCCGTTATGCAAGCAAGATCAACATCGGTAGCAAGGTCAATTTTGACCCGATCGATAAGAACCAG
ATCCAGCTGTTTAATCTGGAATCGAGCAAAATTGAGGTTATCCTGAAAAACGCCATTGTCTACAACTCCATGTACGAGAATTT
CTCCACCAGCTTCTGGATTCGCATCCCGAAATACTTCAACAGCATTAGCCTGAACAACGAGTATACTATCATCAACTGTATGG
AGAACAACAGCGGTTGGAAGGTGTCTCTGAACTATGGTGAGATCATTTGGACCTTGCAGGACACCCAAGAGATCAAGCAGCGC
GTCGTGTTCAAGTACTCTCAAATGATCAACATTTCCGATTACATTAATCGTTGGATCTTCGTGACCATTACGAATAACCGTCT
GAATAACAGCAAGATTTACATCAATGGTCGCTTGATCGATCAGAAACCGATTAGCAACCTGGGTAATATCCACGCAAGCAACA
ACATTATGTTCAAATTGGACGGTTGCCGCGATACCCATCGTTATATCTGGATCAAGTATTTCAACCTGTTTGATAAGAACTG

AATGAGAAGGAGATCAAAGATTTGTATGACAACCAATCTAACAGCGGCATTTTGAAGGACTTCTGGGGCGATTATCTGCAATA
CGATAAGCCGTACTATATGCTGAACCTGTATGATCCGAACAAATATGTGGATGTCAATAATGTGGGTATTCGTGGTTACATGT
ATTTGAAGGGTCCGCGTGGCAGCGTTATGACGACCAACATTTACCTGAACTCTAGCCTGTACCGTGGTACGAAATTCATCATT
AAGAAATATGCCAGCGGCAACAAAGATAACATTGTGCGTAATAACGATCGTGTCTACATCAACGTGGTCGTGAAGAATAAAGA
GTACCGTCTGGCGACCAACGCTTCGCAGGCGGGTGTTGAGAAAATTCTGAGCGCGTTGGAGATCCCTGATGTCGGTAATCTGA
GCCAAGTCGTGGTTATGAAGAGCAAGAACGACCAGGGTATCACTAACAAGTGCAAGATGAACCTGCAAGACAACAATGGTAAC
GACATCGGCTTTATTGGTTTCCACCAGTTCAACAATATTGCTAAACTGGTAGCGAGCAATTGGTACAATCGTCAGATTGAGCG
CAGCAGCCGTACTTTGGGCTGTAGCTGGGAGTTTATCCCGGTCGATGATGGTTGGGGCGAACGTCCGCTG
```

SEQ ID NO: 13 (Polypeptide Sequence of Unmodified BoNT/A)

```
MPFVNKQFNYKDPVNGVDIAYIKIPNAGQMQPVKAFKIHNKIWVIPERDTFTNPEEGDLNPPPEAKQVPVSYYDSTYLSTDNE
KDNYLKGVTKLFERIYSTDLGRMLLTSIVRGIPFWGGSTIDELKVIDTNCINVIQPDGSYRSEELNLVIIGPSADIIQFECK
SFGHEVLNLTRNGYGSTQYIRFSPDFTFGFEESLEVDTNPLLGAGKFATDPAVTLAHELIHAGHRLYGIAINPNRVFKVNTNA
YYEMSGLEVSFEELRTFGGHDAKFIDSLQENEFRLYYYNKFKDIASTLNKAKSIVGTTASLQYMKNVFKEKYLLSEDTSGKFS
VDKLKFDKLYKMLTEIYTEDNFVKFFKVLNRKTYLNFDKAVFKINIVPKVNYTIYDGFNLRNTNLAANFNGQNTEINNMNFTK
LKNFTGLFEFYKLLCVRGIITSKTKSLDKGYNKALNDLCIKVNNWDLFFSPSEDNFTNDLNKGEEITSDTNIEAAEENISLDL
IQQYYLTFNFDNEPENISIENLSSDIIGQLELMPNIERFPNGKKYELDKYTMFHYLRAQEFEHGKSRIALTNSVNEALLNPSR
VYTFFSSDYVKKVNKATEAAMFLGWVEQLVYDFTDETSEVSTTDKIADITIIIPYIGPALNIGNMLYKDDFVGALIFSGAVIL
LEFIPEIAIPVLGTFALVSYIANKVLTVQTIDNALSKRNEKWDEVYKYIVTNWLAKVNTQIDLIRKKMKEALENQAEATKAII
NYQYNQYTEEEKNNINFNIDDLSSKLNESINKAMININKFLNQCSVSYLMNSMIPYGVKRLEDFDASLKDALLKYIYDNRGTL
IGQVDRLKDKVNNTLSTDIPFQLSKYVDNQRLLSTFTEYIKNIINTSILNLRYESNHLIDLSRYASKINIGSKVNFDPIDKNQ
IQLFNLESSKIEVILKNAIVYNSMYENFSTSFWIRIPKYFNSISLNNEYTIINCMENNSGWKVSLNYGEIIWTLQDTQEIKQR
VVFKYSQMINISDYINRWIFVTITNNRLNNSKIYINGRLIDQKPISNLGNIHASNNIMFKLDGCRDTHRYIWIKYFNLFDKEL
NEKEIKDLYDNQSNSGILKDFWGDYLQYDKPYYMLNLYDPNKYVDVNNVGIRGYMYLKGPRGSVMTTNIYLNSSLYRGTKFII
KKYASGNKDNIVRNNDRVYINVVVKNKEYRLATNASQAGVEKILSALEIPDVGNLSQVVVMKSKNDQGITNKCKMNLQDNNGN
DIGFIGFHQFNNIAKLVASNWYNRQIERSSRTLGCSWEFIPVDDGWGERPL
```

SEQ ID NO: 14 (Nucleotide Sequence of Modified BoNT/A "Cat-A")

```
ATGCCATTCGTCAACAAGCAATTCAACTACAAAGACCCAGTCAACGGCGTCGACATCGCATACATCAAGATTCCGAACGCCGG
TCAAATGCAGCCGGTTAAGGCTTTTAAGATCCACAACAAGATTTGGGTTATCCCGGAGCGTGACACCTTCACGAACCCGGAAG
AAGGCGATCTGAACCCGCCACCGGAAGCGAAGCAAGTCCCTGTCAGCTACTACGATTCGACGTACCTGAGCACGGATAACGAA
AAAGATAACTACCTGAAAGGTGTGACCAAGCTGTTCGAACGTATCTACAGCACGGATCTGGGTCGCATGCTGCTGACTAGCAT
TGTTCGCGGTATCCCGTTCTGGGGTGGTAGCACGATTGACACCGAACTGAAGGTTATCGACACTAACTGCATTAACGTTATTC
AACCGGATGGTAGCTATCGTAGCGAAGAGCTGAATCTGGTCATCATTGGCCCGAGCGCAGACATTATCCAATTCGAGTGCAAG
AGCTTTGGTCACGAGGTTCTGAATCTGACCCGCAATGGCTATGGTAGCACCCAGTACATTCGTTTTTCGCCGGATTTTACCTT
CGGCTTTGAAGAGAGCCTGGAGGTTGATACCAATCCGTTGCTGGGTGCGGGCAAATTCGCTACCGATCCGGCTGTCACGCTGG
CCCATGAACTGATCCACGCAGGCCACCGCCTGTACGGCATTGCCATCAACCCAAACCGTGTGTTCAAGGTTAATACGAATGCA
TACTACGAGATGAGCGGCCTGGAAGTCAGCTTCGAAGAACTGCGCACCTTCGGTGGCCATGACGCTAAATTCATTGACAGCTT
GCAAGAGAATGAGTTCCGTCTGTACTACTATAACAAATTCAAAGACATTGCAAGCACGTTGAACAAGGCCAAAAGCATCGTTG
GTACTACCGCGTCGTTGCAGTATATGAAGAATGTGTTTAAAGAGAAGTACCTGCTGTCCGAGGATACCTCCGGCAAGTTTAGC
GTTGATAAGCTGAAGTTTGACAAACTGTACAAGATGCTGACCGAGATTTACACCGAGGACAACTTTGTGAAATTCTTCAAAGT
GTTGAATCGTAAAACCTATCTGAATTTTGACAAAGCGGTTTTCAAGATTAACATCGTGCCGAAGGTGAACTACACCATCTATG
ACGGTTTTAACCTGCGTAACACCAACCTGGCGGCGAACTTTAACGGTCAGAATACGGAAATCAACAACATGAATTTCACGAAG
TTGAAGAACTTCACGGGTCTGTTCGAGTTCTATAAGCTGCTGTGCGTGCGCGGTATCATCACCAGCAAAACCAAAAGCCTGGA
CAAAGGCTACAACAAGGCGCTGAATGACCTGTGCATTAAGGTAAACAATTGGGATCTGTTCTTTTCGCCATCCGAAGATAATT
TTACCAACGACCTGAACAAGGGTGAAGAAATCACCAGCGATACGAATATTGAAGCAGCGGAAGAGAATATCAGCCTGGATCTG
ATCCAGCAGTACTATCTGACCTTTAACTTCGACAATGAACCGGAGAACATTAGCATTGAGAATCTGAGCAGCGACATTATCGG
TCAGCTGGAACTGATGCCGAATATCGAACGTTTCCCGAACGGCAAAAAGTACGAGCTGGACAAGTACACTATGTTCCATTACC
TGCGTGCACAGGAGTTTGAACACGGTAAAAGCCGTATCGCGCTGACCAACAGCGTTAACGAGGCCCTGCTGAACCCGAGCCGT
GTCTATACCTTCTTCAGCAGCGACTATGTTAAGAAAGTGAACAAAGCCACTGAGGCCGCGATGTTCCTGGGCTGGGTGGAACA
GCTGGTATATGACTTCACGGACGAGACGAGCGAAGTGAGCACTACCGACAAAATTGCTGATATTACCATCATTATCCCGTATA
TTGGTCCGGCACTGAACATTGGCAACATGCTGTACAAAGACGATTTTGTGGGTGCCCTGATCTTCTCCGGTGCCGTGATTCTG
CTGGAGTTCATTCCGGAGATTGCGATCCCGGTGTTGGGTACCTTCGCGCTGGTGTCCTACATCGCGAATAAGGTTCTGACGGT
TCAGACCATCGATAACGCGCTGTCGAAACGTAATGAAAAATGGGACGAGGTTTACAAATACATTGTTACGAATTGGCTGGCGA
AAGTCAATACCCAGATCGACCTGATCCGTAAGAAAATGAAAGAGGCGCTGGAGAATCAGGCGGAGGCCACCAAAGCAATTATC
AACTACCAATACAACCAGTACACGGAAGAAGAGAAGAATAACATTAACTTCAATATCGATGATTTGAGCAGCAAGCTGAATGA
ATCTATCAACAAAGCGATGATCAATATCAACAAGTTTTTGAATCAGTGTAGCGTTTCGTACCTGATGAATAGCATGATTCCGT
ATGGCGTCAAACGTCTGGAGGACTTCGACGCCAGCCTGAAAGATGCGTTGCTGAAATACATTTACGACAATCGTGGTACGCTG
ATTGGCCAAGTTGACCGCTTGAAAGACAAAGTTAACAATACCCTGAGCACCGACATCCCATTTCAACTGAGCAAGTATGTTGA
TAATCAACGTCTGTTGAGCACTTTCACCGAGTATATCAAAAACATCATCAATACTAGCATTCTGAACCTGCGTTACGAGAGCA
AGCATCTGATTGATCTGAGCCGTTATGCTAGCAAGATCAACATCGGTAGCAAGGTCAATTTTGACCCGATCGATAAGAACCAG
ATCCAGCTGTTTAATCTGGAATCGAGCAAAATTGAGGTTACCTGAAAAAGGCCATTGTCTACAACTCCATGTACGAGAATTT
CTCCACCAGCTTCTGGATTCGCATCCCGAAATACTTCAACAAGATTAGCCTGAACAACGAGTATACTATCATCAACTGTATGG
AGAACAACAGCGGTTGGAAGGTGTCTCTGAACTATGGTGAGATCATTTGGACCTTGCAGGACACCAAAGAGATCAAGCAGCGC
GTCGTGTTCAAGTACTCTCAAATGATCAACATTTCCGATTACATTAATCGTTGGATCTTCGTGACCATTACGAATAACCGTCT
GAATAAGAGCAAGATTTACATCAATGGTCGCTTGATCGATCAGAAACCGATTAGCAACCTGGGTAATATCCACGCAAGCAACA
```

```
AGATTATGTTCAAATTGGACGGTTGCCGCGATACCCATCGTTATATCTGGATCAAGTATTTCAACCTGTTTGATAAAGAACTG
AATGAGAAGGAGATCAAAGATTTGTATGACAACCAATCTAACAGCGGCATTTTGAAGGACTTCTGGGGCGATTATCTGCAATA
CGATAAGCCGTACTATATGCTGAACCTGTATGATCCGAACAAATATGTGGATGTCAATAATGTGGGTATTCGTGGTTACATGT
ATTTGAAGGGTCCGCGTGGCAGCGTTATGACGACCAACATTTACCTGAACTCTAGCCTGTACCGTGGTACGAAATTCATCATT
AAGAAATATGCCAGCGGCAACAAAGATAACATTGTGCGTAATAACGATCGTGTCTACATCAACGTGGTCGTGAAGAATAAAGA
GTACCGTCTGGCGACCAACGCTTCGCAGGCGGGTGTTGAGAAAATTCTGAGCGCGTTGGAGATCCCTGATGTCGGTAATCTGA
GCCAAGTCGTGGTTATGAAGAGCAAGAACGACAAGGGTATCACTAACAAGTGCAAGATGAACCTGCAAGACAACAATGGTAAC
GACATCGGCTTTATTGGTTTCCACCAGTTCAACAATATTGCTAAACTGGTAGCGAGCAATTGGTACAATCGTCAGATTGAGCG
CAGCAGCcGTACTTTGGGCTGTAGCTGGGAGTTTATCCCGGTCGATGATGGTTGGGGCGAACGTCCGCTG
```

SEQ ID NO: 15 (Polypeptide Sequence of Modified BoNT/A "Cat-A")

```
MPFVNKQFNYKDPVNGVDIAYIKIPNAGQMQPVKAFKIHNKIWVIPERDTFTNPEEGDLNPPPEAKQVPVSYYDSTYLSTDNE
KDNYLKGVTKLFERIYSTDLGRMLLTSIVRGIPFWGGSTIDTELKVIDTNCINVIQPDGSYRSEELNLVIIGPSADIIQFECK
SFGHEVLNLTRNGYGSTQYIRFSPDFTFGFEESLEVDTNPLLGAGKFATDPAVTLAHELIHAGHRLYGIAINPNRVFKVNTNA
YYEMSGLEVSFEELRTFGGHDAKFIDSLQENEFRLYYYNKFKDIASTLNKAKSIVGTTASLQYMKNVFKEKYLLSEDTSGKFS
VDKLKFDKLYKMLTEIYTEDNFVKFFKVLNRKTYLNFDKAVFKINIVPKVNYTIYDGFNLRNTNLAANFNGQNTEINNMNFTK
LKNFTGLFEFYKLLCVRGIITSKTKSLDKGYNKALNDLCIKVNNWDLFFSPSEDNFTNDLNKGEEITSDTNIEAAEENISLDL
IQQYYLTFNFDNEPENISIENLSSDIIGQLELMPNIERFPNGKKYELDKYTMFHYLRAQEFEHGKSRIALTNSVNEALLNPSR
VYTFFSSDYVKKVNKATEAAMFLGWVEQLVYDFTDETSEVSTTDKIADITIIIPYIGPALNIGNMLYKDDFVGALIFSGAVIL
LEFIPEIAIPVLGTFALVSYIANKVLTVQTIDNALSKRNEKWDEVYKYIVTNWLAKVNTQIDLIRKKMKEALENQAEATKAII
NYQYNQYTEEEKNNINFNIDDLSSKLNESINKAMININKFLNQCSVSYLMNSMIPYGVKRLEDFDASLKDALLKYIYDNRGTL
IGQVDRLKDKVNNTLSTDIPFQLSKYVDNQRLLSTFTEYIKNIINTSILNLRYESKHLIDLSRYASKINIGSKVNFDPIDKNQ
IQLFNLESSKIEVILKKAIVYNSMYENFSTSFWIRIPKYFNKISLNNEYTIINCMENNSGWKVSLNYGEIIWTLQDTKEIKQR
VVFKYSQMINISDYINRWIFVTITNNRLNKSKIYINGRLIDQKPISNLGNIHASNKIMFKLDGCRDTHRYIWIKYFNLFDKEL
NEKEIKDLYDNQSNSGILKDFWGDYLQYDKPYYMLNLYDPNKYVDVNNVGIRGYMYLKGPRGSVMTTNIYLNSSLYRGTKFII
KKYASGNKDNIVRNNDRVYINVVVKNKEYRLATNASQAGVEKILSALEIPDVGNLSQVVVMKSKNDKGITNKCKMNLQDNNGN
DIGFIGFHQFNNIAKLVASNWYNRQIERSSRTLGCSWEFIPVDDGWGERPL
```

SEQ ID NO: 16 (Nucleotide Sequence of Modified BoNT/A "Cat-B")

ATGCCATTCGTCAACAAGCAATTCAACTACAAAGACCCAGTCAACGGCGTCGACATCGCATACATCAAGATTCCGAACGCCGG
TCAAATGCAGCCGGTTAAGGCTTTTAAGATCCACAACAAGATTTGGGTTATCCCGGAGCGTGACACCTTCACGAACCCGGAAG
AAGGCGATCTGAACCCGCCACCGGAAGCGAAGCAAGTCCCTGTCAGCTACTACGATTCGACGTACCTGAGCACGGATAACGAA
AAAGATAACTACCTGAAAGGTGTGACCAAGCTGTTCGAACGTATCTACAGCACGGATCTGGGTCGCATGCTGCTGACTAGCAT
TGTTCGCGGTATCCCGTTCTGGGGTGGTAGCACGATTGACACCGAACTGAAGGTTATCGACACTAACTGCATTAACGTTATTC
AACCGGATGGTAGCTATCGTAGCGAAGAGCTGAATCTGGTCATCATTGGCCCGAGCGCAGACATTATCCAATTCGAGTGCAAG
AGCTTTGGTCACGAGGTTCTGAATCTGACCCGCAATGGCTATGGTAGCACCCAGTACATTCGTTTTTCGCCGGATTTTACCTT
CGGCTTTGAAGAGAGCCTGGAGGTTGATACCAATCCGTTGCTGGGTGCGGGCAAATTCGCTACCGATCCGGCTGTCACGCTGG
CCCATGAACTGATCCACGCAGGCCACCGCCTGTACGGCATTGCCATCAACCCAAACCGTGTGTTCAAGGTTAATACGAATGCA
TACTACGAGATGAGCGGCCTGGAAGTCAGCTTCGAAGAACTGCGCACCTTCGGTGGCCATGACGCTAAATTCATTGACAGCTT
GCAAGAGAATGAGTTCCGTCTGTACTACTATAACAAATTCAAAGACATTGCAAGCACGTTGAACAAGGCCAAAAGCATCGTTG
GTACTACCGCGTCGTTGCAGTATATGAAGAATGTGTTTAAAGAGAAGTACCTGCTGTCCGAGGATACCTCCGGCAAGTTTAGC
GTTGATAAGCTGAAGTTTGACAAACTGTACaAGATGCTGACCGAGATTTACACCGAGGACAACTTTGTGAAATTCTTCAAAGT
GTTGAATCGTAAAACCTATCTGAATTTTGACAAAGCGGTTTTCAAGATTAACATCGTGCCGAAGGTGAACTACACCATCTATG
ACGGTTTTAACCTGCGTAACACCAACCTGGCGGCGAACTTTAACGGTCAGAATACGGAAATCAACAACATGAATTTCACGAAG
TTGAAGAACTTCACGGGTCTGTTCGAGTTCTATAAGCTGCTGTGCGTGCGCGGTATCATCACCAGCAAAAACCAAAAGCCTGGA
CAAAGGCTACAACAAGGCGCTGAATGACCTGTGCATTAAGGTAAACAATTGGGATCTGTTCTTTTCGCCATCCGAAGATAATT
TTACCAACGACCTGAACAAGGGTGAAGAAATCACCAGCGATACGAATATTGAAGCAGCGGAAGAGAATATCAGCCTGGATCTG
ATCCAGCAGTACTATCTGACCTTTAACTTCGACAATGAACCGGAGAACATTAGCATTGAGAATCTGAGCAGCGACATTATCGG
TCAGCTGGAACTGATGCCGAATATCGAACGTTTCCCGAACGGCAAAAAGTACGAGCTGGACAAGTACACTATGTTCCATTACC
TGCGTGCACAGGAGTTTGAACACGGTAAAAGCCGTATCGCGCTGACCAACAGCGTTAACGAGGCCCTGCTGAACCCGAGCCGT
GTCTATACCTTCTTCAGCAGCGACTATGTTAAGAAAGTGAACAAAGCCACTGAGGCCGCGATGTTCCTGGGCTGGGTGGAACA
GCTGGTATATGACTTCACGGACGAGACGAGCGAAGTGAGCACTACCGACAAAATTGCTGATATTACCATCATTATCCCGTATA
TTGGTCCGGCACTGAACATTGGCAACATGCTGTACAAAGACGATTTTGTGGGTGCCCTGATCTTCTCCGGTGCCGTGATTCTG
CTGGAGTTCATTCCGGAGATTGCGATCCCGGTGTTGGGTACCTTCGCGCTGGTGTCCTACATCGCGAATAAGGTTCTGACGGT
TCAGACCATCGATAACGCGCTGTCGAAACGTAATGAAAAATGGGACGAGGTTTACAAATACATTGTTACGAATTGGCTGGCGA
AAGTCAATAACCCAGATCGACCTGATCCGTAAGAAAATGAAAGAGGCGCTGGAGAATCAGGCGGAGGCCACCAAAGCAATTATC
AACTACCAATACAACCAGTACACGGAAGAAGCAGGAAGAATAACCATTAACTTCAATATCGATGATTTGAGCAGCGAAGCTGAATGA
ATCTATCAACAAAGCGATGATCAATATCAACAAGTTTTTGAATCAGTGTAGCGTTTCGTACCTGATGAATAGCATGATTCCGT
ATGGCGTCAAACGTCTGGAGGACTTCGACGCCAGCCTGAAAGATGCGTTGCTGAAATACATTTACGACAaTCGTGGTACGCTG
ATTGGCCAAGTTGACCGCTTGAAAGACAAAGTTAACAATACCCTGAGCACCGACATCCCATTTCAACTGAGCAAGTATGTTGA
TAATCAACGTCTGTTGAGCACTTTCACCGAGTATATCAAAAACATCATCAATACTAGCATTCTGAACCTGCGTTACGAGAGCA
ATCATCTGATTGATCTGAGCCGTTATGCTAGCAAGATCAACATCGGTAGCAAGGTCAATTTTGACCCGATCGATAAGAACCAG
ATCCAGCTGTTTAATCTGGAATCGAGCAAAATTGAGGTTATCCTGAAAAAGGCCATTGTCTACAACTCCATGTACGAGAATTT
CTCCACCAGCTTCTGGATTCGCATCCCGAAATACTTCAAGAAGATTAGCCTGAACAACGAGTATACTATCATCAACTGTATGG
AGAACAACAGCGGTTGGAAGGTGTCTCTGAACTATGGTGAGATCATTTGGACCTTGCAGGACACCAAAGAGATCAAGCAGCGC
GTCGTGTTCAAGTACTCTCAAATGATCAACATTTCCGATTACATTAATCGTTGGATCTTCGTGACCATTACGAATAACCGTCT
GAATAAGAGCAAGATTTACATCAATGGTCGCTTGATCGATCAGAAACCGATTAGCAACCTGGGTAATATCCACGCAAGCAACA

AGATTATGTTCAAATTGGACGGTTGCCGCGATACCCATCGTTATATCTGGATCAAGTATTTCAACCTGTTTGATAAGAACTG
AATGAGAAGGAGATCAAAGATTTGTATGACAACCAATCTAACAGCGGCATTTTGAAGGACTTCTGGGGCGATTATCTGCAATA
CGATAAGCCGTACTATATGCTGAACCTGTATGATCCGAACAAATATGTGGATGTCAATAATGTGGGTATTCGTGGTTACATGT
ATTTGAAGGGTCCGCGTGGCAGCGTTATGACGACCAACATTTACCTGAACTCTAGCCTGTACCGTGGTACGAAATTCATCATT
AAGAAATATGCCAGCGGCAACAAAGATAACATTGTGCGTAATAACGATCGTGTCTACATCAACGTGGTCGTGAAGAATAAAGA
GTACCGTCTGGCGACCAACGCTTCGCAGGCGGGTGTTGAGAAAATTCTGAGCGCGTTGGAGATCCCTGATGTCGGTAATCTGA
GCCAAGTCGTGGTTATGAAGAGCAAGAACGACAAGGGTATCACTAACAAGTGCAAGATGAACCTGCAAGACAACAATGGTAAC
GACATCGGCTTTATTGGTTTCCACCAGTTCAACAATATTGCTAAACTGGTAGCGAGCAATTGGTACAATCGTCAGATTGAGCG
CAGCAGCCGTACTTTGGGCTGTAGCTGGGAGTTTATCCCGGTCGATGATGGTTGGGGCGAACGTCCGCTG

SEQ ID NO: 17 (Polypeptide Sequence of Modified BoNT/A "Cat-B")

```
MPFVNKQFNYKDPVNGVDIAYIKIPNAGQMQPVKAFKIHNKIWVIPERDTFTNPEEGDLNPPPEAKQVPVSYYDSTYLSTDNE
KDNYLKGVTKLFERIYSTDLGRMLLTSIVRGIPFWGGSTIDTELKVIDTNCINVIQPDGSYRSEELNLVIIGPSADIIQFECK
SFGHEVLNLTRNGYGSTQYIRFSPDFTFGFEESLEVDTNPLLGAGKFATDPAVTLAHELIHAGHRLYGIAINPNRVFKVNTNA
YYEMSGLEVSFEELRTFGGHDAKFIDSLQENEFRLYYYNKFKDIASTLNKAKSIVGTTASLQYMKNVFKEKYLLSEDTSGKFS
VDKLKFDKLYKMLTEIYTEDNFVKFFKVLNRKTYLNFDKAVFKINIVPKVNYTIYDGFNLRNTNLAANFNGQNTEINNMNFTK
LKNFTGLFEFYKLLCVRGIITSKTKSLDKGYNKALNDLCIKVNNWDLFFSPSEDNFTNDLNKGEEITSDTNIEAAEENISLDL
IQQYYLTFNFDNEPENISIENLSSDIIGQLELMPNIERFPNGKKYELDKYTMFHYLRAQEFEHGKSRIALTNSVNEALLNPSR
VYTFFSSDYVKKVNKATEAAMFLGWVEQLVYDFTDETSEVSTTDKIADITIIIPYIGPALNIGNMLYKDDFVGALIFSGAVIL
LEFIPEIAIPVLGTFALVSYIANKVLTVQTIDNALSKRNEKWDEVYKYIVTNWLAKVNTQIDLIRKKMKEALENQAEATKAII
NYQYNQYTEEEKNNINFNIDDLSSKLNESINKAMININKFLNQCSVSYLMNSMIPYGVKRLEDFDASLKDALLKYIYDNRGTL
IGQVDRLKDKVNNTLSTDIPFQLSKYVDNQRLLSTFTEYIKNIIINTSILNLRYESNHLIDLSRYASKINIGSKVNFDPIDKNQ
IQLFNLESSKIEVILKKAIVYNSMYENFSTSFWIRIPKYFKKISLNNEYTIINCMENNSGWKVSLNYGEIIWTLQDTKEIKQR
VVFKYSQMINISDYINRWIFVTITNNRLNKSKIYINGRLIDQKPISNLGNIHASNKIMFKLDGCRDTHRYIWIKYFNLFDKEL
NEKEIKDLYDNQSNSGILKDFWGDYLQYDKPYYMLNLYDPNKYVDVNNVGIRGYMYLKGPRGSVMTTNIYLNSSLYRGTKFII
KKYASGNKDNIVRNNDRVYINVVVKNKEYRLATNASQAGVEKILSALEIPDVGNLSQVVVMKSKNDKGITNKCKMNLQDNNGN
DIGFIGFHQFNNIAKLVASNWYNRQIERSSRTLGCSWEFIPVDDGWGERPL
```

SEQ ID NO: 18 (Nucleotide Sequence of Modified BoNT/A "Cat-C")

```
ATGCCATTCGTCAACAAGCAATTCAACTACAAAGACCCAGTCAACGGCGTCGACATCGCATACATCAAGATTCCGAACGCCGG
TCAAATGCAGCCGGTTAAGGCTTTTAAGATCCACAACAAGATTTGGGTTATCCCGGAGCGTGACACCTTCACGAACCCGGAAG
AAGGCGATCTGAACCCGCCACCGGAAGCGAAGCAAGTCCCTGTCAGCTACTACGATTCGACGTACCTGAGCACGGATAACGAA
AAAGATAACTACCTGAAAGGTGTGACCAAGCTGTTCGAACGTATCTACAGCACGGATCTGGGTCGCATGCTGCTGACTAGCAT
TGTTCGCGGTATCCCGTTCTGGGGTGGTAGCACGATTGACACCGAACTGAAGGTTATCGACACTAACTGCATTAACGTTATTC
AACCGGATGGTAGCTATCGTAGCGAAGAGCTGAATCTGGTCATCATTGGCCCGAGCGCAGACATTATCCAATTCGAGTGCAAG
AGCTTTGGTCACGAGGTTCTGAATCTGACCCGCAATGGCTATGGTAGCACCCAGTACATTCGTTTTTCGCCGGATTTTACCTT
CGGCTTTGAAGAGAGCCTGGAGGTTGATACCAATCCGTTGCTGGGTGCGGGCAAATTCGCTACCGATCCGGCTGTCACGCTGG
CCCATGAACTGATCCACGCAGGCCACCGCCTGTACGGCATTGCCATCAACCCAAACCGTGTGTTCAAGGTTAATACGAATGCA
TACTACGAGATGAGCGGCCTGGAAGTCAGCTTCGAAGAACTGCGCACCTTCGGTGGCCATGACGCTAAATTCATTGACAGCTT
GCAAGAGAATGAGTTCCGTCTGTACTACTATAACAAATTCAAAGACATTGCAAGCACGTTGAACAAGGCCAAAAGCATCGTTG
GTACTACCGCGTCGTTGCAGTATATGAAGAATGTGTTTAAAGAGAAGTACCTGCTGTCCGAGGATACCTCCGGCAAGTTTAGC
GTTGATAAGCTGAAGTTTGACAAACTGTACAAGATGCTGACCGAGATTTACACCGAGGACAACTTTGTGAAATTCTTCAAAGT
GTTGAATCGTAAAACCTATCTGAATTTTGACAAAGCGGTTTTCAAGATTAACATCGTGCCGAAGGTGAACTACACCATCTATG
ACGGTTTTAACCTGCGTAACACCAACCTGGCGGCGAACTTTAACGGTCAGAATACGGAAATCAACAACATGAATTTCACGAAG
TTGAAGAACTTCACGGGTCTGTTCGAGTTCTATAAGCTGCTGTGCGTGCGCGGTATCATCACCAGCAAAACCAAAAGCCTGGA
CAAAGGCTACAACAAGGCGCTGAATGACCTGTGCATTAAGGTAAACAATTGGGATCTGTTCTTTTCGCCATCCGAAGATAATT
TTACCAACGACCTGAACAAGGGTGAAGAAATCACCAGCGATACGAATATTGAAGCAGCGGAAGAGAATATCAGCCTGGATCTG
ATCCAGCAGTACTATCTGACCTTTAACTTCGACAATGAACCGGAGAACATTAGCATTGAGAATCTGAGCAGCGACATTATCGG
TCAGCTGGAACTGATGCCGAATATCGAACGTTTCCCGAACGGCAAAAAGTACGAGCTGGACAAGTACACTATGTTCCATTACC
TGCGTGCACAGGAGTTTGAACACGGTAAAAGCCGTATCGCGCTGACCAACAGCGTTAACGAGGCCCTGCTGAACCCGAGCCGT
GTCTATACCTTCTTCAGCAGCGACTATGTTAAGAAAGTGAACAAAGCCACTGAGGCCGCGATGTTCCTGGGCTGGGTGGAACA
GCTGGTATATGACTTCACGGACGAGACGAGCGAAGTGAGCACTACCGACAAAATTGCTGATATTACCATCATTATCCCGTATA
TTGGTCCGGCACTGAACATTGGCAACATGCTGTACAAAGACGATTTTGTGGGTGCCCTGATCTTCTCCGGTGCCGTGATTCTG
CTGGAGTTCATTCCGGAGATTGCGATCCCGGTGTTGGGTACCTTCGCGCTGGTGTCCTACATCGCGAATAAGGTTCTGACGGT
TCAGACCATCGATAACGCGCTGTCGAAACGTAATGAAAAATGGGACGAGGTTTACAAATACATTGTTACGAATTGGCTGGCGA
AAGTCAATACCCAGATCGACCTGATCCGTAAGAAAATGAAAGAGGCGCTGGAGAATCAGGCGGAGGCCACCAAAGCAATTATC
AACTACCAATACAACCAGTACACGGAAGAAGAGAAGAATAACATTAACTTCAATATCGATGATTTGAGCAGCAAGCTGAATGA
ATCTATCAACAAAGCGATGATCAATATCAACAAGTTTTTGAATCAGTGTAGCGTTTCGTACCTGATGAATAGCATGATTCCGT
ATGGCGTCAAACGTCTGGAGGACTTCGACGCCAGCCTGAAAGATGCGTTGCTGAAATACATTTACGACAATCGTGGTACGCTG
ATTGGCCAAGTTGACCGCTTGAAAGACAAAGTTAACAATACCCTGAGCACCGACATCCCATTTCAACTGAGCAAGTATGTTGA
TAATCAACGTCTGTTGAGCACTTTCACCGAGTATATCAAAAACATCATCAATACTAGCATTCTGAACCTGCGTTACGAGAGCA
ATCATCTGATTGATCTGAGCCGTTATGCTAGCAAGATCAACATCGGTAGCAAGGTCAATTTTGACCCGATCGATAAGAACCAG
ATCCAGCTGTTTAATCTGGAATCGAGCAAAATTGAGGTATCCTGAAAAAGGCCATTGTCTACAACTCCATGTACGAGAATTT
CTCCACCAGCTTCTGGATTCGCATCCCGAAATACTTCAACAAGATTAGCCTGAACAACGAGTATACTATCATCAACTGTATGG
AGAACAACAGCGGTTGGAAGGTGTCTCTGAACTATGGTGAGATCATTTGGACCTTGCAGGACACCAAAGAGATCAAGCAGCGC
GTCGTGTTCAAGTACTCTCAAATGATCAACATTTCCGATTACATTAATCGTTGGATCTTCGTGACCATTACGAATAACCGTCT
GAAGAAGAGCAAGATTTACATCAATGGTCGCTTGATCGATCAGAAACCGATTAGCAACCTGGGTAATATCCACGCAAGCAACA
```

```
AGATTATGTTCAAATTGGACGGTTGCCGCGATACCCATCGTTATATCTGGATCAAGTATTTCAACCTGTTTGATAAAGAACTG
AATGAGAAGGAGATCAAAGATTTGTATGACAACCAATCTAACAGCGGCATTTTGAAGGACTTCTGGGGCGATTATCTGCAATA
CGATAAGCCGTACTATATGCTGAACCTGTATGATCCGAACAAATATGTGGATGTCAATAATGTGGGTATTCGTGGTTACATGT
ATTTGAAGGGTCCGCGTGGCAGCGTTATGACGACCAACATTTACCTGAACTCTAGCCTGTACCGTGGTACGAAATTCATCATT
AAGAAATATGCCAGCGGCAACAAAGATAACATTGTGCGTAATAACGATCGTGTCTACATCAACGTGGTCGTGAAGAATAAAGA
GTACCGTCTGGCGACCAACGCTTCGCAGGCGGGTGTTGAGAAAATTCTGAGCGCGTTGGAGATCCCTGATGTCGGTAATCTGA
GCCAAGTCGTGGTTATGAAGAGCAAGAACGACAAGGGTATCACTAACAAGTGCAAGATGAACCTGCAAGACAACAATGGTAAC
GACATCGGCTTTATTGGTTTCCACCAGTTCAACAATATTGCTAAACTGGTAGCGAGCAATTGGTACAATCGTCAGATTGAGCG
CAGCAGCCGTACTTTGGGCTGTAGCTGGGAGTTTATCCCGGTCGATGATGGTTGGGGCGAACGTCCGCTG
```

SEQ ID NO: 19 (Polypeptide Sequence of Modified BoNT/A "Cat-C")

```
MPFVNKQFNYKDPVNGVDIAYIKIPNAGQMQPVKAFKIHNKIWVIPERDTFTNPEEGDLNPPPEAKQVPVSYYDSTYLSTDNE
KDNYLKGVTKLFERIYSTDLGRMLLTSIVRGIPFWGGSTIDTELKVIDTNCINVIQPDGSYRSEELNLVIIGPSADIIQFECK
SFGHEVLNLTRNGYGSTQYIRFSPDFTFGFEESLEVDTNPLLGAGKFATDPAVTLAHELIHAGHRLYGIAINPNRVFKVNTNA
YYEMSGLEVSFEELRTFGGHDAKFIDSLQENEFRLYYYNKFKDIASTLNKAKSIVGTTASLQYMKNVFKEKYLLSEDTSGKFS
VDKLKFDKLYKMLTEIYTEDNFVKFFKVLNRKTYLNFDKAVFKINIVPKVNYTIYDGFNLRNTNLAANFNGQNTEINNMNFTK
LKNFTGLFEFYKLLCVRGIITSKTKSLDKGYNKALNDLCIKVNNWDLFFSPSEDNFTNDLNKGEEITSDTNIEAAEENISLDL
IQQYYLTFNFDNEPENISIENLSSDIIGQLELMPNIERFPNGKKYELDKYTMFHYLRAQEFEHGKSRIALTNSVNEALLNPSR
VYTFFSSDYVKKVNKATEAAMFLGWVEQLVYDFTDETSEVSTTDKIADITIIIPYIGPALNIGNMLYKDDFVGALIFSGAVIL
LEFIPEIAIPVLGTFALVSYIANKVLTVQTIDNALSKRNEKWDEVYKYIVTNWLAKVNTQIDLIRKKMKEALENQAEATKAII
NYQYNQYTEEEKNNINFNIDDLSSKLNESINKAMININKFLNQCSVSYLMNSMIPYGVKRLEDFDASLKDALLKYIYDNRGTL
IGQVDRLKDKVNNTLSTDIPFQLSKYVDNQRLLSTFTEYIKNIINTSILNLRYESNHLIDLSRYASKINIGSKVNFDPIDKNQ
IQLFNLESSKIEVILKKAIVYNSMYENFSTSFWIRIPKYFNKISLNNEYTIINCMENNSGWKVSLNYGEIIWTLQDTKEIKQR
VVFKYSQMINISDYINRWIFVTITNNRLKKSKIYINGRLIDQKPISNLGNIHASNKIMFKLDGCRDTHRYIWIKYFNLFDKEL
NEKEIKDLYDNQSNSGILKDFWGDYLQYDKPYYMLNLYDPNKYVDVNNVGIRGYMYLKGPRGSVMTTNIYLNSSLYRGTKFII
KKYASGNKDNIVRNNDRVYINVVVKNKEYRLATNASQAGVEKILSALEIPDVGNLSQVVVMKSKNDKGITNKCKMNLQDNNGN
DIGFIGFHQFNNIAKLVASNWYNRQIERSSRTLGCSWEFIPVDDGWGERPL
```

SEQ ID NO: 20 (Nucleotide Sequence of Modified BoNT/A "Cat-D")

ATGCCATTCGTCAACAAGCAATTCAACTACAAAGACCCAGTCAACGGCGTCGACATCGCATACATCAAGATTCCGAACGCCGG
TCAAATGCAGCCGGTTAAGGCTTTTAAGATCCACAACAAGATTTGGGTTATCCCGGAGCGTGACACCTTCACGAACCCCGGAAG
AAGGCGATCTGAACCCGCCACCGGAAGCGAAGCAAGTCCCTGTCAGCTACTACGATTCGACGTACCTGAGCACGGATAACGAA
AAAGATAACTACCTGAAAGGTGTGACCAAGCTGTTCGAACGTATCTACAGCACGGATCTGGGTCGCATGCTGCTGACTAGCAT
TGTTCGCGGTATCCCGTTCTGGGGTGGTAGCACGATTGACACCGAACTGAAGGTTATCGACACTAACTGCATTAACGTTATTC
AACCGGATGGTAGCTATCGTAGCGAAGAGCTGAATCTGGTCATCATTGGCCCGAGCGCAGACATTATCCAATTCGAGTGCAAG
AGCTTTGGTCACGAGGTTCTGAATCTGACCCGCAATGGCTATGGTAGCACCCAGTACATTCGTTTTTCGCCGGATTTTACCTT
CGGCTTTGAAGAGAGCCTGGAGGTTGATACCAATCCGTTGCTGGGTGCGGGCAAATTCGCTACCGATCCGGCTGTCACGCTGG
CCCATGAACTGATCCACGCAGGCCACCGCCTGTACGGCATTGCCATCAACCCAAACCGTGTGTTCAAGGTTAATACGAATGCA
TACTACGAGATGAGCGGCCTgGAAGTCAGCTTCGAAGAACTGCGCACCTTCGGTGGCCATGACGCTAAATTCATTGACAGCTT
GCAAGAGAATGAGTTCCGTCTGTACTACTATAACAAATTCAAAGACATTGCAAGCACGTTGAACAAGGCCAAAAGCATCGTTG
GTACTACCGCGTCGTTGCAGTATATGAAGAATGTGTTTAAAGAGAAGTACCTGCTGTCCGAGGATACCTCCGGCAAGTTTAGC
GTTGATAAGCTGAAGTTTGACAAACTGTACAAGATGCTGACCGAGATTTACACCGAGGACAACTTTGTGAAATTCTTCAAaGT
GTTGAATCGTAAAACCTATCTGAATTTTGACAAAGCGGTTTTCaAGATTAACATCGTGCCGAAGGTGAACTACACCATCTATG
ACGGTTTTAACCTGCGTAACACCAACCTGGCGGCGAACTTTAACGGTCAGAATACGGAAATCAACAACATGAATTTCACGAAG
TTGAAGAACTTCACGGGTCTGTTCGAGTTCTATAAGCTGCTGTGCGTGCGCGGTATCATCACCAGCAAAAACCAAAAGCCTGGA
CAAAGGCTACAACAAGGCGCTGAATGACCTGTGCATTAAGGTAAACAATTGGGATCTGTTCTTTTCGCCATCCGAAGATAATT
TTACCAACGACCTGAACAAGGGTGAAGAAATCACCAGCGATACGAATATTGAAGCAGCGGAAGAGAATATCAGCCTGGATCTG
ATCCAGCAGTACTATCTGACCTTTAACTTCGACAATGAACCGGAGAACATTAGCATTGAGAATCTGAGCAGCGACATTATCGG
TCAGCTGGAACTGATGCCGAATATCGAACGTTTCCCGAACGGCAAAAAGTACGAGCTGGACAAGTACACTATGTTCCATTACC
TGCGTGCACAGGAGTTTGAACACGGTAAAAGCCGTATCGCGCTGACCAACAGCGTTAACGAGGCCCTGCTGAACCCGAGCCGT
GTCTATACCTTCTTCAGCAGCGACTATGTTAAGAAAGTGAACAAAGCCACTGAGGCCGCGATGTTCCTGGGCTGGGTGGAACA
GCTGGTATATGACTTCACGGACGAGACGAGCGAAGTGAGCACTACCGACAAAaTTGCTGATaTTACCATCATTATCCCGTATA
TTGGTCCGGCACTGAACATTGGCAACATGCTGTACAAAGACGATTTTGTGGGTGCCCTGATCTTCTCCGGTGCCGTGATTCTG
CTGGAGTTCATTCCGGAGATTGCGATCCCGGTGTTGGGTACCTTCGCGCTGGTGTCCTACATCGCGAATAAGGTTCTGACGGT
TCAGACCATCGATAACGCGCTGTCGAAACGTAATGAAAAATGGGACGAGGTTTACAAATACATTGTTACGAATTGGCTGGCGA
AAGTCaATACCCAGATCGACCTGATCCGTAAGAAAATGAAAGAGGCGCTGGAGAATCAGGCGGAGGCCACCAAAGCAATTATC
AACTACCTACAACCAGTACACGGAAGAAGGAAGAAGAATAACATTAACTTCAATATCGATGATTTGAGCAGCAAGCTGAATGA
ATCTATCAACAAAGCGATGATCAATATCAACAAGTTTTTGAATCAGTGTAGCGTTTCGTACCTGATGAATAGCATGATTCCGT
ATGGCGTCAAACGTCTGGAGGACTTCGACGCCAGCCTGAAAGATGCGTTGCTGAAATACATTTACGACAATCGTGGTACGCTG
ATTGGCCAAGTTGACCGCTTGAAAGACAAAGTTAACAATACCCTGAGCACCGACATCCCATTTCAACTGAGCAAGTATGTTGA
TAATCAACGTCTGTTGAGCACTTTCACCGAGTATATCAAAAACATCATCAATACTAGCATTCTGAACCTGCGTTACGAGAGCA
ATCATCTGATtGATCTGAGCCGTTATGCAAGCAAGATCAACATCGGTAGCAAGGTCAATTTTGACCCGATCGATAAGAACCAG
ATCCAGCTGTTTAATCTGGAATCGAGCAAAATTGAGGTTATCCTGAAAAACGCCATTGTCTACAACTCCATGTACGAGAATTT
CTCCACCAGCTTCTGGATTCGCATCCCGAAATACTTCAACAGCATTAGCCTGAACAACGAGTATACTATCATCAACTGTATGG
AGAACAACAGCGGTTGGAAGGTGTCTCTGAACTATGGTGAGATCATTTGGACCTTGCAGGACACCCAAGAGATCAAGCAGCGC
GTCGTGTTCAAGTACTCTCAAATGATCAACATTTCCGATTACATTAATCGTTGGATCTTCGTGACCATTACGAATAACCGTCT
GAATAACAGCAAGATTTACATCAATGGTCGCTTGATCGATCAGAAACCGATTAGCAACCTGGGTAATATCCACGCAAGCAACA

ACATTATGTTCAAATTGGACGGTTGCCGCGATACCCATCGTTATATCTGGATCAAGTATTTCAACCTGTTTGATAAAGAACTG
AATGAGAAGGAGATCAAAGATTTGTATGACAACCAATCTAACAGCGGCATTTTGAAGGACTTCTGGGGCGATTATCTGCAATA
CGATAAGCCGTACTATATGCTGAACCTGTATGATCCGAACAAATATGTGGATGTCAATAATGTGGGTATTCGTGGTTACATGT
ATTTGAAGGGGTCCGCGTGGCAGCGTTATGACGACCAACATTTACCTGAACTCTAGCCTGTACCGTGGTACGAAATTCATCATT
AAGAAATATGCCAGCGGCAACAAAGATAACATTGTGCGTAATAACGATCGTGTCTACATCAACGTGGTCGTGAAGCGTAAAGA
GTACCGTCTGGCGACCAACGCTTCGCAGGCGGGTGTTGAGAAAATTCTGAGCGCGTTGGAGATCCCTCGTGTCCGTCGTCTGA
GCCAAGTCGTGGTTATGAAGAGCAAGAACGACCAGGGTATCACTAACAAGTGCAAGATGAACCTGCAAGACCGTCGTGGTAAC
GACATCGGCTTTATTGGTTTCCACCAGTTCAACAATATTGCTAAACTGGTAGCGAGCAATTGGTACAATCGTCAGATTGAGCG
CCGTAGCCGTCGTTTGGGGCTGTAGCTGGGAGTTTATCCCGGTCGATGATGGTTGGGGCGAACGTCCGCTG

SEQ ID NO: 21 (Polypeptide Sequence of Modified BoNT/A "Cat-D")

```
MPFVNKQFNYKDPVNGVDIAYIKIPNAGQMQPVKAFKIHNKIWVIPERDTFTNPEEGDLNPPPEAKQVPVSYYDSTYLSTDNE
KDNYLKGVTKLFERIYSTDLGRMLLTSIVRGIPFWGGSTIDTELKVIDTNCINVIQPDGSYRSEELNLVIIGPSADIIQFECK
SFGHEVLNLTRNGYGSTQYIRFSPDFTFGFEESLEVDTNPLLGAGKFATDPAVTLAHELIHAGHRLYGIAINPNRVFKVNTNA
YYEMSGLEVSFEELRTFGGHDAKFIDSLQENEFRLYYYNKFKDIASTLNKAKSIVGTTASLQYMKNVFKEKYLLSEDTSGKFS
VDKLKFDKLYKMLTEIYTEDNFVKFFKVLNRKTYLNFDKAVFKINIVPKVNYTIYDGFNLRNTNLAANFNGQNTEINNMNFTK
LKNFTGLFEFYKLLCVRGIITSKTKSLDKGYNKALNDLCIKVNNWDLFFSPSEDNFTNDLNKGEEITSDTNIEAAEENISLDL
IQQYYLTFNFDNEPENISIENLSSDIIGQLELMPNIERFPNGKKYELDKYTMFHYLRAQEFEHGKSRIALTNSVNEALLNPSR
VYTFFSSDYVKKVNKATEAAMFLGWVEQLVYDFTDETSEVSTTDKIADITIIIPYIGPALNIGNMLYKDDFVGALIFSGAVIL
LEFIPEIAIPVLGTFALVSYIANKVLTVQTIDNALSKRNEKWDEVYKYIVTNWLAKVNTQIDLIRKKMKEALENQAEATKAII
NYQYNQYTEEEKNNINFNIDDLSSKLNESINKAMININKFLNQCSVSYLMNSMIPYGVKRLEDFDASLKDALLKYIYDNRGTL
IGQVDRLKDKVNNTLSTDIPFQLSKYVDNQRLLSTFTEYIKNIINTSILNLRYESNHLIDLSRYASKINIGSKVNFDPIDKNQ
IQLFNLESSKIEVILKNAIVYNSMYENFSTSFWIRIPKYFNSISLNNEYTIINCMENNSGWKVSLNYGEIIWTLQDTQEIKQR
VVFKYSQMINISDYINRWIFVTITNNRLNNSKIYINGRLIDQKPISNLGNIHASNNIMFKLDGCRDTHRYIWIKYFNLFDKEL
NEKEIKDLYDNQSNSGILKDFWGDYLQYDKPYYMLNLYDPNKYVDVNNVGIRGYMYLKGPRGSVMTTNIYLNSSLYRGTKFII
KKYASGNKDNIVRNNDRVYINVVVKRKEYRLATNASQAGVEKILSALEIPRVRRLSQVVVMKSKNDQGITNKCKMNLQDRRGN
DIGFIGFHQFNNIAKLVASNWYNRQIERRSRRLGCSWEFIPVDDGWGERPL
```

## EXAMPLES

## MATERIALS AND METHODS

### Animal model

Male domestic pigs weighing 11-13 kg were used in the following study. The pig is a suitable model for studying the treatment of post-operative surgical pain as porcine skin shares similarities with human skin in terms of structure, thickness, innervation, pigmentation, collagen and lipid composition, wound-healing and immune responses.

### Reconstitution of Dysport

Dysport was provided in vials containing 500U. For dosing, vials of 500U were reconstituted with saline (0.9% NaCl). Subsequent dilutions were done with saline according to the testing doses as follows:
2.5 ml of saline was drawn with 3 ml syringe + 21 G needle and transferred to the Dysport 500U vial; the concentration was 200U/ml = 400U/2ml; the vial was gently swirled until material was dissolved. Each vial was tilted side-to-side 2-3 times (to ensure solution homogeneity); pigs were dosed with 2 ml using 2 syringes of 1 ml connected to a 30 G needle. This solution was used for dosing of 400U.

### Preparation for 200U/2 ml dose:

Dysport was reconstituted as explained above; 3 ml syringe and 21 G needle were used to draw 2 ml of reconstituted Dysport 500U; 3 ml syringe and 21 G needle were used to draw 2 ml of saline; a vacutainer vial was used to mix the 2 solutions mentioned above; the mixed solution was tilted side-to-side 5-6 times (to ensure solution homogeneity); the pigs were dosed with 2 ml using 2 syringes of 1 ml connected to a 30 G needle.

### Preparation for 100U/2 ml dose:

Dysport was reconstituted as explained above; 3 ml syringe and 21 G needle were used to draw 2 ml of reconstituted Dysport 500U; 3 ml syringe and 21 G needle were used to draw 2 ml of saline; a vacutainer vial was used to mix the 2 solutions mentioned above; the mixed solution was tilted side-to-side 5-6 times (to ensure solution homogeneity); 3 ml syringe and 21 G needle were used to draw 2 ml of prepared solution from the vacutainer vial; 3 ml syringe and 21 G needle were used to draw 2 ml of saline; a new vacutainer was used to mix the 2 solutions mentioned above; the mixed solution was tilted side-to-side 5-6 times (to ensure solution homogeneity); pigs were dosed with 2 ml using 2 syringes of 1 ml connected to 30 G needle.

### Induction of post-operative surgical pain

Pigs were anesthetized by an isoflurane/oxygen mixture, which was delivered through a facemask. The area of the incision was cleaned using Septol and Polydine (Iodo-Vit) solution. A 6-7 cm long skin incision was made in the left flank, towards the caudal end of the pig and 3 cm lateral to the spine line (Day 1) or a 7 cm long skin incision was made in the left leg. Then the fascia was cut and the muscle was retracted (Castel et al., Characterization of a porcine model of post-operative pain, Eur. J. Pain. 2014, 18(4), 496-505

). The sub-cutis was then sutured with 3-0 Vicryl thread. The skin was sutured with 3-0 silk thread using continuous suturing methods. Following the incision closure and material injection, the pigs received antibiotic (Marbocyl 10%). The area of the incision was covered with the thin layer of Syntomicine 3%. The animals were kept under anesthesia for the duration of the surgery and dosing (about 20 minutes). Post-surgery the animals were returned to their pens for recovery and observation.

Treatment

*Intradermal peri-operative administration of Dysport*

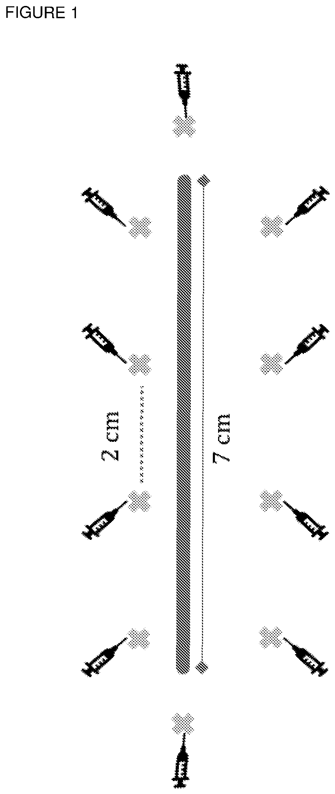

For peri-operative administration, animals were injected just after suturing the incision made in the left flank. Dysport (test item), saline (negative control) or the reference compound Exparel (positive control) were injected intradermally (or subcutaneously for Exparel) using 30G needles attached to 1 ml syringes and into 10 sites around the incision. Each site was injected with a fixed dosing volume and fixed dosing level. In more detail, 4 sites along each side (e.g. 8 sites) of a 7 cm horizontal incision/ suture in the left flank were injected (at 2cm intervals), as were sites at each end of the incision/suture in the left flank (see Figure 1). The following experimental groups were assessed as follows:

| Group Number | Treatment | Number of Animals | Dosing volume | Dosing level per animal |
|---|---|---|---|---|
| 1 | Saline | 6 | 200 μL/site of injection | Saline |
| 2 | Exparel | 6 | 20 ml | 266 mg |
| 3 | Dysport | 6 | 200 μL/site of injection | 100 U |
| 4 | Dysport | 6 | 200 μL/site of injection | 200 U |
| 5 | Dysport | 6 | 200 μL/site of injection | 400 U |

*Intradermal pre-operative administration of Dysport*

For pre-operative injections, as animals were injected before the incision (either in the left flank or left leg of the pig) with a fixed total volume of 2 ml, the location of the further incision was tattooed first. Dysport (test item) or saline (negative control) were injected intradermally using 30G needles attached to 1 ml syringes and into 10 sites around the incision. Each site was injected with a fixed dosing volume and fixed dosing level. Administrations were performed either at 15 days, 5 days or 1 day prior to surgery. The following experimental groups (when an incision was made in the left flank of the pig) were assessed as follows:

| Group Number | Treatment | No. of Animals | Dosing volume | Dosing level per animal | Dosing day vs. operation day |
|---|---|---|---|---|---|
| 1 | Dysport | 6 | 200 μL/site of injection | Saline | - 15 |
| 2 | Saline | 6 | 200 μL/site of injection | 200 U/pig as 10 sites injected with 20 U | |
| 3 | Dysport | 6 | 200 μL/site of injection | Saline | - 5 |
| 4 | Saline | 6 | 200 μL/site of injection | 200 U/pig as 10 sites injected with 20 U | |
| 5 | Dysport | 6 | 200 μL/site of injection | Saline | - 1 |
| 6 | Saline | 6 | 200 μL/site of injection | 200 U/pig as 10 sites injected with 20 U | |

The following experimental groups (when an incision was made in the left leg of the pig) were assessed as follows:

| Group Number | Treatment | No. of Animals | Dosing volume | Dosing level per animal | Dosing day vs. operation day |
|---|---|---|---|---|---|
| 1 | Saline | 6 | 200 µL/site of injection | Saline | - 15 |
| 2 | Dysport | 5 | 200 µL/site of injection | 200 U/pig as 10 sites injected with 20 U | |
| 3 | Exparel | 6 | 20 ml | 266 mg | 1 |

*Administration of Dysport via intradermal, intramuscular or subcutaneous routes*

As animals were injected 15 days before the incision, the location of the further incision was tattooed. Dysport (test item) or saline (negative control) were injected using 30G needles attached to 1 ml syringes and into 10 sites around the incision. Each site was injected with a fixed dosing volume and fixed dosing level. Administrations were performed either via the intradermal, subcutaneous or intramuscular route.

The following experimental groups were assessed as follows:

| Group Number | Treatment | Route of administration | Dosing volume and dosing level | Dosing day vs. operation day |
|---|---|---|---|---|
| 1 | Dysport | Intramuscular | 200 U/2 ml/ pig split into 10 sites of 200 µL | -15 |
| 2 | Saline | | | |
| 3 | Dysport | Intradermal | 200 U/2 ml/ pig split into 10 sites of 200 µL | -15 |
| 4 | Saline | | | |
| 5 | Dysport | Subcutaneous | 200 U/2 ml/ pig split into 10 sites of 200 µL | -15 |
| 6 | Saline | | | |

Von Frey assay

Von Frey assay was performed in healthy, unoperated animals after Dysport/saline injections at 1, 2, 4 and 6 hours post-surgery on day 1 and once-daily for 10 days. Von Frey filaments (Ugo Basile, Italy) were applied at approximately ~0.5 cm proximal to the incision line to the surface of the flank or leg skin. As the gram number of filaments increases, the force on the flanks' or legs' skin increases. The maximum force used was 60 g. Filaments were applied until the animal withdrew from the stimuli. Each filament was applied 3-5 times. If withdrawal was not achieved, a thicker filament was applied. If a withdrawal was achieved, a thinner filament was applied (thicker or thinner refers to higher/thicker or lower/thinner gram force). By alternating the filament thickness, the force required to achieve withdrawal reaction was determined and recorded. The size and force of the Von Frey filaments are outlined in the table below:

| Size | 1.65 | 2.36 | 2.44 | 2.83 | 3.22 | 3.61 | 3.84 | 4.08 | 4.17 | 4.31 | 4.56 | 4.74 | 4.93 | 5.07 | 5.18 | 5.46 | 5.88 | 6.10 | 6.45 | 6.65 |
|---|---|---|---|---|---|---|---|---|---|---|---|---|---|---|---|---|---|---|---|---|
| Force (g) | 0.008 | 0.02 | 0.04 | 0.07 | 0.16 | 0.40 | 060 | 1.00 | 1.40 | 2.00 | 4.00 | 6.00 | 8.00 | 10.0 | 15.0 | 26.0 | 60.0 | 100 | 180 | 300 |
| | | | | | | | | Fitament in use | | | | | | | | | | | | |

Inclusion criteria: Animals were included in the study if the flank withdrawal force at baseline was ≥26 g (preferably 60g). After surgery, pain (allodynia) was considered present if flank withdrawal force was ≤10g. If the animal did not meet this criteria it was excluded from the study. One animal was excluded from the study due to relatively low threshold before operation (≤10g).

Animals were included in the study if the leg withdrawal force at baseline was ≥13 g. After surgery, pain (allodynia) was considered present if leg withdrawal force was ≤2g. If the animal did not meet this criteria it was excluded from the study.

Approaching Time test

Prior to the dosing of pigs, the researcher who was conducting the approaching time (AT) test entered the pen for the first time. The normal behaviour of the pigs when someone entering their housing pen is moving away from the intruder and then approaching the person. The more familiar the pigs are with the person and the more comfortable they feel, the less time it takes them to approach. The latency to approach the researcher entering their home-pen was measured in seconds (cut-off time at 120 sec). This test was done in the morning, at least 1 hour post morning feeding (at 6:30 am) before the distress behaviour score and during the habituation period.

Distress behaviour score

Following incision, the behaviour of the animals changed. When approached, the animals moved away from the researcher entering their pen, tended to guard the incision side and sometimes used vocalization. This is the main phenomenon observed following this type of surgery, in rare cases the animals became restless or showed an isolation behaviour. The distress behaviour is scored from 0 (normal) to 7 (very distressed). The distress behaviour score test is performed immediately after the approaching time test. The animal general behaviour was monitored in their home pen during the morning period. The distress behaviour score also allows the overall health status of animals to be assessed. The behaviour of the animals was scored by an observer blind to the treatment, where the total score is the sum of all sections shown in the table below.

| Scoring Section | Parameter | Score |
|---|---|---|
| Section 1 | Avoiding standing (lying down) | 1 |
| | Standing | 0 |
| Section 2 | Avoiding walking | 1 |
| | Walking | 0 |
| Section 3 | Protecting the incision side while walking | 1 |
| | Acting normal | 0 |
| Section 4 | Moving away when approached by researcher | 1 |
| | Not moving away when approached by researcher | 0 |
| Section 5 | Restlessness | 1 |
| | Normal | 0 |
| Section 6 | Staying in isolation from other animals | 1 |
| | Staying together with other animals | 0 |
| Section 7 | Screaming (high vocalisation) | 1 |
| | Normal vocalisation | 0 |

The assessment of the behaviour score was not done in a particular order but according to the animal's total spontaneous behaviour.

Open field test for locomotion activity

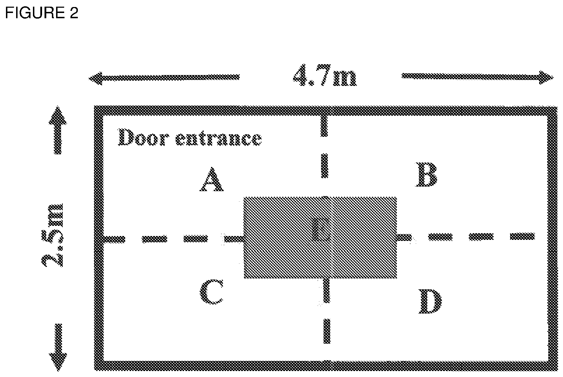

The open field is a rectangle arena 2.5m wide and 4.7m long. The walls of the arena are smooth and 1.6m high. In the morning of the test, the animals from all groups were introduced to the open field individually, one at a time, for a period of 5 minutes (5). The locomotor activity of the animals was recorded using a CCTV camera and analysed with the AnyMaze software (Stoelting Co.). The open field test was performed at the end of behavioural testing performed in the pen (i.e., approaching time, distress behaviour and von Frey). After each open field experiment, the following parameters were analysed: total walking distance (m) and percentages of time spent in the center of the area (Zone E; See Figure 2).

Distressed animals and animals under pain normally walk closer to the walls of the pen or the open field apparatus. Animals with no distress will not hesitate to enter the center of the open field apparatus.

## EXAMPLE 1

**Peri-operative administration of Dysport provides a delayed analgesic and anxiolytic effect post-surgery (incision on left flank of the pig)**

Pigs were administered intradermal injections of either saline, Exparel (266mg fixed doses) or different concentrations of Dysport immediately after suturing the incision made on the left flank of the pig (that is, peri-operatively). The mechanical sensitivity of pigs was measured by a von Frey assay as an assessment of treatment of post-operative surgical pain. When compared to the saline-treated group, Exparel showed an analgesic effect for a duration of 1 day but showed no effective analgesic activity afterwards. Administration of 400U of Dysport induced a moderate analgesic effect 2 days post-surgery. A greater analgesic effect was induced by 4 days post-surgery when pigs were administered either 200U or 400U of Dysport. All the concentrations of Dysport tested completely suppressed post-operative surgical pain 6 days post-operation. This suggests that Dysport provides an effective and prolonged analgesic effect for treating post-operative surgical pain. This data is illustrated in a bar chart in Figure 3A.

The latency of pigs to approach their handler was measured. Pigs were administered intradermal injections of either saline, Exparel or different concentrations of Dysport at the time of incision. By 2 hours post-surgery, all treatment groups showed a delay in approaching their handler. By 6 hours, intradermal administration with either 200U or 400U of Dysport reduced the time taken for pigs to approach their handler, with these effects continuing for up to 5 days post-surgery, suggesting a potential reduction in post-operative distress and anxiety-like reactivity. Pigs treated with either saline or Exparel failed to show any improvements in approaching their handler, suggesting that these treatments do not reduce post-operative distress and anxiety-like reactivity. This data is illustrated in Figure 3B.

The distress behaviour score of pigs was measured. Pigs administered either 100U, 200U or 400U of Dysport showed a reduction in their distress behaviour score by 2 days post-surgery, unlike saline and Exparel treated groups. This data is illustrated in Figure 3C.

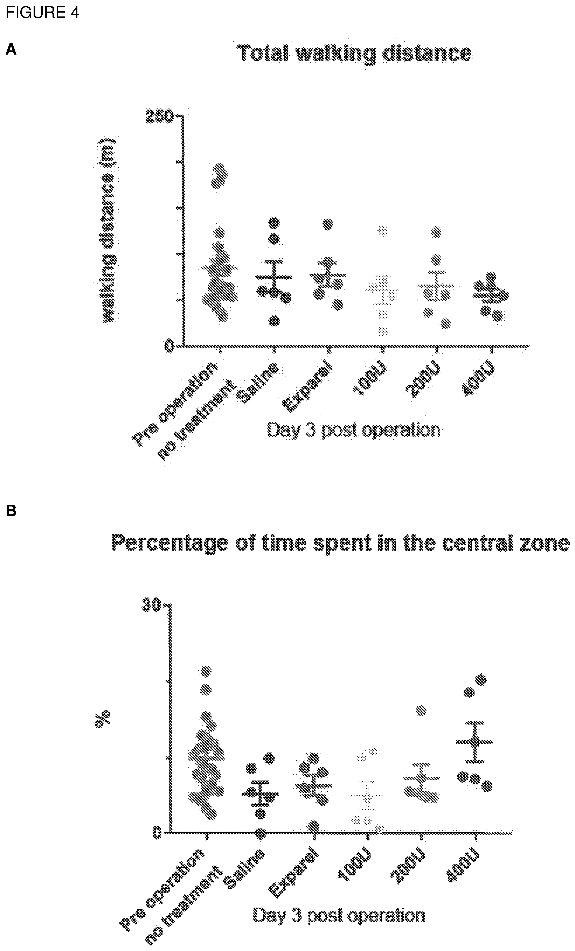

The open field test showed there was no difference between the total distance that the animals walked prior to surgery and post-surgery following saline treatment. Treatment with Exparel or Dysport did not affect the total walking distance at 3 days post dosing, suggesting that there was no change in the animals' motor function following the surgery. This data is illustrated in Figure 4A. Animals treated with 400U Dysport spent more time in the center of the open field apparatus, although this difference was not statistically significant (see Fig. 4B).

## EXAMPLE 2

**Pre-operative administration of Dysport induces a faster analgesic effect and suppresses the emergence of post-operative distress and anxiety-like reactivity when a surgical incision is made in the left flank of the pig**

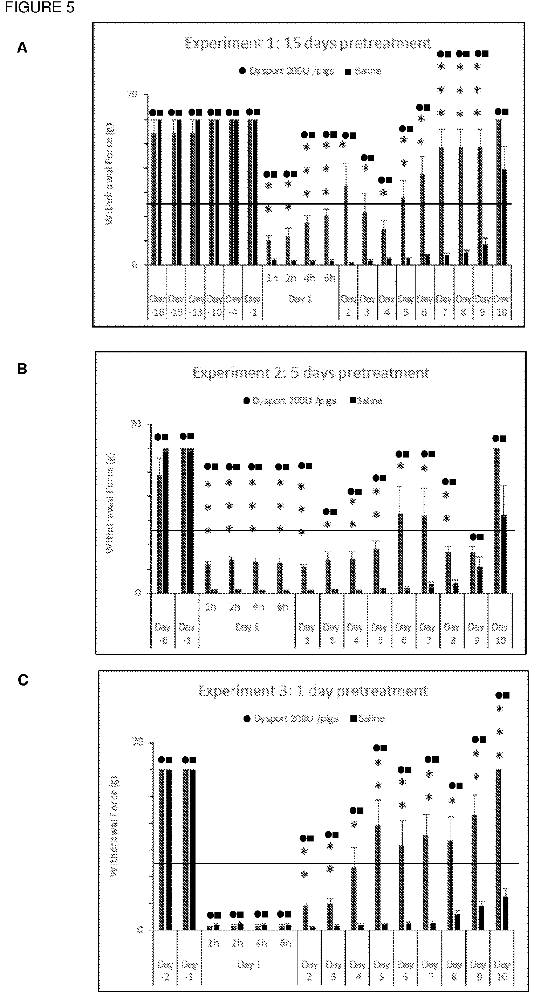

As the peri-operative administration of Dysport showed a delay in inducing an analgesic effect, the analgesic and anxiolytic effects of pre-operative administration of Dysport were measured. Pigs were administered intradermal injections of either saline or 200U of Dysport, at 15 days (see Fig. 5A), 5 days (see Fig. 5B) or 1 day (see Fig. 5C) prior to surgery (incision in the left flank of the pig). By using a Von Frey assay, the fastest analgesic effect was observed when Dysport was administered 15 days prior to surgery, where post-operative surgical pain was reduced by 1 day post-surgery. In comparison, when Dysport was administered 5 days prior to surgery, post-operative surgical pain was reduced by 5 days post-surgery.

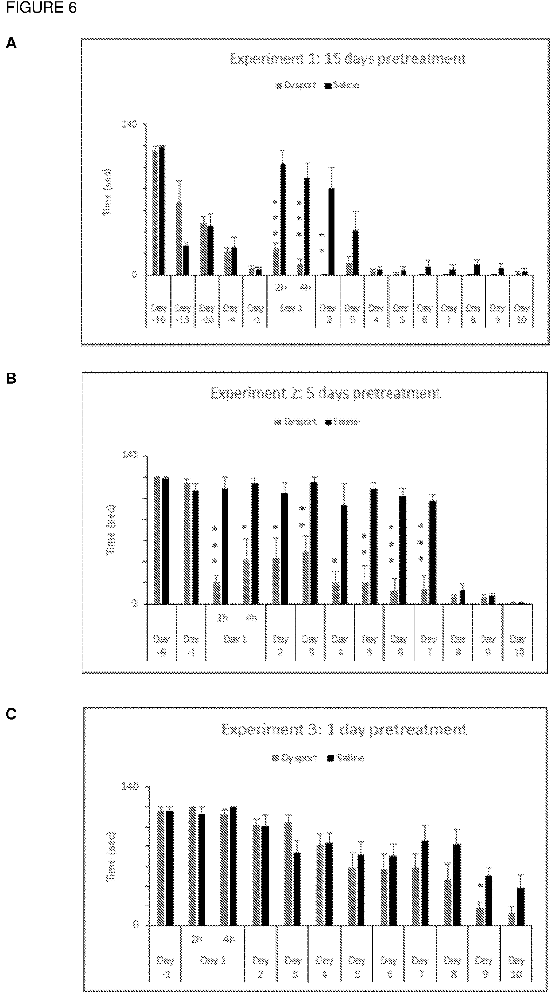

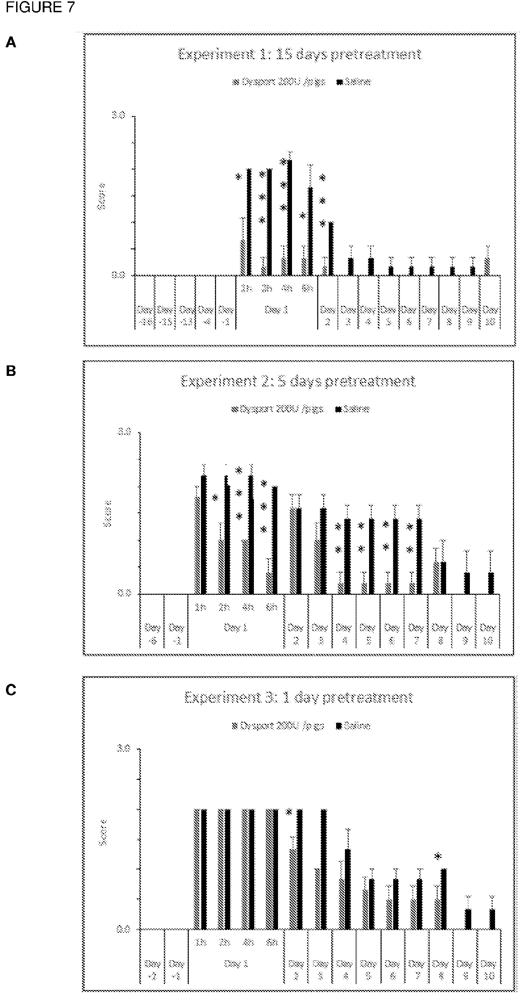

Pigs showed a reduced time to approach their handlers when administered intradermal injections of Dysport 15 or 5 days prior to surgery (see Fig. 6). Similarly, pigs showed a reduced distress behaviour score when administered intradermal injections of Dysport 15 or 5 days prior to surgery (see Fig. 7). The administration of Dysport 1 day prior to surgery did not induce as effective anxiolytic effects. This suggests that the pre-operative administration of Dysport 15 or 5 days prior to surgery fully prevents the emergence of post-operative distress and anxiety-like reactivity.

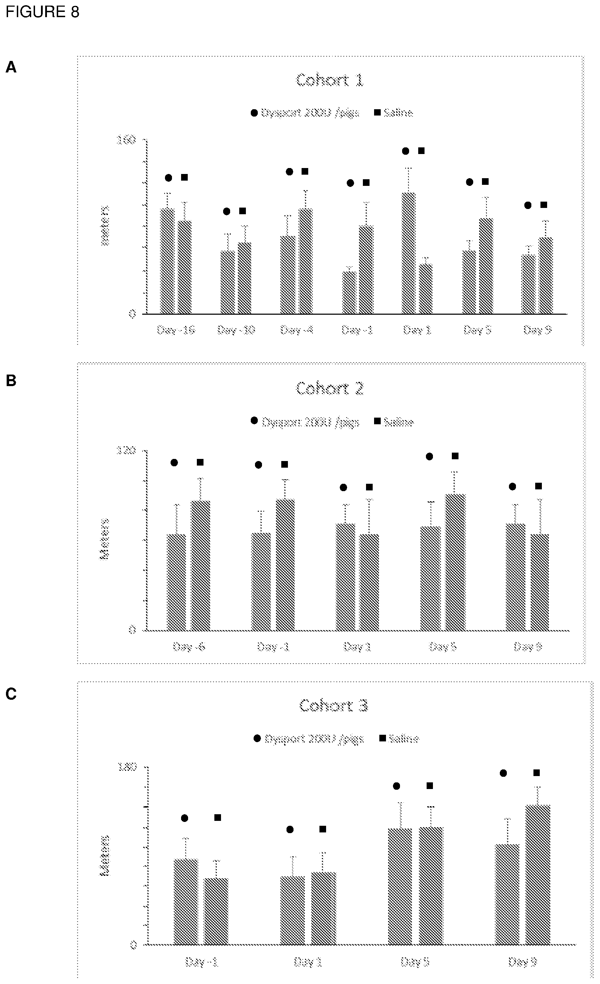

None of the treatment groups (intradermal injections of Dysport 15 days, 5 days or 1 day prior to surgery) showed a difference in their post-operative total walking distance (see Fig. 8), suggesting that muscle activity was unaffected and there was no systemic spread of the toxin.

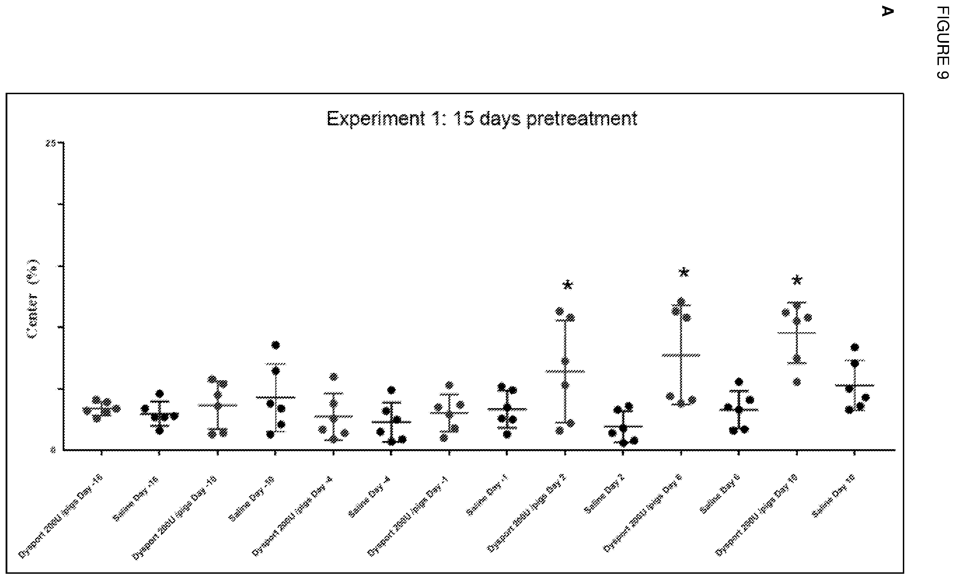

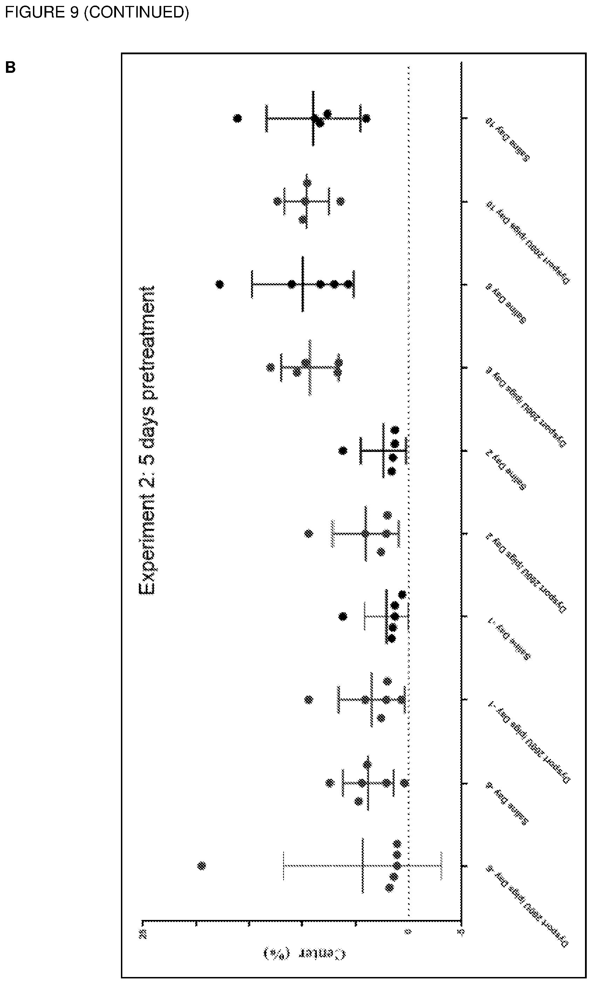

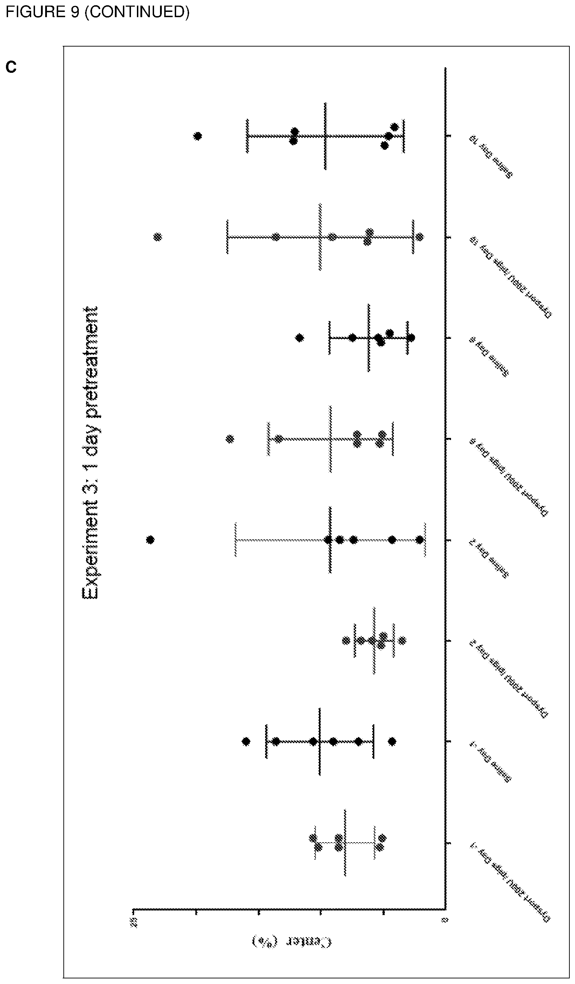

The percentage of time spent in the center of the open-field apparatus by saline-injected animals was similar before and following the surgery. Animals treated with Dysport 15 days prior to surgery spent more time in the center of the open filed apparatus (see Fig. 9A). There was no difference in the percentage of time spent in the central zone between saline and Dysport treated animals, when administered either at 5 days or 1 day prior to surgery (see Fig. 9B and Fig. 9C).

## EXAMPLE 3

### Intradermal administration of Dysport provides an advantageous route for mitigating post-operative surgical pain and suppressing the emergence of post-operative anxiety

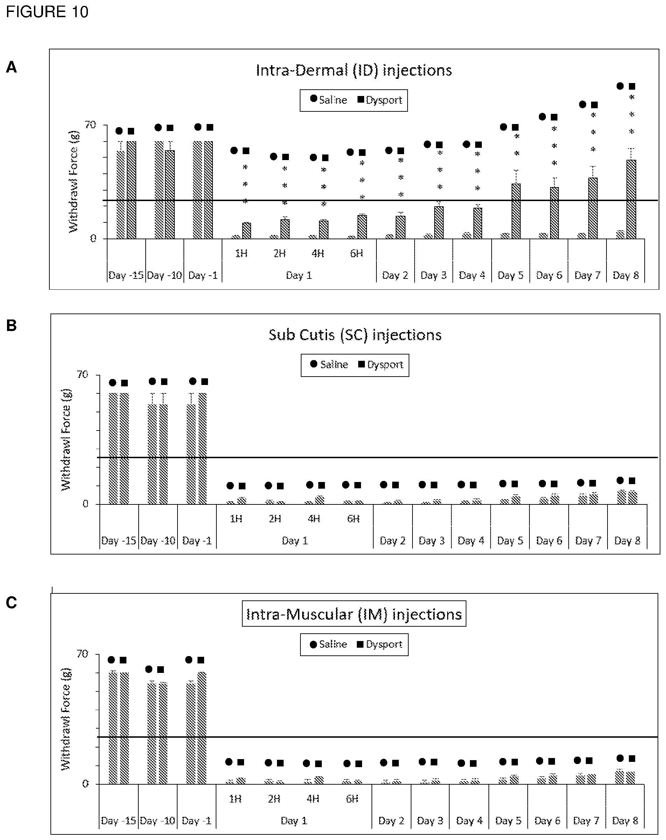

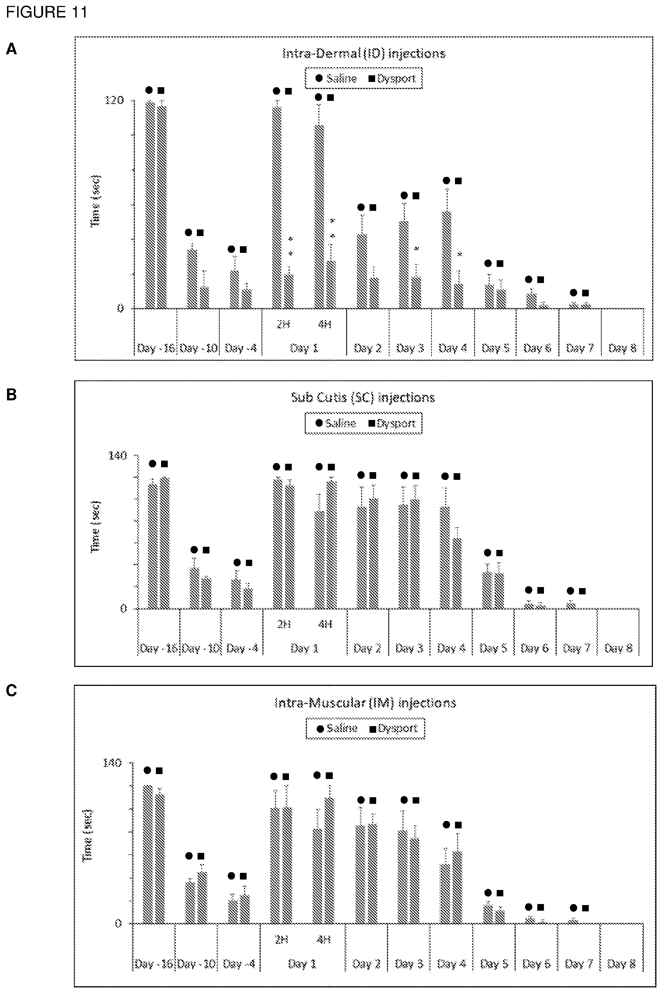

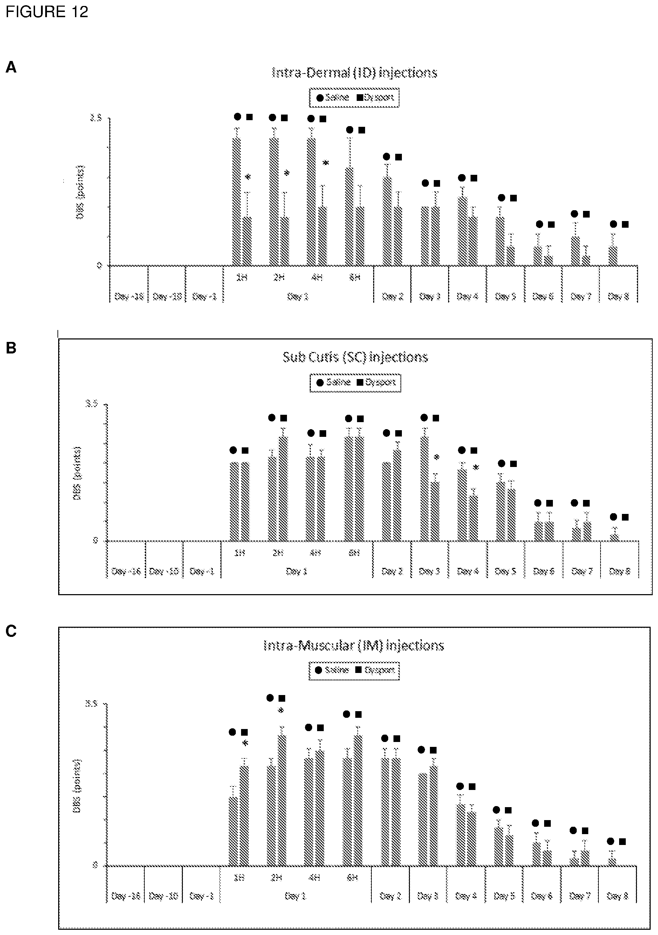

Different routes of 200U of Dysport administration (intradermal, subcutaneous and intramuscular injections) 15 days prior to surgery were assessed for their ability to induce analgesic and anxiolytic effects post-operatively. Surprisingly, intradermal administration provided for better results than the alternative routes (indeed, it was generally observed that only the intradermal route of Dysport administration showed a rapid, analgesic effect (see Fig. 10). Both the subcutaneous and intramuscular routes of Dysport administration showed little to no effects on analgesic activity. Pigs showed a reduced time to approach their handler and a reduced distress behaviour score when administered intradermal injections of Dysport 15 days prior to surgery (see Fig. 11 and Fig. 12). This suggests that the intradermal route of administration is effective at relieving post-operative surgical pain and preventing the full emergence of post-operative distress and anxiety-like reactivity.

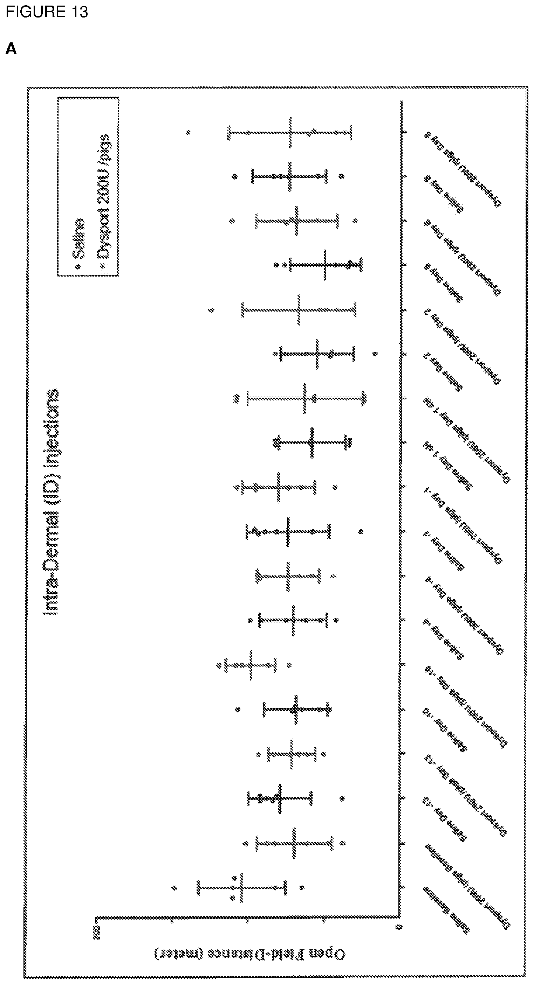

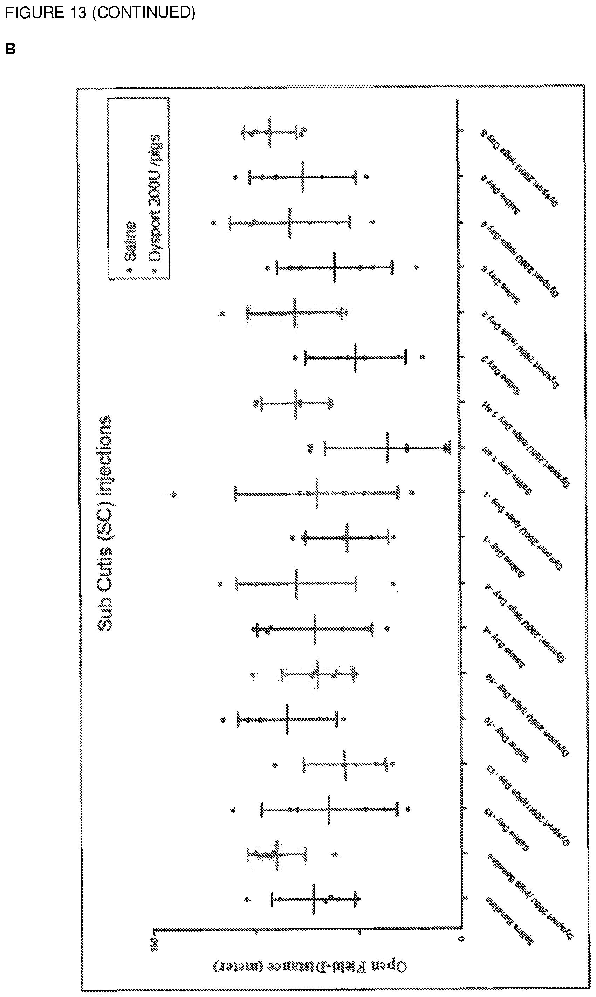

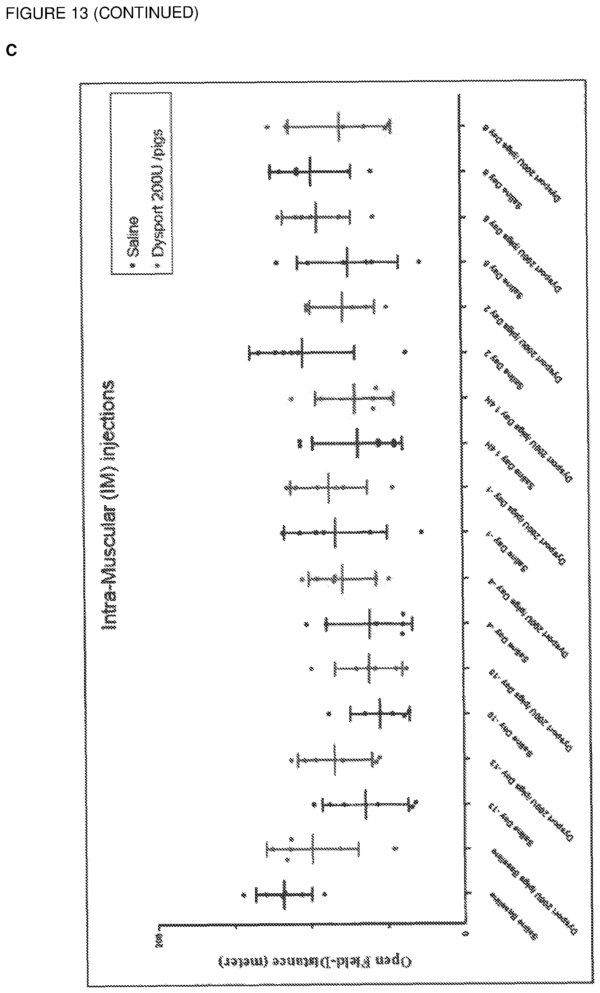

The walking distance recorded post-surgery was the same as that recorded prior to the surgery in all saline groups. Furthermore, treatment with Dysport and its route of administration (intradermal, subcutaneous or intramuscular routes) did not affect the total walking distance post-surgery (see Fig. 13).

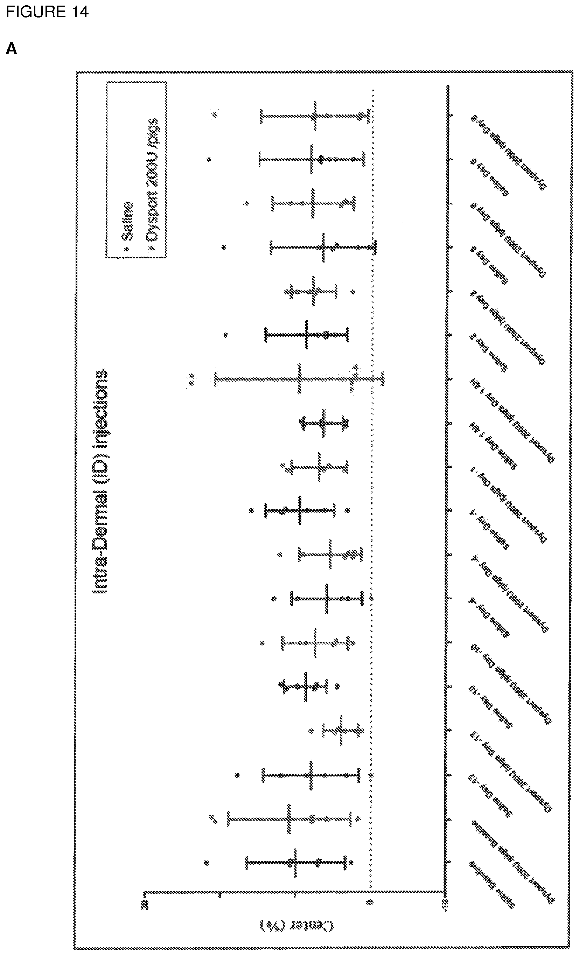

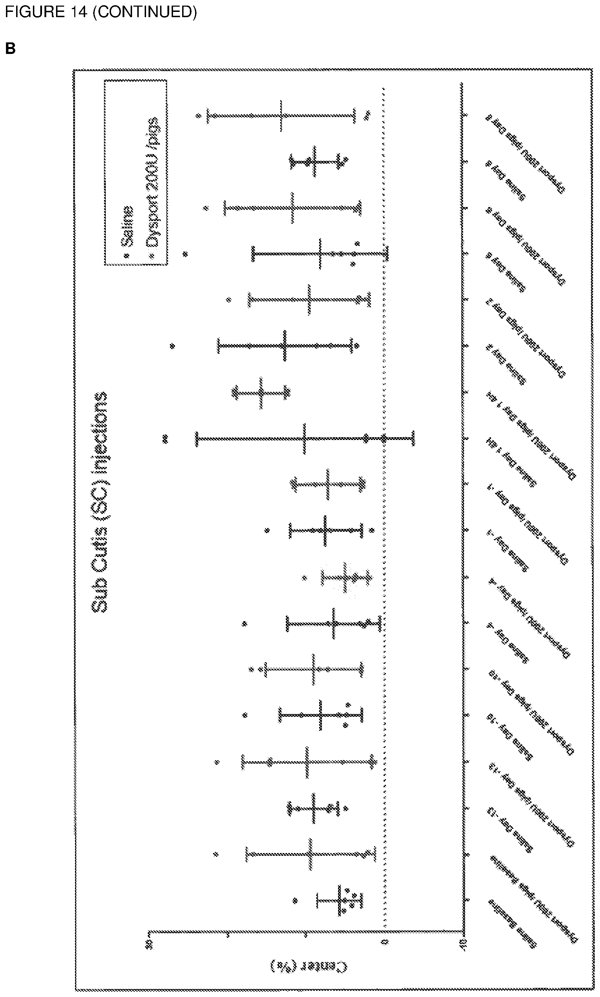

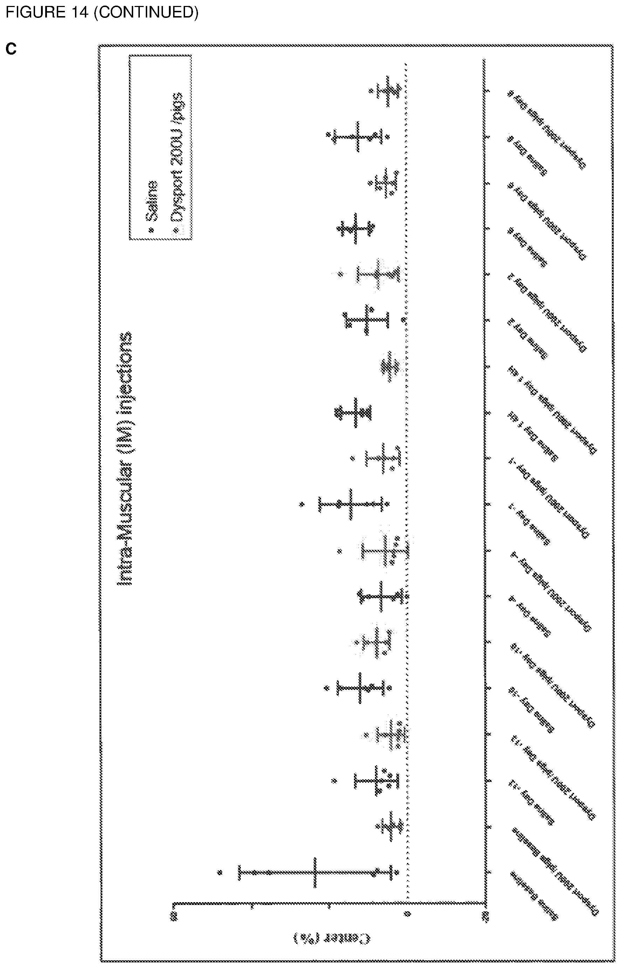

The percentage of time spent in the center of the open field apparatus by saline-injected animals was similar before and after surgery. There was no difference in the percentage of time animals spent in the center of the open field apparatus between the different administration routes (see Fig. 14).

## EXAMPLE 4

### SNAP-25 cleavage occurs at a site distal to Dysport injection, in the ipsilateral dorsal horn of the spinal cord

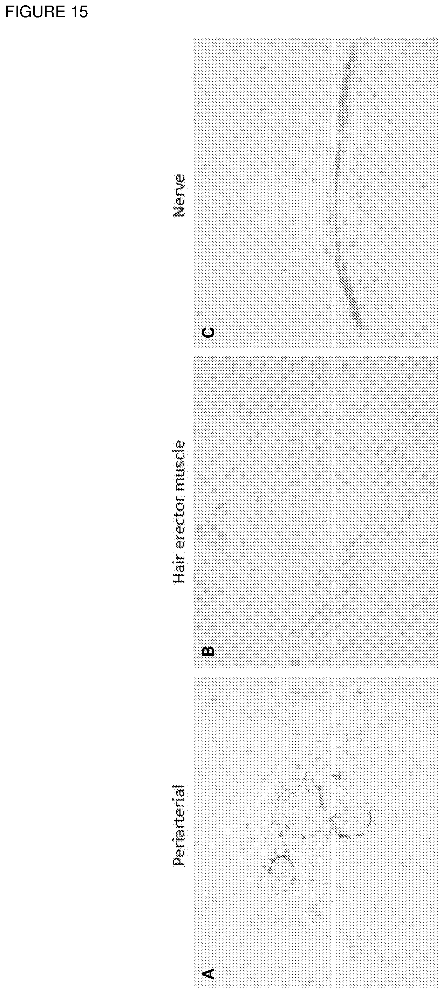

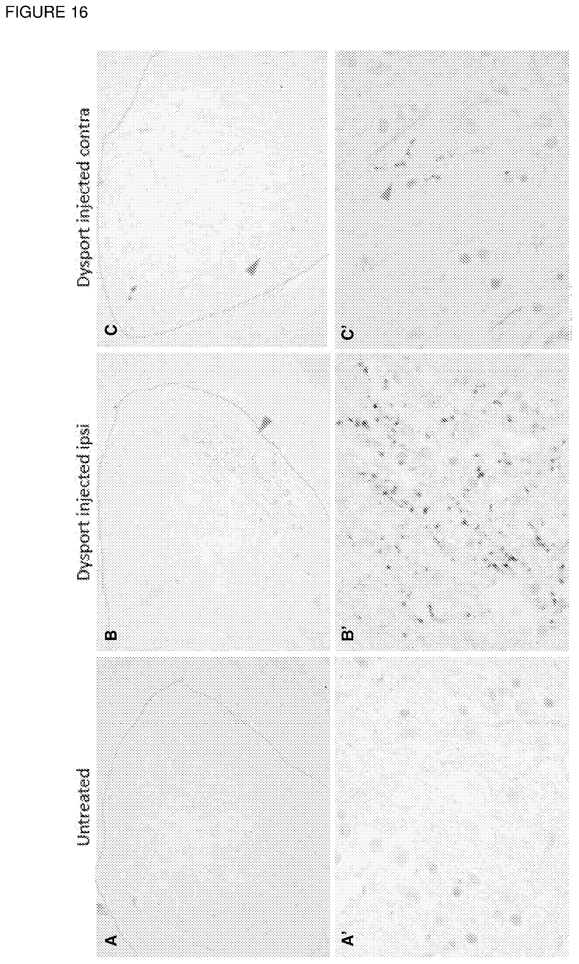

To assess the mechanism of action of Dysport (intradermal injection), immunohistochemistry was performed on both tissue samples at the site of surgical incision (left flank of the pig) and at the spinal cord. Cleaved SNAP-25 was not detected in the nerves of skin samples (see Fig. 15). Unexpectedly, cleaved SNAP-25 was visualised in the ipsilateral dorsal horn of the spinal cord (see Fig. 16), indicative of BoNT/A activity in the spinal cord and that post-operative surgical pain/anxiety control may be provided via a central effect in the spinal cord. This also highlights that Dysport may be administered directly into the spinal cord via intrathecal administration.

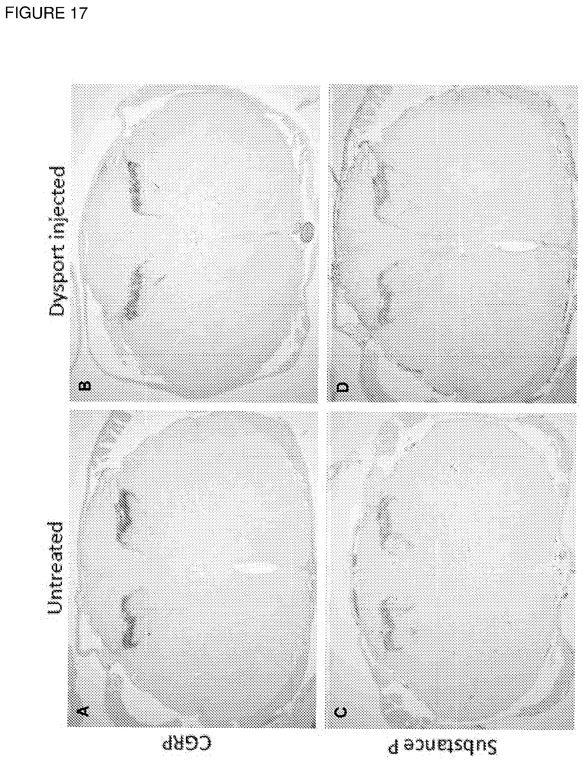

The expression levels of two neuropeptides involved in pain modulation, calcitonin gene related peptide (CGRP) and Substance P, were assessed in the spinal cord by immunohistochemistry staining. Neither neuropeptide showed a difference in their expression level in the spinal cord of pigs treated with Dysport compared to untreated (see Fig. 17).

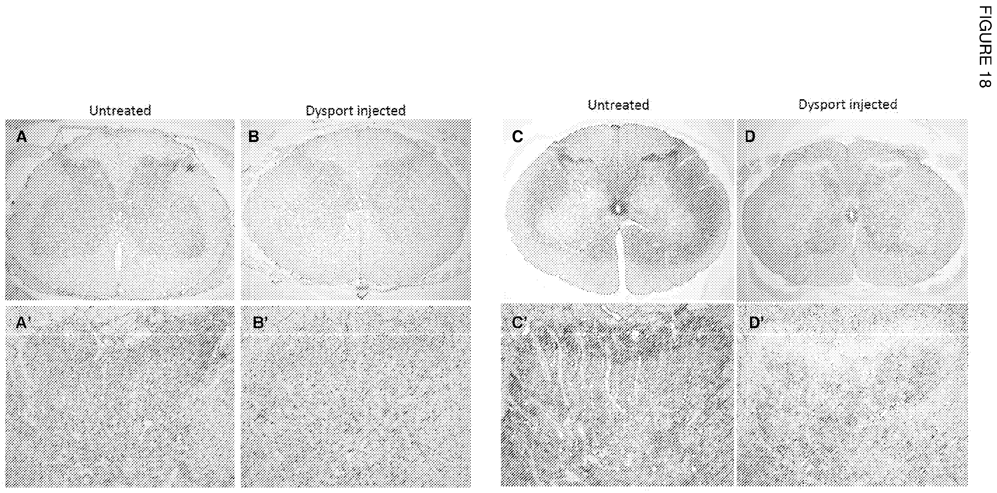

The expression levels of a marker of microglial cell activation, Iba1, were decreased in the spinal cord of pigs treated with Dysport when compared to untreated (see Fig 18A, B). Similarly, expression levels of a marker of astrocyte activation, GFAP, were decreased in the spinal cord of pigs treated with Dysport when compared to untreated (Fig. 18C, D).

## EXAMPLE 5

### Pre-operative administration of Dysport induces a fast analgesic effect and suppresses the emergence of post-operative distress and anxiety-like reactivity when a surgical incision is made in the left leg of the pig

Analgesic and anxiolytic effects of pre-operative administration of Dysport were measured when a different site of surgical incision was made to the pig (surgical incision to the left leg instead of the left flank). Pigs were administered intradermal injections of either saline or 200U of Dysport at 15 days prior to surgery or Exparel on the day of the surgery (day 1) (see Fig. 19A), (surgical incision to the left leg). By using a Von Frey assay, a fast analgesic effect was observed, where post-operative surgical pain was reduced by 1 day post-surgery and a long-lasting reversal of mechanical allodynia was observed by day 4.

Pigs (with a sutured incision in the left leg) showed a reduced time to approach their handlers when administered intradermal injections of Dysport 15 days prior to surgery when compared to administration of saline and Exparel (see Fig. 19B). Similarly, pigs (with a sutured incision in the left leg) showed a reduced distress behaviour score when administered intradermal injections of Dysport 15 days prior to surgery when compared to administration of saline and Exparel (see Fig. 19C). This suggests that the pre-operative administration of Dysport 15 days prior to surgery (incision to the left leg) fully prevents the emergence of post-operative distress and anxiety-like reactivity.

Overall, this experiment provides further support for the fast analgesic and anxiolytic effects when Dysport is administered 15 days prior to surgery.

## EXAMPLE 6

## SNAP-25 cleavage occurs in the ipsilateral dorsal horn of the spinal cord in pigs with a surgical incision to the left leg

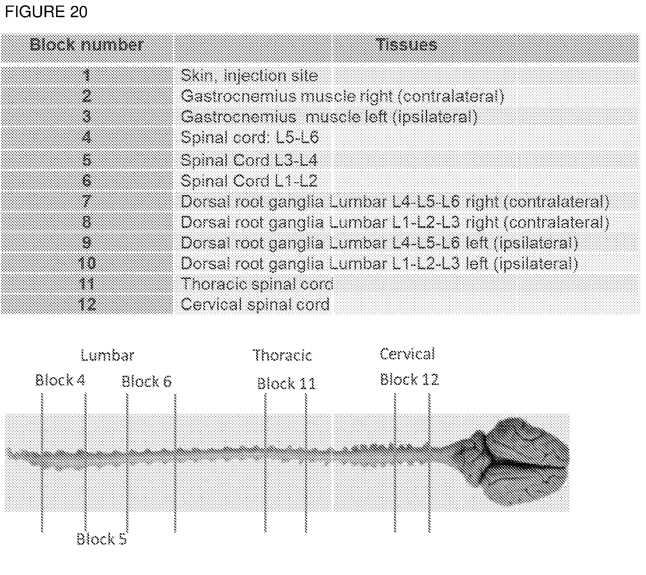

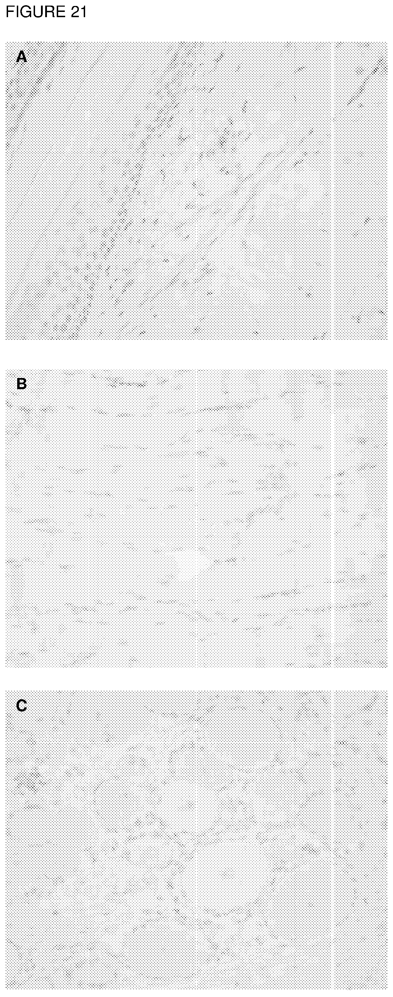

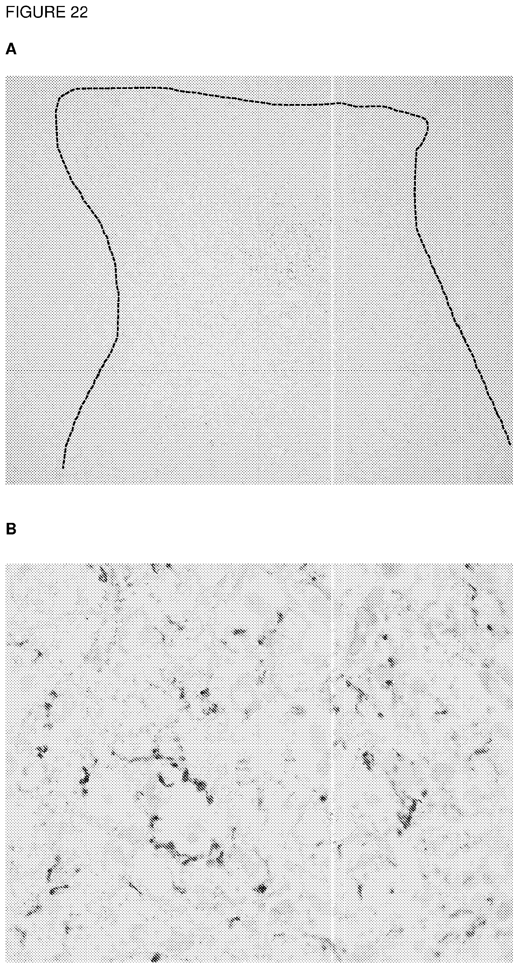

To assess whether a same mechanism of action of Dysport occurred when Dysport was intradermally administered at a different site in the pig, immunohistochemistry was performed on tissue samples at the site of surgical incision (left leg of the pig) and at different regions of the spinal cord (see Fig. 20). Cleaved SNAP-25 was not detected in the nerves of skin samples on samples collected 5-7 days after the incision and injection of Dysport (see Fig. 21). Cleaved SNAP-25 was visualised in the ipsilateral dorsal horn of the spinal cord, specifically in lumbar regions L5-L6 (see Fig. 22), similar to findings in pigs with a surgical incision made to their left flank. These findings are indicative of BoNT/A activity in the spinal cord and that post-operative surgical pain/anxiety control may be provided via a central effect in the spinal cord. The localisation of cleaved SNAP-25 staining in the ipsilateral dorsal horn was different when compared to cleaved SNAP-25 staining in pigs with a surgical incision made to their left flank.

The intensity of cleaved SNAP-25 staining was graded on a scale of 1-3, with grade 0 = no cleaved SNAP-25 staining, grade 1 = low intensity cleaved SNAP-25 staining, grade 2 = average cleaved SNAP-25 intensity staining and grade 3 = high intensity cleaved SNAP-25 staining (see Fig. 23A). Based on said grading system, pigs with a surgical incision made to their left leg had lower intensity cleaved SNAP-25 staining in the ipsilateral dorsal horn when compared to pigs with a surgical incision made to their left flank.

The intensity of cleaved SNAP-25 staining was quantified (see Fig. 23B). A H-Score was calculated as a measure of cleaved SNAP-25 staining intensity. The H-Score was calculated by multiplying the % of positive spinal cord sections by the staining intensity in the dorsal horns. In pigs treated with Dysport (and with a surgical incision in the left leg), the highest staining of cleaved SNAP-25 was observed in the spinal cord in lumbar regions L5-L6 (assigned a cleaved SNAP-25 intensity staining of grade 2) when compared to lumbar regions L3-L4 and L1-L2, and the thoracic and cervical regions of the spinal cord (the cervical region had traces of cleaved SNAP-25 staining). There was no evidence of cleaved SNAP-25 staining in saline or Exparel injected pigs.

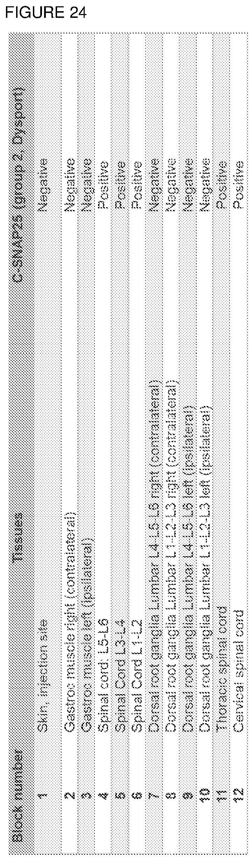

The above immunohistochemistry staining is summarised in Fig. 24 and confirm that SNAP-25 cleavage is observed in localised regions of the spinal cord. Localised regions L5-L6, L3-L4 and L1-L2, and the thoracic and the cervical regions of the spinal cord tested positive for cleaved SNAP-25 staining whilst the remaining tissues (including the skin, at the injection site) tested negative for cleaved SNAP-25 staining.

Various modifications and variations of the described methods and system of the present invention will be apparent to those skilled in the art.

Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

### Claims

1. A botulinum neurotoxin A (BoNT/A) for use in treating post-operative surgical pain in a patient, said method comprising administering to a patient the BoNT/A 5-50 days prior to surgery; and

    wherein the BoNT/A is administered intradermally; and
    wherein said post-operative surgical pain is caused by surgical intervention comprising an incision that cuts through the:

        (i) skin; and
        (ii) fascia, muscle, bone, and/or an organ in the patient.

2. The BoNT/A for use according to claim 1, wherein:

    a) the BoNT/A is administered 6-50 days prior to surgery; preferably 10-20 days prior to surgery;
    b) wherein the BoNT/A is administered 14-16 days prior to surgery, preferably about 15 days prior to surgery; or

c) wherein the BoNT/A is administered 5-20 days prior to surgery, preferably 5-15 days prior to surgery.

3. The BoNT/A for use according to claim 1 or 2, wherein the patient is administered a therapeutic dose of the BoNT/A ranging from about 0.0003 ng to about 2 ng, from about 0.0004 ng to about 1.5 ng, from about 0.0005 ng to about 1 ng, or from about 0.0006 ng to about 0.5 ng of said BoNT/A , and wherein administration of the BoNT/A substantially reduces post-operative surgical pain perception by the patient and wherein the reduced post-operative surgical pain perception is maintained for 24 hours immediately following surgery.

4. The BoNT/A for use according to any one of the preceding claims, wherein the BoNT/A is administered at a site distal to the site of surgical intervention.

5. The BoNT/A for use according to claim 4, wherein the distal site to the surgical incision is at least 15 cm, 50 cm or 100 cm from the site of surgical intervention.

6. The BoNT/A for use according to any one of the preceding claims, wherein:

   a) following administration, the BoNT/A travels by retrograde transport to the spinal cord and effects SNARE protein cleavage (SNAP-25 protein cleavage) in said spinal cord; and/or
   b) minimal or no SNARE protein cleavage (SNAP-25 protein cleavage) by said BoNT/A is observed at or proximal to said intradermal site following administration of the BoNT/A.

7. The BoNT/A for use according to any one of the preceding claims, wherein the post-operative surgical pain is acute post-operative surgical pain.

8. The BoNT/A for use according to any one of the preceding claims, wherein the post-operative surgical pain is chronic post-operative surgical pain.

9. The BoNT/A for use according to any one of the preceding claims, wherein the patient is administered a total dose of 1-3 ng of the BoNT/A.

10. The BoNT/A for use according to any one of the preceding claims, wherein the patient is administered 80-250 pg of the BoNT/A per kg (bodyweight).

11. The BoNT/A for use according to any one of the preceding claims, wherein the BoNT/A is administered at more than one administration site; preferably wherein the patient is administered 10-170 pg of the BoNT/A per administration site; more preferably 1-14 pg/kg bodyweight per administration site.

12. The BoNT/A for use according to any one of the preceding claims, wherein the patient experiences post-operative anxiety caused by post-operative pain, and wherein following administration of said BoNT/A the patient experiences reduced or suppressed post operative anxiety.

**Patentansprüche**

1. Ein Botulinumneurotoxin A (BoNT/A) zur Verwendung bei der Behandlung postoperativer Operationsschmerzen bei einem Patienten, wobei das genannte Verfahren das Verabreichen des BoNT/A 5-50 Tage vor der Operation an den Patienten umfasst; und

   wobei das BoNT/A intradermal verabreicht wird; und
   wobei die genannten postoperativen Operationsschmerzen durch einen chirurgischen Eingriff verursacht werden, der einen Einschnitt umfasst, der durch Folgendes schneidet:

   (i) Haut; und
   (ii) Faszien, Muskel, Knochen und/oder ein Organ in dem Patienten.

2. BoNT/A zur Verwendung nach Anspruch 1, wobei:

   a) das BoNT/A 6-50 Tage vor der Operation, bevorzugt 10-20 Tage vor der Operation verabreicht wird;

b) wobei das BoNT/A 14-16 Tage vor der Operation, bevorzugt etwa 15 Tage vor der Operation verabreicht wird; oder

c) wobei das BoNT/A 5-20 Tage vor der Operation, bevorzugt 5-15 Tage vor der Operation verabreicht wird.

**3.** BoNT/A zur Verwendung nach Anspruch 1 oder 2, wobei dem Patienten eine therapeutische Dosis des BoNT/A verabreicht wird, die im Bereich von etwa 0,0003 ng bis etwa 2 ng, von etwa 0,0004 ng bis etwa 1,5 ng, von etwa 0,0005 ng bis etwa 1 ng oder von etwa 0,0006 ng bis etwa 0,5 ng des genannten BoNT/A liegt, und wobei die Verabreichung des BoNT/A im Wesentlichen die postoperative Operationsschmerzempfindung durch den Patienten reduziert und wobei die reduzierte postoperative Operationsschmerzempfindung 24 Stunden unmittelbar nach der Operation aufrechterhalten bleibt.

**4.** BoNT/A zur Verwendung nach einem der vorhergehenden Ansprüche, wobei das BoNT/A an einem Ort distal des Orts des chirurgischen Eingriffs verabreicht wird.

**5.** BoNT/A zur Verwendung nach Anspruch 4, wobei der distal des Orts des chirurgischen Einschnitts liegende Ort mindestens 15 cm, 50 cm oder 100 cm von dem Ort des chirurgischen Eingriffs entfernt liegt.

**6.** BoNT/A zur Verwendung nach einem der vorhergehenden Ansprüche, wobei:

a) sich das BoNT/A nach der Verabreichung mittels retrogradem Transport zum Rückenmark bewegt und sich in dem genannten Rückenmark auf die SNARE-Proteinspaltung (SNAP-25-Proteinspaltung) auswirkt; und/oder

b) nach Verabreichung des BoNT/A eine minimale oder keine SNARE-Proteinspaltung (SNAP-25-Proteinspaltung) durch das genannte BoNT/A an dem oder proximal des genannten intradermalen Orts zu beobachten ist.

**7.** BoNT/A zur Verwendung nach einem der vorhergehenden Ansprüche, wobei die postoperativen Operationsschmerzen akute postoperative Operationsschmerzen sind.

**8.** BoNT/A zur Verwendung nach einem der vorhergehenden Ansprüche, wobei die postoperativen Operationsschmerzen chronische postoperative Operationsschmerzen sind.

**9.** BoNT/A zur Verwendung nach einem der vorhergehenden Ansprüche, wobei dem Patienten eine Gesamtdosis von 1-3 ng des BoNT/A verabreicht werden.

**10.** BoNT/A zur Verwendung nach einem der vorhergehenden Ansprüche, wobei dem Patienten 80-250 pg des BoNT/A pro kg (Körpergewicht) verabreicht werden.

**11.** BoNT/A zur Verwendung nach einem der vorhergehenden Ansprüche, wobei das BoNT/A an mehr als einem Verabreichungsort verabreicht wird; wobei dem Patienten bevorzugt 10-170 pg des BoNT/A pro Verabreichungsort, bevorzugter 1-14 pg/kg Körpergewicht pro Verabreichungsort verabreicht werden.

**12.** BoNT/A zur Verwendung nach einem der vorhergehenden Ansprüche, wobei der Patient postoperative Angst, die durch postoperative Schmerzen ausgelöst wird, erlebt und wobei der Patient nach Verabreichung des genannten BoNT/A eine reduzierte oder supprimierte postoperative Angst erlebt.

**Revendications**

**1.** Neurotoxine botulique A (BoNT/A) destinée à être utilisée dans le traitement de la douleur chirurgicale postopératoire chez un patient, ledit procédé comprenant l'administration à un patient de la BoNT/A 5 à 50 jours avant la chirurgie ; et

dans laquelle la BoNT/A est administrée par voie intradermique ; et
dans laquelle ladite douleur chirurgicale postopératoire est causée par une intervention chirurgicale comprenant une incision qui coupe à travers :

(i) la peau ; et
(ii) le fascia, le muscle, l'os, et/ou un organe dans le patient.

**2.** BoNT/A destinée à être utilisée selon la revendication 1, dans laquelle :

(a) la BoNT/A est administrée 6 à 50 jours avant la chirurgie, de préférence 10 à 20 jours avant la chirurgie ;
(b) dans laquelle la BoNT/A est administrée 14 à 16 jours avant la chirurgie, de préférence environ 15 jours avant la chirurgie ; ou
(c) dans laquelle la BoNT/A est administrée 5 à 20 jours avant la chirurgie, de préférence 5 à 15 jours avant la chirurgie.

**3.** BoNT/A destinée à être utilisée selon la revendication 1 ou 2, dans laquelle le patient reçoit l'administration d'une dose thérapeutique de la BoNT/A allant d'environ 0,0003 ng à environ 2 ng, d'environ 0,0004 ng à environ 1,5 ng, d'environ 0,0005 ng à environ 1 ng, ou d'environ 0,0006 ng à environ 0,5 ng de ladite BoNT/A, et dans laquelle l'administration de la BoNT/A réduit sensiblement la perception de la douleur chirurgicale post-opératoire par le patient et dans laquelle la perception de la douleur chirurgicale post-opératoire réduite est maintenue pendant 24 heures immédiatement après l'opération.

**4.** BoNT/A destinée être utilisée selon l'une quelconque des revendications précédentes, dans laquelle la BoNT/A est administrée au niveau d'un site distal par rapport au site de l'intervention chirurgicale.

**5.** BoNT/A destinée à être utilisée selon la revendication 4, dans laquelle le site distal par rapport à l'incision chirurgicale est à au moins 15 cm, 50 cm ou 100 cm du site de l'intervention chirurgicale.

**6.** BoNT/A destinée à être utilisée selon l'une quelconque des revendications précédentes, dans laquelle :

(a) après l'administration, la BoNT/A se déplace par transport rétrograde jusqu'à la moelle épinière et provoque un clivage de la protéine SNARE (clivage de la protéine SNAP-25) dans ladite moelle épinière ; et/ou
(b) un clivage minimal ou nul de la protéine SNARE (clivage de la protéine SNAP-25) par ladite BoNT/A est observé au niveau ou à proximité dudit site intradermique suite à l'administration de la BoNT/A.

**7.** BoNT/A destinée être utilisée selon l'une quelconque des revendications précédentes, dans laquelle la douleur chirurgicale postopératoire est une douleur chirurgicale postopératoire aiguë.

**8.** BoNT/A destinée être utilisée selon l'une quelconque des revendications précédentes, dans laquelle la douleur chirurgicale postopératoire est une douleur chirurgicale postopératoire chronique.

**9.** BoNT/A destinée être utilisée selon l'une quelconque des revendications précédentes, dans laquelle le patient reçoit l'administration d'une dose totale de 1 à 3 ng de BoNT/A.

**10.** BoNT/A destinée être utilisée selon l'une quelconque des revendications précédentes, dans laquelle le patient reçoit l'administration de 80 à 250 pg de BoNT/A par kg (poids corporel).

**11.** BoNT/A destinée à être utilisée selon l'une quelconque des revendications précédentes, dans laquelle la BoNT/A est administrée au niveau de plus d'un site d'administration ; de préférence, dans laquelle le patient reçoit l'administration de 10 à 170 pg de BoNT/A par site d'administration ; de manière plus préférée de 1 à 14 pg/kg de poids corporel par site d'administration.

**12.** BoNT/A destinée à être utilisée selon l'une quelconque des revendications précédentes, dans laquelle le patient éprouve une anxiété post-opératoire causée par une douleur post-opératoire, et dans laquelle après l'administration de ladite BoNT/A, le patient éprouve une anxiété post-opératoire réduite ou supprimée.

FIGURE 1

FIGURE 2

FIGURE 3

**A**

**B**

**C**

FIGURE 4

A

**Total walking distance**

B

**Percentage of time spent in the central zone**

FIGURE 5

A — Experiment 1: 15 days pretreatment

B — Experiment 2: 5 days pretreatment

C — Experiment 3: 1 day pretreatment

FIGURE 6

A

B

C

FIGURE 7

**A**

**B**

**C**

FIGURE 8

FIGURE 9

A

Experiment 1: 15 days pretreatment

FIGURE 9 (CONTINUED)

Experiment 2: 5 days pretreatment

FIGURE 9 (CONTINUED)

FIGURE 10

A

B

C

FIGURE 11

A

B

C

FIGURE 12

FIGURE 13

A

FIGURE 13 (CONTINUED)

B

FIGURE 13 (CONTINUED)

C

FIGURE 14

A

FIGURE 14 (CONTINUED)

B

FIGURE 14 (CONTINUED)

C

FIGURE 15

FIGURE 16

FIGURE 17

FIGURE 18

FIGURE 19

A

B

FIGURE 19 (CONTINUED)

C

FIGURE 20

| Block number | Tissues |
|---|---|
| 1 | Skin, injection site |
| 2 | Gastrocnemius muscle right (contralateral) |
| 3 | Gastrocnemius muscle left (ipsilateral) |
| 4 | Spinal cord: L5-L6 |
| 5 | Spinal Cord L3-L4 |
| 6 | Spinal Cord L1-L2 |
| 7 | Dorsal root ganglia Lumbar L4-L5-L6 right (contralateral) |
| 8 | Dorsal root ganglia Lumbar L1-L2-L3 right (contralateral) |
| 9 | Dorsal root ganglia Lumbar L4-L5-L6 left (ipsilateral) |
| 10 | Dorsal root ganglia Lumbar L1-L2-L3 left (ipsilateral) |
| 11 | Thoracic spinal cord |
| 12 | Cervical spinal cord |

FIGURE 21

FIGURE 22

A

B

FIGURE 23

A

Grade 3     Grade 2     Grade 1

B

**Dysport ID**

Grade 2

Grade 0,5 (traces)

H-Score = % of positive spinal cord sections x staining intensity in the dorsal horns

FIGURE 24

| Block number | Tissues | C-SNAP25 (group 2, Dysport) |
|---|---|---|
| 1 | Skin, injection site | Negative |
| 2 | Gastroc muscle right (contralateral) | Negative |
| 3 | Gastroc muscle left (ipsilateral) | Negative |
| 4 | Spinal cord: L5-L6 | Positive |
| 5 | Spinal Cord L3-L4 | Positive |
| 6 | Spinal Cord L1-L2 | Positive |
| 7 | Dorsal root ganglia Lumbar L4-L5-L6 right (contralateral) | Negative |
| 8 | Dorsal root ganglia Lumbar L1-L2-L3 right (contralateral) | Negative |
| 9 | Dorsal root ganglia Lumbar L4-L5-L6 left (ipsilateral) | Negative |
| 10 | Dorsal root ganglia Lumbar L1-L2-L3 left (ipsilateral) | Negative |
| 11 | Thoracic spinal cord | Positive |
| 12 | Cervical spinal cord | Positive |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0178760 A2
- US 6113915 A1
- WO 2019195454 A1
- RU 2628238 C1
- WO 2020234573 A1
- US 20080113051 A1
- WO 99032272 A

- WO 2011022357 A
- US 20070166332 A
- WO 2016156113 A
- WO 2015004461 A1
- WO 2017191315 A
- US 5223409 A
- WO 9206204 A

### Non-patent literature cited in the description

- **BARWOOD et al.** *Dev Med Child Neurol.*, February 2000, vol. 42 (2), 116-21
- **DOHIN et al.** *Rev Chir Orthop Reparatrice Appar Mot.*, November 2007, vol. 93 (7), 674-81
- **SAEIDIBOROJENI et al.** *Toxicon.*, 2020, vol. 188, 48-54
- **POGATZKI-ZAHN**. *Pain Rep.*, March 2017, vol. 2 (2), e588
- **GERALD K**. Cell and Molecular Biology. John Wiley & Sons, 2002
- **ROSSETTO, O. et al.** Botulinum neurotoxins: genetic structural and mechanistic insights.. *Nature Reviews Microbiology*, 2014, vol. 12 (8), 535-549
- **FIELD**. AbobotulinumtoxinA (Dysport®), OnabotulinumtoxinA (Botox®), and IncobotulinumtoxinA (Xeomin®) Neurotoxin Content and Potential Implications for Duration of Response in Patients. *Toxins*, 2018, vol. 10 (12), 535
- **FREVERT**. Content of botulinum neurotoxin in Botox®/Vistabel®, Dysport®/Azzalure®, and Xeomin®/Bocouture. *Drugs R D.*, 2010, vol. 10 (2), 67-73
- **MUÑOZ et al.** Quantification of protein calibrants by amino acid analysis using isotope dilution mass spectrometry. *Anal. Biochem.*, 2011, vol. 408, 124-131
- **PORAS et al.** Detection and Quantification of Botulinum Neurotoxin Type A by a Novel Rapid In Vitro Fluorimetric Assay. *Appl Environ Microbiol.*, July 2009, vol. 75 (13), 4382-4390
- **JULIE D. THOMPSON et al.** CLUSTAL W: Improving the Sensitivity of Progressive Multiple Sequence Alignment Through Sequence Weighting, Position-Specific Gap Penalties and Weight Matrix Choice. *Nucleic Acids Research*, 1994, vol. 22 (22), 4673-4680

- **OSAMU GOTOH**. Significant Improvement in Accuracy of Multiple Protein. Sequence Alignments by Iterative Refinement as Assessed by Reference to Structural Alignments. *J. Mol. Biol.*, 1996, vol. 264 (4), 823-838
- **ERIC DEPIEREUX** ; **ERNEST FEYTMANS**. MatchBox: A Fundamentally New Algorithm for the Simultaneous Alignment of Several Protein Sequences. *CABIOS*, 1992, vol. 8 (5), 501-509
- **GIBBS** ; **C. E. LAWRENCE et al.** Detecting Subtle Sequence Signals: A Gibbs Sampling Strategy for Multiple Alignment. *Science*, 1993, vol. 262 (5131), 208-214
- **IVO VAN WALLE et al.** Align-M - A New Algorithm for Multiple Alignment of Highly Divergent Sequences. *Bioinformatics*, 2004, vol. 20 (9), 1428-1435
- **ALTSCHUL et al.** *Bull. Math. Bio.*, 1986, vol. 48, 603-16
- **HENIKOFF** ; **HENIKOFF**. *Proc. Natl. Acad. Sci. USA*, 1992, vol. 89, 10915-19
- **ROBERTSON et al.** *J. Am. Chem. Soc.*, 1991, vol. 113, 2722
- **ELLMAN et al.** *Methods Enzymol.*, 1991, vol. 202, 301
- **CHUNG et al.** *Science*, 1993, vol. 259, 806-9
- **CHUNG et al.** *Proc. Natl. Acad. Sci. USA*, 1993, vol. 90, 10145-9
- **TURCATTI et al.** *J. Biol. Chem.*, 1996, vol. 271, 19991-8
- **KOIDE et al.** *Biochem.*, 1994, vol. 33, 7470-6
- **WYNN** ; **RICHARDS**. *Protein Sci.*, 1993, vol. 2, 395-403
- **CUNNINGHAM** ; **WELLS**. *Science*, 1989, vol. 244, 1081-5
- **VOS et al.** *Science*, 1992, vol. 255, 306-12
- **SMITH et al.** *J. Mol. Biol.*, 1992, vol. 224, 899-904

- **WLODAVER et al.** *FEBS Lett.*, 1992, vol. 309, 59-64
- **REIDHAAR-OLSON** ; **SAUER**. *Science*, 1988, vol. 241, 53-7
- **BOWIE** ; **SAUER**. *Proc. Natl. Acad. Sci. USA*, 1989, vol. 86, 2152-6
- **LOWMAN et al.** *Biochem.*, 1991, vol. 30, 10832-7
- **DERBYSHIRE et al.** *Gene*, 1986, vol. 46, 145
- **NER et al.** *DNA*, 1988, vol. 7, 127
- **SINGLETON et al.** DICTIONARY OF MICROBIOLOGY AND MOLECULAR BIOLOGY. John Wiley and Sons, 1994
- **HALE** ; **MARHAM**. THE HARPER COLLINS DICTIONARY OF BIOLOGY. Harper Perennial, 1991
- **CASTEL et al.** Characterization of a porcine model of post-operative pain. *Eur. J. Pain.*, 2014, vol. 18 (4), 496-505